(19) Europäisches Patentamt
European Patent Office
Office européen des brevets

(11) EP 4 410 791 A1

(12) # EUROPEAN PATENT APPLICATION
published in accordance with Art. 153(4) EPC

(43) Date of publication:
**07.08.2024 Bulletin 2024/32**

(21) Application number: **22875187.1**

(22) Date of filing: **30.09.2022**

(51) International Patent Classification (IPC):
***C07D 471/04*** *(2006.01)* ***A61K 31/4353*** *(2006.01)*
***A61P 35/00*** *(2006.01)*

(52) Cooperative Patent Classification (CPC):
**A61K 31/4353; A61P 35/00; C07D 471/04**

(86) International application number:
**PCT/CN2022/123423**

(87) International publication number:
**WO 2023/051807 (06.04.2023 Gazette 2023/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.09.2021 CN 202111163555**
**14.05.2022 CN 202210525022**
**25.05.2022 CN 202210578890**
**20.06.2022 CN 202210694483**
**04.07.2022 CN 202210786346**

(71) Applicant: **Xizang Haisco Pharmaceutical Co., Ltd.**
**Lhoka, Tibet 856099 (CN)**

(72) Inventors:
- **LI, Yao**
  **Shannan, Tibet 856000 (CN)**
- **WANG, Wenjing**
  **Shannan, Tibet 856000 (CN)**
- **CHEN, Lei**
  **Shannan, Tibet 856000 (CN)**
- **KANG, Peng**
  **Shannan, Tibet 856000 (CN)**
- **FU, Chao**
  **Shannan, Tibet 856000 (CN)**
- **CHENG, Fengkai**
  **Shannan, Tibet 856000 (CN)**
- **TANG, Pingming**
  **Shannan, Tibet 856000 (CN)**
- **YU, Yan**
  **Shannan, Tibet 856000 (CN)**
- **ZHANG, Chen**
  **Shannan, Tibet 856000 (CN)**
- **YAN, Pangke**
  **Shannan, Tibet 856000 (CN)**

(74) Representative: **Casalonga**
**Casalonga & Partners**
**Bayerstraße 71/73**
**80335 München (DE)**

(54) **BICYCLIC DERIVATIVE PARP INHIBITOR AND USE THEREOF**

(57) Disclosed are a compound as shown in formula (I), a stereoisomer, pharmaceutically acceptable salt, solvate, cocrystal or deuterated compound thereof, or a pharmaceutical composition containing same, and the use thereof as a PARP-1 inhibitor in the preparation of a drug for treating related diseases, wherein the definition of each group in formula (I) is as defined in the description.

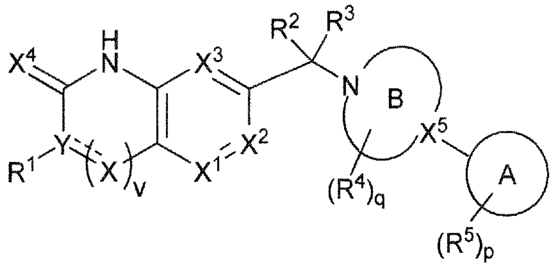

(I)

EP 4 410 791 A1

## Description

### Technical Field

The present invention belongs to the field of drugs, and in particular relates to a small molecule compound having a PARP-1 inhibitory activity, or a stereoisomer, pharmaceutically acceptable salt, solvate, cocrystal or deuterated material thereof, and the use thereof in the preparation of a drug for treating related diseases.

### Background Art

Approximately 5% of breast cancer patients are associated with germline mutations in the BRCA1/2 genes (3% in the BRCA1 gene and 2% in the BRCA2 gene). Most breast cancers caused by BRCA1 mutations are triple-negative breast cancers (70%), while BRCA2 mutations are more likely to cause estrogen receptor-positive breast cancers (70%). The BRCA1/2 genes are tumour suppressor genes and play an important role in DNA damage repair, normal cell growth, etc. Mutations in the genes can inhibit the normal repair ability after DNA damage and cause homologous recombination deficiency (HRD), that is, loss of BRCA function or mutation or loss of function in other homologous recombination-related genes, making repair of DNA double-strand breaks impossible through homologous recombination repair (HRR), eventually leading to cancer.

Poly(ADP-ribose) polymerase (PARP) is a DNA repair enzyme that plays a key role in the DNA repair pathway. PARP is activated by DNA damage and breakage. As a molecular sensor of DNA damage, it has the function of identifying and binding to the DNA break location, thereby activating and catalysing the polyADP ribosylation of the receptor protein and participating in the DNA repair process. PARP plays a key role in the process of DNA single-strand base excision and repair. In HRD tumour cells, DNA double-strand breaks cannot be repaired, and PARP inhibitors block the single-strand repair, resulting in a "synthetic lethal" effect, leading to tumour cell death.

PARP inhibitors have a "trapping" effect on the PARP protein, causing the PARP protein that binds to damaged DNA to be trapped on the DNA, directly causing other DNA repair proteins to be unable to bind, eventually leading to cell death. At present, several PARP inhibitors have been successfully developed, such as olaparib, rucapalib and niraparib. However, adverse reactions limit their ability to be used in combination with chemotherapy drugs. This may be related to the lack of selectivity of marketed PARP inhibitors against the PARP family. These side effects include intestinal toxicity caused by tankyrase inhibition and haematological toxicity caused by PARP-2 inhibition. Therefore, it is of great clinical significance to develop highly selective PARP-1 inhibitors and reduce the toxic and side effects associated with non-selective PARP inhibitors.

### Summary of the Invention

The objective of the present invention is to provide a compound that inhibits PARP-1, or a stereoisomer, solvate, deuterated compound, or pharmaceutically acceptable salt thereof, and the medical application thereof. The compound has the advantages of good efficacy, low toxic and side effects, high safety, strong selectivity, good pharmacokinetics, high bioavailability, and no inhibition to CYP enzymes.

The present invention relates to a compound as shown in formula (I), (II), (II-a), (II-b), (III), (III-a), (III-b), (III-c), (IV), (IV-a), (IV-b), (IV-c), (V), (VI), (VII), (VIII) or (IX), or a stereoisomer, solvate, deuterated compound, or pharmaceutically acceptable salt thereof,

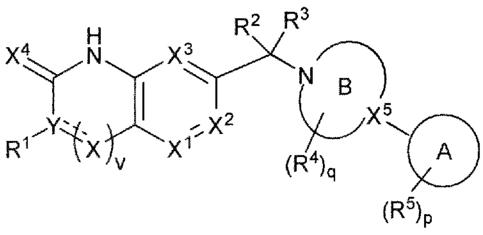
(I)

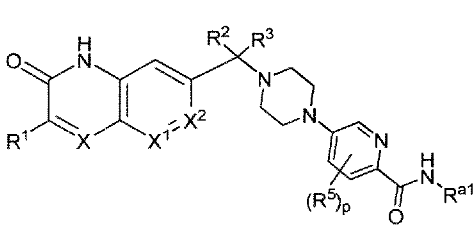
(II)

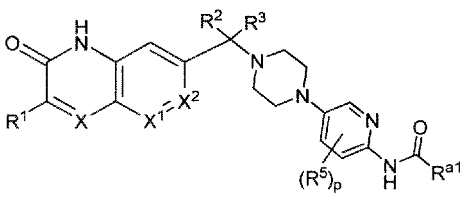
(II-b)

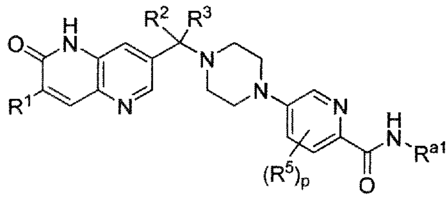
(II-a)

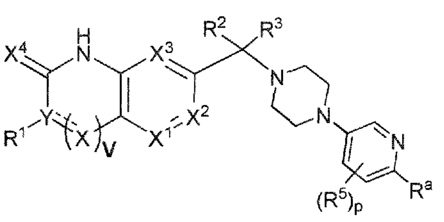
(III)

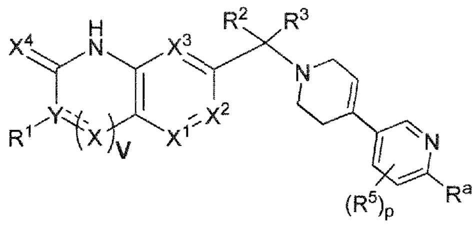
(IV)

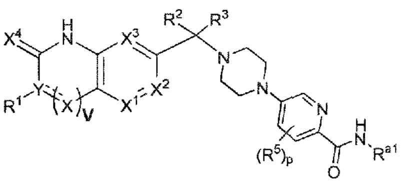
(III-a)

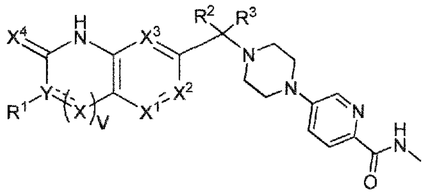
(III-b)

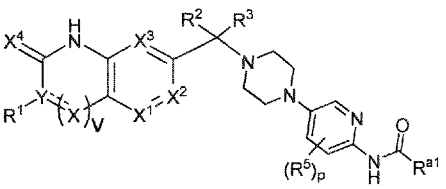
(III-c)

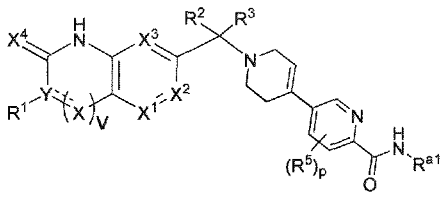
(IV-a)

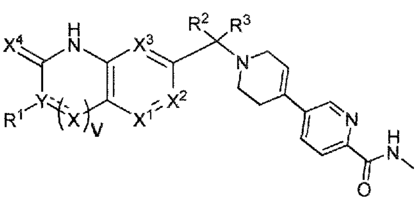
(IV-b)

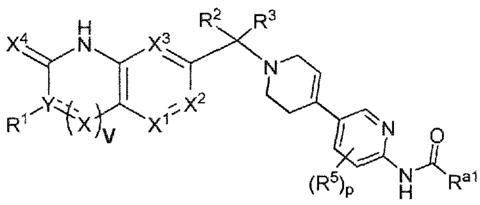
(IV-c)

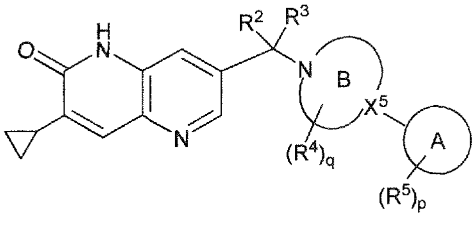
(V)

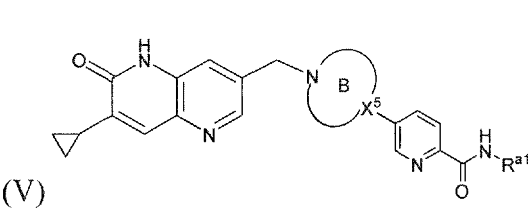
(VI)

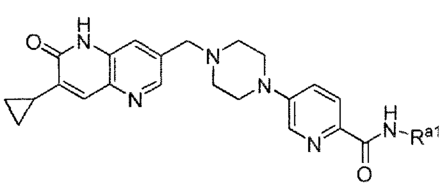
(VII)

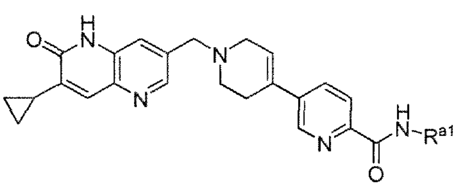
(VIII)

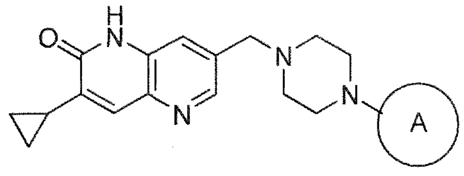

(IX)

wherein X is selected from $CR^x$, $C(R^x)_2$, O, N or $NR^x$;

Y is selected from N, C or CH; in some embodiments, Y is selected from N or C; in some embodiments, Y is selected from C;

------ represents a single bond or a double bond;

v is selected from 1, 2 or 3; in some embodiments, v is selected from 1 or 2; in some embodiments, v is selected from 1;

$X^1$, $X^2$ and $X^3$ are each independently selected from N or $CR^x$; in some embodiments, $X^1$ is selected from N, and $X^2$ and $X^3$ are selected from $CR^x$; in some embodiments, $X^1$, $X^2$ and $X^3$ are selected from N; in some embodiments, $X^1$ is selected from N, $X^2$ is selected from N, and $X^3$ is selected from $CR^x$; in some embodiments, X' is selected from N, $X^2$ is selected from $CR^x$, and $X^3$ is selected from N; in some embodiments, $X^1$, $X^2$ and $X^3$ are selected from $CR^x$; in some embodiments, $X^1$ is selected from $CR^x$, and $X^2$ and $X^3$ are selected from N; in some embodiments, $X^1$ is selected from $CR^x$, $X^2$ is selected from N, and $X^3$ is selected from $CR^x$; in some embodiments, $X^1$ is selected from $CR^x$, $X^2$ is selected from $CR^x$, and $X^3$ is selected from N;

provided that when ------- represents a double bond, and v is selected from 1, X, $X^1$, $X^2$ and $X^3$ are not all selected from $CR^x$;

$X^4$ is selected from O or S; in some embodiments, $X^4$ is selected from O; in some embodiments, $X^4$ is selected from S;

$X^5$ is selected from N or $CR^x$; in some embodiments, $X^5$ is selected from N or CH; in some embodiments, $X^5$ is selected from N; in some embodiments, $X^5$ is selected from $CR^x$; in some embodiments, $X^5$ is selected from CH;

each $R^x$ is independently selected from H, D, halogen, cyano, amino, hydroxyl, $-SF_5$, $C_{1-6}$ alkyl, halo $C_{1-6}$ alkyl, halo $C_{1-6}$ alkoxy, deuterated $C_{1-6}$ alkyl, deuterated $C_{1-6}$ alkoxy, $C_{1-6}$ alkoxy, $C_{2-6}$ alkenyl, $C_{2-6}$ alkynyl, $C_{1-6}$ alkyl-O-$C_{1-6}$ alkyl, $-(CH_2)_r-C_{3-12}$ cycloalkyl or $-(CH_2)_r$-(3- to 12-membered heterocycloalkyl); or two $R^x$ on the same carbon atom together form =O; in some embodiments, each $R^x$ is independently selected from H, D, halogen, cyano, amino, hydroxyl, $C_{1-4}$ alkyl, halo $C_{1-4}$ alkyl, halo $C_{1-4}$ alkoxy, deuterated $C_{1-4}$ alkyl, deuterated $C_{1-4}$ alkoxy, $C_{1-4}$ alkoxy, $C_{2-4}$ alkenyl, $C_{2-4}$ alkynyl, $C_{1-2}$ alkyl-O-$C_{1-2}$ alkyl, $-(CH_2)_r-C_{3-6}$ monocyclic cycloalkyl, $-(CH_2)_r-C_{5-7}$ bicyclic spiro cycloalkyl, $-(CH_2)_r$-(4- to 6-membered heterocycloalkyl), or $-(CH_2)_r$-(7- to 9-membered bicyclic spiro heterocycloalkyl); or two $R^x$ on the same carbon atom together form =O; in some embodiments, each $R^x$ is independently selected from H, D, F, Cl, cyano, amino, hydroxyl, $C_{1-2}$ alkyl, halo $C_{1-2}$ alkyl, deuterated $C_{1-2}$ alkyl, $C_{1-2}$ alkoxy, $-(CH_2)_r-C_{3-4}$ monocyclic cycloalkyl, - $(CH_2)_r-C_{5-6}$ bicyclic spiro cycloalkyl, $-(CH_2)_r$-(4- to 5-membered heterocycloalkyl), or $-(CH_2)_r$-(7- to 8-membered bicyclic spiro heterocycloalkyl); or two $R^x$ on the same carbon atom together form =O; in some embodiments, each $R^x$ is independently selected from H, D, F, Cl, cyano, hydroxyl, $C_{1-2}$ alkyl, halo $C_{1-2}$ alkyl, or deuterated $C_{1-2}$ alkyl; or two $R^x$ on the same carbon atom together form =O; in some embodiments, each $R^x$ is independently selected from H, D, $C_{1-2}$ alkyl, halo $C_{1-2}$ alkyl, or deuterated $C_{1-2}$ alkyl; or two $R^x$ on the same carbon atom together form =O; in some embodiments, each $R^x$ is independently selected from H or D, or two $R^x$ on the same carbon atom together form =O; in some embodiments, each $R^x$ is independently selected from H or D;

R' is selected from halogen, nitro, cyano, amino, hydroxyl, $-SF_5$, $C_{1-6}$ alkyl, $C_{1-6}$ alkoxy, $C_{2-6}$ alkenyl, $C_{2-6}$ alkynyl, $C_{1-6}$ alkyl-O-$C_{1-6}$ alkyl, $-(CH_2)_r-C_{3-12}$ cycloalkyl or $-(CH_2)_r$-(3- to 12-membered heterocycloalkyl), wherein the alkyl, alkoxy, alkenyl, alkynyl, cycloalkyl or heterocycloalkyl is optionally further substituted with 1, 2 or 3 groups selected from D, halogen, cyano, amino, hydroxyl, $C_{1-6}$ alkyl or $C_{1-6}$ alkoxy; in some embodiments, $R^1$ is selected from halogen, nitro, cyano, amino, hydroxyl, - $SF_5$, $C_{1-4}$ alkyl, $C_{1-4}$ alkoxy, $C_{2-4}$ alkenyl, $C_{2-4}$ alkynyl, $C_{1-2}$ alkyl-O-$C_{1-2}$ alkyl, - $(CH_2)_r-C_{3-6}$ monocyclic cycloalkyl, $-(CH_2)_r-C_{5-7}$ bicyclic spiro cycloalkyl, $-(CH_2)_r$-(4- to 6-membered heterocycloalkyl), or $-(CH_2)_r$-(6- to 9-membered bicyclic spiro heterocycloalkyl), wherein the alkyl, alkoxy, alkenyl, alkynyl, cycloalkyl, or heterocycloalkyl is optionally further substituted with 1, 2 or 3 groups selected from D, halogen, cyano, amino, hydroxyl, $C_{1-2}$ alkyl, or $C_{1-2}$ alkoxy; in some embodiments, $R^1$ is selected from F, Cl, cyano, amino, hydroxyl, $C_{1-2}$ alkyl, $C_{1-2}$ alkoxy, $C_{2-3}$ alkenyl, $C_{1-2}$ alkyl-O-$C_{1-2}$ alkyl, $-(CH_2)_r-C_{3-4}$ monocyclic cycloalkyl, $-(CH_2)_r-C_{5-7}$ bicyclic spiro cycloalkyl, $-(CH_2)_r$-(4- to 5-membered heterocycloalkyl), or $-(CH_2)_r$-(6- to 8-membered bicyclic spiro heterocycloalkyl), wherein the alkyl, alkoxy, alkenyl, alkynyl, cycloalkyl, or heterocycloalkyl is optionally further substituted with 1, 2 or 3 groups selected from D, halogen, cyano, amino, hydroxyl, $C_{1-2}$ alkyl, or $C_{1-2}$ alkoxy; in some embodiments, $R^1$ is selected from cyano, $C_{1-2}$ alkyl, $C_{2-3}$ alkenyl, $C_{1-2}$ alkyl-O-$C_{1-2}$ alkyl, $C_{3-4}$ monocyclic cycloalkyl, or 4- to 5-membered heterocycloalkyl, wherein the alkyl, alkenyl, cycloalkyl, or heterocycloalkyl is optionally further substituted with 1, 2 or 3 groups selected from D, F, Cl, cyano, amino, hydroxyl, $C_{1-2}$ alkyl, or $C_{1-2}$ alkoxy; in some embodiments, $R^1$ is selected from cyano, $C_{1-2}$ alkyl, $C_{2-3}$ alkenyl, $C_{1-2}$ alkyl-O-$C_{1-2}$ alkyl, or $C_{3-4}$ monocyclic cycloalkyl;

each r is independently selected from 0, 1, 2 or 3; in some embodiments, each r is independently selected from 0 or 1; in some embodiments, r is selected from 0;

$R^2$ and $R^3$ are each independently selected from H, D, halogen, cyano, amino, hydroxyl, $C_{1-6}$ alkyl-O-$C_{1-6}$ alkyl, hydroxy $C_{1-6}$ alkyl, $C_{1-6}$ alkoxy, halo $C_{1-6}$ alkyl, halo $C_{1-6}$ alkoxy, deuterated $C_{1-6}$ alkyl, deuterated $C_{1-6}$ alkoxy or $C_{1-6}$ alkyl; or $R^2$ and $R^3$ together with the carbon atom to which they are attached form $C_{3-5}$ cycloalkyl or 4- to 5-membered heterocycloalkyl;

in some embodiments, $R^2$ and $R^3$ are each independently selected from H, D, halogen, cyano, amino, hydroxyl, $C_{1-2}$ alkyl-O-$C_{1-2}$ alkyl, hydroxy $C_{1-4}$ alkyl, $C_{1-4}$ alkoxy, halo $C_{1-4}$ alkyl, halo $C_{1-4}$ alkoxy, deuterated $C_{1-4}$ alkyl, deuterated $C_{1-4}$ alkoxy or $C_{1-4}$ alkyl; or $R^2$ and $R^3$ together with the carbon atom to which they are attached form $C_{3-5}$ cycloalkyl or 4- to 5-membered heterocycloalkyl; in some embodiments, $R^2$ and $R^3$ are each independently selected from H, D, halogen, cyano, amino, hydroxyl, $C_{1-2}$ alkyl-O-$C_{1-2}$ alkyl, hydroxy $C_{1-2}$ alkyl, $C_{1-2}$ alkoxy, halo $C_{1-2}$ alkyl, halo $C_{1-2}$ alkoxy, deuterated $C_{1-2}$ alkyl, deuterated $C_{1-2}$ alkoxy or $C_{1-2}$ alkyl; or $R^2$ and $R^3$ together with the carbon atom to which they are attached form $C_{3-4}$ cycloalkyl or 4-membered heterocycloalkyl; in some embodiments, $R^2$ and $R^3$ are each independently selected from H, D, F, hydroxyl, halo $C_{1-2}$ alkyl, deuterated $C_{1-2}$ alkyl or $C_{1-2}$ alkyl; or $R^2$ and $R^3$ together with the carbon atom to which they are attached form $C_{3-4}$ cycloalkyl; in some embodiments, $R^2$ and $R^3$ are each independently selected from H, D, or $C_{1-2}$ alkyl; or $R^2$ and $R^3$ together with the carbon atom to which they are attached form $C_{3-4}$ cycloalkyl; in some embodiments, $R^2$ and $R^3$ are each independently selected from H, D, or $C_{1-2}$ alkyl; or $R^2$ and $R^3$ together with the carbon atom to which they are attached form $C_{3-4}$ cycloalkyl; in some embodiments, $R^2$ and $R^3$ are each independently selected from H or D; in some embodiments, $R^2$ and $R^3$ are each independently selected from H, D, halogen, cyano, amino, hydroxyl, $C_{1-2}$ alkyl-O-$C_{1-2}$ alkyl, hydroxy $C_{1-2}$ alkyl, $C_{1-2}$ alkoxy, halo $C_{1-2}$ alkyl, halo $C_{1-2}$ alkoxy, deuterated $C_{1-2}$ alkyl, deuterated $C_{1-2}$ alkoxy or $C_{1-2}$ alkyl; or $R^2$ and $R^3$ together with the carbon atom to which they are attached form $C_{3-5}$ cycloalkyl or 4-to 5-membered heterocycloalkyl;

each $R^4$ is independently selected from D, halogen, cyano, amino, hydroxyl, - $SF_5$, $C_{1-6}$ alkyl, $C_{1-6}$ alkoxy, halo $C_{1-6}$ alkyl, halo $C_{1-6}$ alkoxy, deuterated $C_{1-6}$ alkyl or deuterated $C_{1-6}$ alkoxy; or two $R^4$ on the same carbon atom together with the carbon atom to which they are attached form =O; in some embodiments, each $R^4$ is independently selected from D, halogen, cyano, amino, hydroxyl, $C_{1-4}$ alkyl, $C_{1-4}$ alkoxy, halo $C_{1-4}$ alkyl, halo $C_{1-4}$ alkoxy, deuterated $C_{1-4}$ alkyl, or deuterated $C_{1-4}$ alkoxy; or two $R^4$ on the same carbon atom together with the carbon atom to which they are attached form =O; in some embodiments, each $R^4$ is independently selected from D, halogen, cyano, amino, hydroxyl, -$SF_5$, $C_{1-2}$ alkyl, $C_{1-2}$ alkoxy, halo $C_{1-2}$ alkyl, halo $C_{1-2}$ alkoxy, deuterated $C_{1-2}$ alkyl or deuterated $C_{1-2}$ alkoxy; or two $R^4$ on the same carbon atom together with the carbon atom to which they are attached form =O; in some embodiments, each $R^4$ is independently selected from D, F, Cl, cyano, amino, hydroxyl, $C_{1-2}$ alkyl, $C_{1-2}$ alkoxy, halo $C_{1-2}$ alkyl, halo $C_{1-2}$ alkoxy, deuterated $C_{1-2}$ alkyl, or deuterated $C_{1-2}$ alkoxy; or two $R^4$ on the same carbon atom together with the carbon atom to which they are attached form =O; in some embodiments, each $R^4$ is independently selected from D, F, Cl, methyl, ethyl, methoxy, ethoxy, - $CH_2F$, -$CHF_2$, -$CF_3$, -$CH_2CH_2F$, -$CH_2CHF_2$, -$CH_2CF_3$, -$CHFCH_2F$, -$CHFCHF_2$, - $CHFCF_3$, -$CF_2CH_2F$, -$CF_2CHF_2$, -$CF_2CF_3$, -$CH_2D$, -$CHD_2$, -$CD_3$, -$CH_2CH_2D$, - $CH_2CHD_2$, -$CH_2CD_3$, -$CHDCH_2D$, -$CHDCHD_2$, -$CHDCD_3$, -$CD_2CH_2D$, - $CD_2CHD_2$, or -$CD_2CD_3$; or two $R^4$ on the same carbon atom together with the carbon atom to which they are attached form =O;

each $R^5$ is independently selected from D, halogen, cyano, amino, hydroxyl, - $SF_5$, $C_{1-6}$ alkyl, $C_{1-6}$ alkoxy, halo $C_{1-6}$ alkyl, halo $C_{1-6}$ alkoxy, deuterated $C_{1-6}$ alkyl or deuterated $C_{1-6}$ alkoxy; in some embodiments, each $R^5$ is independently selected from D, halogen, cyano, amino, hydroxyl, $C_{1-4}$ alkyl. $C_{1-4}$ alkoxy, halo $C_{1-4}$ alkyl, halo $C_{1-4}$ alkoxy, deuterated $C_{1-4}$ alkyl, or deuterated $C_{1-4}$ alkoxy; in some embodiments, each $R^5$ is independently selected from D, halogen, cyano, amino, hydroxyl, -$SF_5$, $C_{1-2}$ alkyl, $C_{1-2}$ alkoxy, halo $C_{1-2}$ alkyl, halo $C_{1-2}$ alkoxy, deuterated $C_{1-2}$ alkyl or deuterated $C_{1-2}$ alkoxy; in some embodiments, each $R^5$ is independently selected from D, F, Cl, cyano, amino, hydroxyl, $C_{1-2}$ alkyl, $C_{1-2}$ alkoxy, halo $C_{1-2}$ alkyl, halo $C_{1-2}$ alkoxy, deuterated $C_{1-2}$ alkyl, or deuterated $C_{1-2}$ alkoxy; in some embodiments, each $R^5$ is independently selected from D, F, Cl, $C_{1-2}$ alkyl, halo $C_{1-2}$ alkyl, or deuterated $C_{1-2}$ alkyl; in some embodiments, each $R^5$ is independently selected from D, F, Cl, methyl, ethyl, -$CH_2F$, -$CHF_2$, -$CF_3$, -$CH_2CH_2F$, -$CH_2CHF_2$, -$CH_2CF_3$, -$CHFCH_2F$, -$CHFCHF_2$, -$CHFCF_3$, -$CF_2CH_2F$, -$CF_2CHF_2$, -$CF_2CF_3$, - $CH_2D$, -$CHD_2$, -$CD_3$, -$CH_2CH_2D$, -$CH_2CHD_2$, -$CH_2CD_3$, -$CHDCH_2D$, -$CHDCHD_2$, -$CHDCD_3$, -$CD_2CH_2D$, -$CD_2CHD_2$, or -$CD_2CD_3$;

q is selected from 0, 1, 2 or 3; in some embodiments, q is selected from 0, 1 or 2; in some embodiments, q is selected from 0 or 1; in some embodiments, q is selected from 0;

p is selected from 0, 1, 2 or 3; in some embodiments, p is selected from 0, 1 or 2; in some embodiments, p is selected from 0 or 1; in some embodiments, p is selected from 0;

ring B is 5- to 6-membered saturated monocyclic heterocycloalkane containing 1-2 nitrogen atoms, 5- to 6-membered partially unsaturated monocyclic heterocycloalkane containing 1-2 nitrogen atoms, 6- to 8-membered saturated bridged heterocycle containing 1-4 nitrogen atoms, 5- to 10-membered saturated fused heterocycle containing 1-4 nitrogen atoms, or 5- to 11-membered saturated spiro heterocycle containing 1-4 nitrogen atoms; in some embodiments, ring B is 5-membered saturated monocyclic heterocycloalkane containing 1-2 nitrogen atoms, 6-membered saturated monocyclic heterocycloalkane containing 1-2 nitrogen atoms, 6-membered saturated bridged heterocycle containing 1, 2, 3 or 4 nitrogen atoms, 7-membered saturated bridged heterocycle containing 1, 2, 3 or 4 nitrogen atoms, 8-membered saturated bridged heterocycle containing 1, 2, 3 or 4 nitrogen atoms, 8-membered saturated fused heterocycle containing 1, 2, 3 or 4 nitrogen atoms, 9-membered saturated fused heterocycle containing 1 , 2, 3 or 4 nitrogen atoms, 10-membered saturated fused heterocycle containing 1, 2, 3 or 4 nitrogen atoms, 7-membered saturated spiro heterocycle containing 1, 2, 3 or 4 nitrogen atoms, 8-membered saturated spiro heterocycle containing 1, 2, 3 or 4 nitrogen atoms, 9-membered saturated spiro heterocycle containing 1, 2, 3 or 4 nitrogen atoms, 10-membered saturated spiro heterocycle containing 1, 2, 3 or 4 nitrogen atoms, or 11-membered saturated spiro heterocycle containing 1, 2, 3 or 4 nitrogen atoms; in some embodiments, ring B is 6-membered saturated monocyclic heterocycloalkane containing 1-2 nitrogen atoms, 6-membered saturated bridged heterocycle containing 1, 2, 3 or 4 nitrogen atoms, 7-membered saturated bridged heterocycle containing 1, 2, 3 or 4 nitrogen atoms, 8-membered saturated bridged heterocycle containing 1, 2, 3 or 4 nitrogen atoms, 7-membered saturated spiro heterocycle containing 1, 2, 3 or 4 nitrogen atoms, 9-membered saturated spiro heterocycle containing 1, 2, 3 or 4 nitrogen atoms, or 11-membered saturated spiro heterocycle containing 1, 2, 3 or 4 nitrogen atoms; in some embodiments, ring B is 6-membered saturated monocyclic heterocycloalkane containing 1-2 nitrogen atoms; in some embodiments, ring B is selected from

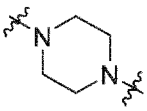
or
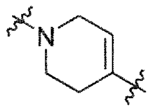
;

ring A is selected from 5- to 6-membered monocyclic heteroaromatic ring containing 1, 2, 3, 4 or 5 nitrogen, oxygen or sulphur atoms, wherein the heteroaromatic ring is further substituted with 1-3 substituents selected from $R_a$; in some embodiments, ring A is selected from 5-membered monocyclic heteroaromatic ring containing 1, 2, 3, 4 or 5 nitrogen, oxygen or sulphur atoms, or 6-membered monocyclic heteroaromatic ring containing 1, 2, 3, 4 or 5 nitrogen, oxygen or sulphur atoms, wherein the heteroaromatic ring is further substituted with 1, 2 or 3 substituents selected from $R_a$; in some embodiments, ring A is selected from 6-membered monocyclic heteroaromatic ring containing 1, 2, 3, 4 or 5 nitrogen, oxygen or sulphur atoms, wherein the heteroaromatic ring is further substituted with 1 substituent selected from $R_a$; in some embodiments, ring A is selected from 6-membered monocyclic heteroaromatic ring containing 1 or 2 nitrogen atoms, wherein the heteroaromatic ring is further substituted with 1 substituent selected from $R_a$; in some embodiments, ring A is selected from 5-membered monocyclic heteroaromatic ring containing 2, 3, 4 or 5 nitrogen, oxygen or sulphur atoms, wherein the heteroaromatic ring is further substituted with 1 substituent selected from $R_a$; or

ring A is selected from 7- to 10-membered bicyclic heteroaromatic ring containing 1-5 nitrogen, oxygen or sulphur atoms, or 7- to 10-membered bicyclic aromatic ring, wherein the heteroaromatic ring or aromatic ring is optionally further substituted with 1-3 substituents selected from $R_b$; in some embodiments, ring A is selected from 8- to 10-membered bicyclic fused heteroaromatic ring containing 1, 2, 3, 4 or 5 nitrogen, oxygen or sulphur atoms, or 8- to 10-membered bicyclic fused aromatic ring, wherein the heteroaromatic ring or aromatic ring is optionally further substituted with 1, 2 or 3 substituents selected from $R_b$; in some embodiments, ring A is selected from 8-membered bicyclic fused heteroaromatic ring containing 1, 2, 3, 4 or 5 nitrogen, oxygen or sulphur atoms, 9-membered bicyclic fused heteroaromatic ring containing 1, 2, 3, 4 or 5 nitrogen, oxygen or sulphur atoms, 10-membered bicyclic fused heteroaromatic ring containing 1, 2, 3, 4 or 5 nitrogen, oxygen or sulphur atoms, 8-membered bicyclic fused aromatic ring, 9-membered bicyclic fused aromatic ring, or 10-membered bicyclic fused aromatic ring, wherein the heteroaromatic ring or aromatic ring is optionally further substituted with 1 substituent selected from $R_b$; in some embodiments, ring A is selected from 8-membered bicyclic fused heteroaromatic ring containing 1, 2, 3, 4 or 5 nitrogen, oxygen or sulphur atoms, 9-membered bicyclic fused heteroaromatic ring containing 1, 2, 3, 4 or 5 nitrogen, oxygen or sulphur atoms, 10-membered bicyclic fused heteroaromatic ring containing 1, 2, 3, 4 or 5 nitrogen, oxygen or sulphur atoms, 8-membered bicyclic fused aromatic ring, 9-membered bicyclic fused aromatic ring, or 10-membered bicyclic fused aromatic ring, wherein the heteroaromatic ring or aromatic ring is optionally further substituted with 1 substituent selected from $R_b$;

in some embodiments, ring A is selected from

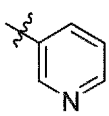
,

which is further substituted with 1-3 substituents selected from $R_a$; or

ring A is selected from 7- to 10-membered bicyclic heteroaromatic ring containing 1-5 nitrogen, oxygen or sulphur atoms, or 7- to 10-membered bicyclic aromatic ring, wherein the heteroaromatic ring or aromatic ring is optionally further substituted with 1-3 substituents selected from $R_b$;

in some embodiments, ring A is selected from 8- to 10-membered bicyclic heteroaromatic ring containing 1-5 nitrogen, oxygen or sulphur atoms, wherein the heteroaromatic ring is optionally further substituted with 1, 2 or 3 substituents selected from $-C(O)N(R^{a1})_2$, $-NHC_{1-2}$ alkyl, $-N(C_{1-2}$ alkyl$)_2$, $C_{1-2}$ alkyl, =O, $C_{1-2}$ alkyl, $C_{3-5}$ cycloalkyl, halo $C_{1-2}$ alkyl, halo $C_{1-2}$ alkoxy, deuterated $C_{1-2}$ alkyl, or deuterated $C_{1-2}$ alkoxy;

$R_a$ is selected from $-C(O)N(R^{a1})_2$, $-NR^{a1}C(O)R^{a1}$, $-C(O)R^{a1}$, $-NR^{a1}$, $-NR^{a1}C(O)OR^{a1}$, $-NR^{a1}C(O)N(R^{a1})_2$, $-C(=S)N(R^{a1})_2$, $-S(O)_2N(R^{a1})_2$, 5- to 6-membered monocyclic heteroaryl containing 1-5 nitrogen, oxygen or sulphur atoms, 4- to 7-membered monocyclic heterocycloalkyl containing 1-4 nitrogen, oxygen or sulphur atoms, or 3- to 7-membered monocyclic cycloalkyl, wherein the heteroaryl, heterocycloalkyl, or cycloalkyl is optionally further substituted with 1-3 substituents selected from D, halogen, cyano, hydroxyl, amino, $-NHC_{1-6}$ alkyl, $-N(C_{1-6}$ alkyl$)_2$, $C_{1-6}$ alkyl, halo $C_{1-6}$ alkyl, $C_{1-6}$ alkoxy, halo $C_{1-6}$ alkoxy, deuterated $C_{1-6}$ alkyl, or deuterated $C_{1-6}$ alkoxy; in some embodiments, $R_a$ is selected from $-C(O)N(R^{a1})_2$, $-NR^{a1}C(O)R^{a1}$, $-C(O)R^{a1}$, $-NR^{a1}$, $-NR^{a1}C(O)OR^{a1}$, $-NR^{a1}C(O)N(R^{a1})_2$, $-C(=S)N(R^{a1})_2$, $-S(O)_2N(R^{a1})_2$, 5-membered monocyclic heteroaryl containing 1, 2, 3, 4 or 5 nitrogen, oxygen or sulphur atoms, 6-membered monocyclic heteroaryl containing 1, 2, 3, 4 or 5 nitrogen, oxygen or sulphur atoms, 4- to 7-membered monocyclic heterocycloalkyl containing 1, 2, 3 or 4 nitrogen, oxygen or sulphur atoms, or 3- to 7-membered monocyclic cycloalkyl, wherein the heteroaryl, heterocycloalkyl, or cycloalkyl is optionally further substituted with 1, 2 or 3 substituents selected from D, halogen, cyano, hydroxyl, amino, $-NHC_{1-2}$ alkyl, $-N(C_{1-2}$ alkyl$)_2$, $C_{1-2}$ alkyl, halo $C_{1-2}$ alkyl, $C_{1-2}$ alkoxy, halo $C_{1-2}$ alkoxy, deuterated $C_{1-2}$ alkyl, or deuterated $C_{1-2}$ alkoxy; in some embodiments, $R_a$ is selected from $-C(O)N(R^{a1})_2$, $-NR^{a1}C(O)R^{a1}$, $-C(O)R^{a1}$, $-NR^{a1}$, $-NR^{a1}C(O)OR^{a1}$, $-NR^{a1}C(O)N(R^{a1})_2$, $-C(=S)N(R^{a1})_2$, $-S(O)_2N(R^{a1})_2$, 5-membered monocyclic heteroaryl containing 1, 2, 3 or 4 nitrogen, oxygen or sulphur atoms, 4-membered monocyclic heterocycloalkyl containing 1 or 2 nitrogen, oxygen or sulphur atoms, 5-membered monocyclic heterocycloalkyl containing 1 or 2 nitrogen, oxygen or sulphur atoms, 6-membered monocyclic heterocycloalkyl containing 1 or 2 nitrogen, oxygen or sulphur atoms, 4-membered monocyclic cycloalkyl, 5-membered monocyclic cycloalkyl, or 6-membered monocyclic cycloalkyl, wherein the heteroaryl, heterocycloalkyl, or cycloalkyl is optionally further substituted with 1, 2 or 3 substituents selected from D, halogen, cyano, hydroxyl, amino, $-NHCH_3$, $-NHCH_2CH_3$, $-N(CH_3)_2$, methyl, ethyl, methoxy, ethoxy, $-CH_2F$, $-CHF_2$, $-CF_3$, $-CH_2CH_2F$, $-CH_2CHF_2$, $-CH_2CF_3$, $-CHFCH_2F$, $-CHFCHF_2$, $-CHFCF_3$, $-CF_2CH_2F$, $-CF_2CHF_2$, $-CF_2CF_3$, $-OCHF_2$, $-OCH_2F$, $-OCF_3$, $-OCH_2CH_2F$, $-OCH_2CHF_2$, $-OCH_2CF_3$, $-OCHFCH_2F$, $-OCHFCHF_2$, $-OCHFCF_3$, $-OCF_2CH_2F$, $-OCF_2CHF_2$, $-OCF_2CF_3$, $-CH_2D$, $-CHD_2$, $-CD_3$, $-CH_2CH_2D$, $-CH_2CHD_2$, $-CH_2CD_3$, $-CHDCH_2D$, $-CHDCHD_2$, $-CHDCD_3$, $-CD_2CH_2D$, $-CD_2CHD_2$, $-CD_2CD_3$, $-OCHD_2$, $-OCH_2D$, $-OCD_3$, $-OCH_2CH_2D$, $-OCH_2CHD_2$, $-OCH_2CD_3$, $-OCHDCH_2D$, $-OCHDCHD_2$, $-OCHDCD_3$, $-OCD_2CH_2D$, $-OCD_2CHD_2$, or $-OCD_2CD_3$; in some embodiments, $R_a$ is selected from $-C(O)N(R^{a1})_2$, $-NR^{a1}C(O)R^{a1}$ or $-C(O)R^{a1}$;

$R_b$ is selected from $-C(O)N(R^{a1})_2$, $-NR^{a1}C(O)OR^{a1}$, $-NR^{a1}C(O)N(R^{a1})_2$, $-C(=S)N(R^{a1})_2$, $-S(O)_2N(R^{a1})_2$, =O, D, halogen, cyano, hydroxyl, amino, $-NHC_{1-6}$ alkyl, $-N(C_{1-6}$ alkyl$)_2$, $C_{1-6}$ alkyl, $C_{3-12}$ cycloalkyl, 3- to 12-membered heterocycloalkyl, $C_{1-6}$ alkoxy, $C_{1-6}$ alkyl-O-$C_{1-6}$ alkyl, halo $C_{1-6}$ alkyl, halo $C_{1-6}$ alkoxy, deuterated $C_{1-6}$ alkyl or deuterated $C_{1-6}$ alkoxy; in some embodiments, $R_b$ is selected from $-C(O)N(R^{a1})_2$, $-NR^{a1}C(O)OR^{a1}$, $-NR^{a1}C(O)N(R^{a1})_2$, $-C(=S)N(R^{a1})_2$, $-S(O)_2N(R^{a1})_2$, =O, D, halogen, cyano, hydroxyl, amino, $-NHC_{1-2}$ alkyl, $-N(C_{1-2}$ alkyl$)_2$, $C_{1-2}$ alkyl, $C_{1-2}$ alkoxy, $C_{1-2}$ alkyl-O-$C_{1-2}$ alkyl, halo $C_{1-2}$ alkyl, halo $C_{1-2}$ alkoxy, deuterated $C_{1-2}$ alkyl or deuterated $C_{1-2}$ alkoxy; in some embodiments, $R_b$ is selected from $-C(O)N(R^{a1})_2$, =O, D, halogen, cyano, hydroxyl, amino, $-NHC_{1-2}$ alkyl, $-N(C_{1-2}$ alkyl$)_2$, $C_{1-2}$ alkyl, $C_{1-2}$ alkoxy, $C_{1-2}$ alkyl-O-$C_{1-2}$ alkyl, halo $C_{1-2}$ alkyl, halo $C_{1-2}$ alkoxy, deuterated $C_{1-2}$ alkyl or deuterated $C_{1-2}$ alkoxy; in some embodiments, $R_b$ is selected from $-C(O)N(R^{a1})_2$, =O, D, halogen, cyano, hydroxyl, amino, $-NHC_{1-2}$ alkyl, $-N(C_{1-2}$ alkyl$)_2$, $C_{1-2}$ alkyl, or halo $C_{1-2}$ alkyl; in some embodiments, $R_b$ is selected from $-C(O)N(R^{a1})_2$, =O, D, halogen, cyano, hydroxyl, amino, $-NHCH_3$, $-NHCH_2CH_3$, $-N(CH_3)_2$, methyl, ethyl, $-CH_2F$, $-CHF_2$, $-CF_3$, $-CH_2CH_2F$, $-CH_2CHF_2$, $-CH_2CF_3$, $-CHFCH_2F$, $-CHFCHF_2$, or $-CHFCF_3$; in some embodiments, $R_b$ is selected from D, halogen, cyano, hydroxyl, amino, $C_{1-2}$ alkyl, $C_{3-5}$ cycloalkyl, 3- to 5-membered heterocycloalkyl, $C_{1-2}$ alkoxy, $C_{1-2}$ alkyl-O-$C_{1-2}$ alkyl, halo $C_{1-2}$ alkyl, halo $C_{1-2}$ alkoxy, deuterated $C_{1-2}$ alkyl, or deuterated $C_{1-2}$ alkoxy;

each $R^{a1}$ is independently selected from H, D, $C_{1-6}$ alkyl, $C_{3-12}$ cycloalkyl, 3- to 12-membered heterocycloalkyl, 5- to 10-membered heteroaryl, $C_{1-6}$ alkoxy, $C_{1-6}$ alkyl-O-$C_{1-6}$ alkyl, $C_{1-6}$ alkyl-O-$C_{3-12}$ cycloalkyl, halo $C_{1-6}$ alkyl, halo $C_{1-6}$ alkoxy, deuterated $C_{1-6}$ alkyl, or deuterated $C_{1-6}$ alkoxy, wherein the alkyl, cycloalkyl, heterocycloalkyl, or heteroaryl is optionally further substituted with 1-3 substituents selected from D, halogen, cyano, hydroxyl, amino, $C_{1-6}$ alkyl, halo $C_{1-6}$ alkyl, deuterated $C_{1-6}$ alkyl, $C_{3-6}$ cycloalkyl or phenyl; in some embodiments, each $R^{a1}$ is independently selected from $C_{1-4}$ alkyl, $C_{3-5}$ cycloalkyl, $C_{5-9}$ bicyclic spiro cycloalkyl, 4- to 6-membered heterocycloalkyl, 5- to 9-membered bicyclic spiro heterocycloalkyl, 5- to 10-membered heteroaryl, $C_{1-4}$ alkoxy, $C_{1-2}$ alkyl-O-$C_{1-2}$ alkyl, $C_{1-4}$ alkyl-O-$C_{3-5}$ cycloalkyl, halo $C_{1-4}$ alkyl, halo $C_{1-4}$ alkoxy, deuterated $C_{1-4}$ alkyl, or deuterated $C_{1-4}$

alkoxy, wherein the alkyl, cycloalkyl, heterocycloalkyl, or heteroaryl is optionally further substituted with 1-3 substituents selected from D, halogen, cyano, hydroxyl, amino, $C_{1-2}$ alkyl, halo $C_{1-2}$ alkyl, deuterated $C_{1-2}$ alkyl, $C_{3-4}$ cycloalkyl or phenyl; in some embodiments, each $R^{a1}$ is independently selected from $C_{1-4}$ alkyl, $C_{3-5}$ cycloalkyl, $C_{5-9}$ bicyclic spiro cycloalkyl, 4- to 6-membered heterocycloalkyl, 5- to 9-membered bicyclic spiro heterocycloalkyl, 5- to 10-membered heteroaryl. $C_{1-4}$ alkoxy, $C_{1-2}$ alkyl-O-$C_{1-2}$ alkyl, $C_{1-4}$ alkyl-O-$C_{3-6}$ cycloalkyl, halo $C_{1-4}$ alkyl, halo $C_{1-4}$ alkoxy, deuterated $C_{1-4}$ alkyl, or deuterated $C_{1-4}$ alkoxy, wherein the alkyl, cycloalkyl, heterocycloalkyl, or heteroaryl is optionally further substituted with 1-3 substituents selected from D, F, Cl, cyano, hydroxyl, amino, $C_{1-2}$ alkyl, halo $C_{1-2}$ alkyl, deuterated $C_{1-2}$ alkyl, $C_{3-4}$ cycloalkyl or phenyl; in some embodiments, $R^{a1}$ is selected from $C_{1-4}$ alkyl, $C_{3-5}$ cycloalkyl, $C_{5-9}$ bicyclic spiro cycloalkyl, 4- to 6-membered heterocycloalkyl, 5- to 9-membered bicyclic spiro heterocycloalkyl, 5- to 6-membered heteroaryl, $C_{1-4}$ alkoxy, $C_{1-2}$ alkyl-O-$C_{1-2}$ alkyl, $C_{1-2}$ alkyl-O-$C_{3-5}$ cycloalkyl, halo $C_{1-4}$ alkyl, halo $C_{1-4}$ alkoxy, deuterated $C_{1-4}$ alkyl, or deuterated $C_{1-4}$ alkoxy, wherein the alkyl, cycloalkyl, heterocycloalkyl, or heteroaryl is optionally further substituted with 1-3 substituents selected from D, halogen, cyano, hydroxyl, amino, $C_{1-2}$ alkyl, halo $C_{1-2}$ alkyl, deuterated $C_{1-2}$ alkyl, $C_{3-5}$ cycloalkyl or phenyl; in some embodiments, each $R^{a1}$ is independently selected from methyl, ethyl, propyl, isopropyl, tert-butyl, cyclopropyl, cyclobutyl, methoxymethyl, ethoxymethyl, methoxyethyl, ethoxyethyl, cyclopropyloxyethyl, difluorocyclopropyloxyethyl, $-CH_2F$, $-CHF_2$, $-CF_3$, $-CH_2CH_2F$, $-CH_2CHF_2$, $-CH_2CF_3$, $-CHFCH_2F$, $-CHFCHF_2$, $-CHFCF_3$, $-CF_2CH_2F$, $-CF_2CHF_2$, $-CF_2CF_3$, $-CH_2CH_2CH_2F$, $-CH_2CH_2CHF_2$, $-CH_2CH_2CF_3$,

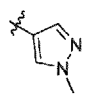,

$-CH_2$-cyclopropyl,

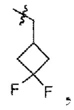,

fluorocyclopropyl, difluorocyclopropyl, difluorocyclobutyl,

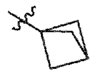 or 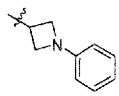;

in some embodiments, each $R^{a1}$ is independently selected from H, D, $C_{1-6}$ alkyl, $C_{3-12}$ cycloalkyl, 3- to 12-membered heterocycloalkyl, 5- to 10-membered heteroaryl, $C_{1-6}$ alkoxy, $C_{1-6}$ alkyl-O-$C_{1-6}$ alkyl, halo $C_{1-6}$ alkyl, halo $C_{1-6}$ alkoxy, deuterated $C_{1-6}$ alkyl, or deuterated $C_{1-6}$ alkoxy, wherein the alkyl, cycloalkyl, heterocycloalkyl, or heteroaryl is optionally further substituted with 1-3 substituents selected from D, halogen, cyano, hydroxyl, amino, $C_{1-6}$ alkyl, or $C_{3-6}$ cycloalkyl; in some embodiments, each $R^{a1}$ is independently selected from $C_{1-4}$ alkyl, $C_{3-5}$ cycloalkyl, $C_{5-9}$ bicyclic spiro cycloalkyl, 4- to 6-membered heterocycloalkyl, 5- to 9-membered bicyclic spiro heterocycloalkyl, 5- to 10-membered heteroaryl, $C_{1-4}$ alkoxy, $C_{1-2}$ alkyl-O-$C_{1-2}$ alkyl, halo $C_{1-4}$ alkyl, halo $C_{1-4}$ alkoxy, deuterated $C_{1-4}$ alkyl, or deuterated $C_{1-4}$ alkoxy, wherein the alkyl, cycloalkyl, heterocycloalkyl, or heteroaryl is optionally further substituted with 1-3 substituents selected from D, halogen, cyano, hydroxyl, amino, $C_{1-2}$ alkyl, or $C_{3-4}$ cycloalkyl; in some embodiments, each $R^{a1}$ is independently selected from H, D, $C_{1-6}$ alkyl, $C_{3-12}$ cycloalkyl, 3- to 12-membered heterocycloalkyl, $C_{1-6}$ alkoxy, $C_{1-6}$ alkyl-O-$C_{1-6}$ alkyl, halo $C_{1-6}$ alkyl, halo $C_{1-6}$ alkoxy, deuterated $C_{1-6}$ alkyl or deuterated $C_{1-6}$ alkoxy; in some embodiments, each $R^{a1}$ is independently selected from H, D, $C_{1-4}$ alkyl, $C_{3-6}$ monocyclic cycloalkyl, $C_{5-11}$ bicyclic spiro cycloalkyl, $C_{6-8}$ bicyclic bridged cycloalkyl, $C_{7-10}$ bicyclic fused cycloalkyl, 4- to 6-membered heterocycloalkyl, 6- to 9-membered bicyclic spiro heterocycloalkyl, $C_{6-8}$ bicyclic bridged heterocycloalkyl, $C_{7-10}$ bicyclic fused heterocycloalkyl, $C_{1-4}$ alkoxy, $C_{1-2}$ alkyl-O-$C_{1-2}$ alkyl, halo $C_{1-4}$ alkyl, halo $C_{1-4}$ alkoxy, deuterated $C_{1-4}$ alkyl, or deuterated $C_{1-4}$ alkoxy; in some embodiments, each $R^{a1}$ is independently selected from $C_{1-4}$ alkyl, $C_{3-6}$ monocyclic cycloalkyl, $C_{5-7}$ bicyclic spiro cycloalkyl, 4- to 6-membered heterocycloalkyl, $C_{1-2}$ alkyl-O-$C_{1-2}$ alkyl, halo $C_{1-4}$ alkyl, or deuterated $C_{1-4}$ alkyl; in some embodiments, each $R^{a1}$ is independently selected from H, D, $C_{1-2}$ alkyl, $C_{3-5}$ cycloalkyl, 3- to 5-membered heterocycloalkyl, 5- to 6-membered heteroaryl, halo $C_{1-2}$ alkyl, halo $C_{1-2}$ alkoxy, deuterated $C_{1-2}$ alkyl, or deuterated $C_{1-2}$ alkoxy, wherein the cycloalkyl, heterocycloalkyl, or heteroaryl is optionally further substituted with 1-3 substituents selected from D, halogen, cyano, hydroxyl, amino, $C_{1-2}$ alkyl, halo

$C_{1-2}$ alkyl, deuterated $C_{1-2}$ alkyl, or $C_{3-4}$ cycloalkyl; in some embodiments, each $R^{a1}$ is independently selected from $C_{1-4}$ alkyl, $C_{3-5}$ cycloalkyl, or 5- to 6-membered heteroaryl, wherein the alkyl, cycloalkyl, or heteroaryl is optionally further substituted with 1, 2 or 3 substituents selected from D, F, Cl, cyano, hydroxyl, amino, $C_{1-2}$ alkyl, halo $C_{1-2}$ alkyl, or deuterated $C_{1-2}$ alkyl; in some embodiments, each $R^{a1}$ is independently selected from $C_{1-4}$ alkyl, $C_{3-4}$ monocyclic cycloalkyl, $C_{1-2}$ alkyl-O-$C_{1-2}$ alkyl, halo $C_{1-4}$ alkyl, or deuterated $C_{1-4}$ alkyl; in some embodiments, each $R^{a1}$ is independently selected from methyl, ethyl, propyl, isopropyl, tert-butyl, cyclopropyl, cyclobutyl, methoxymethyl, ethoxymethyl, methoxyethyl, ethoxyethyl, $-CH_2F$, $-CHF_2$, $-CF_3$, $-CH_2CH_2F$, $-CH_2CHF_2$, $-CH_2CF_3$, $-CHFCH_2F$, $-CHFCHF_2$, - $CHFCF_3$, $-CF_2CH_2F$, $-CF_2CHF_2$, $-CF_2CF_3$, $-CH_2CH_2CH_2F$, $-CH_2CH_2CHF_2$, or - $CH_2CH_2CF_3$;

unless otherwise specified, the above-mentioned heterocycloalkane, heterocycloalkyl, heteroaryl, or heteroaromatic ring contains 1, 2, 3, 4 or 5 heteroatoms selected from nitrogen, oxygen or sulphur; in some embodiments, the heterocycloalkane, heterocycloalkyl, heteroaryl, or heteroaromatic ring contains 1, 2, 3 or 4 heteroatoms selected from nitrogen, oxygen or sulphur; the heterocycloalkane, heterocycloalkyl, heteroaryl, or heteroaromatic ring contains 1, 2 or 3 heteroatoms selected from nitrogen, oxygen or sulphur; the heterocycloalkane, heterocycloalkyl, heteroaryl, or heteroaromatic ring contains 1 or 2 heteroatoms selected from nitrogen, oxygen or sulphur.

The present invention provides a compound as shown in formula (I), or a stereoisomer, solvate, deuterated compound, or pharmaceutically acceptable salt thereof, wherein in some embodiments, when - - - - - - - represents a single bond, $X^1$, $X^2$ and $X^3$ are all selected from $CR^x$, and v is selected from 1, X is selected from $C(R^x)_2$ or $NR^x$.

The present invention provides a compound as shown in formula (I), or a stereoisomer, solvate, deuterated compound, or pharmaceutically acceptable salt thereof, wherein in some embodiments, the following conditions are met: (1) when - - - - - - - represents a single bond, $X^1$, $X^2$ and $X^3$ are all selected from $CR^x$, and v is selected from 1, X is selected from $C(R^x)_2$ or $NR^x$; (2) when

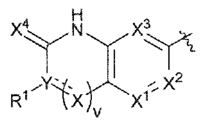

is selected from

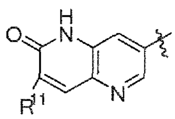 , 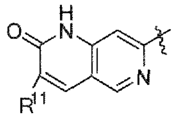 , 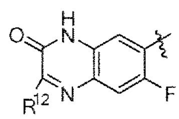 and 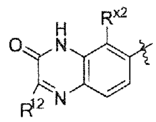 ,

wherein $R^{x2}$ is selected from H, $C_{1-6}$ alkyl or halogen, $R^{12}$ is selected from H, $C_{1-6}$ alkyl, halo $C_{1-6}$ alkyl, $C_{1-6}$ alkoxy or $C_{3-6}$ cycloalkyl, $R^{11}$ is selected from $C_{1-6}$ alkyl, and $R^2$ is selected from H or D, $R^3$ is not selected from H or D.

The present invention provides a compound as shown in formula (I), or a stereoisomer, solvate, deuterated compound, or pharmaceutically acceptable salt thereof, wherein in some embodiments, the following conditions are met: (1) when - - - - - - - represents a single bond, $X^1$, $X^2$ and $X^3$ are all selected from $CR^x$, and v is selected from 1, X is selected from $C(R^x)_2$ or $NR^x$; (2) when - - - - - - - represents a double bond, and v is selected from 1, X, $X^1$, $X^2$ and $X^3$ are not all selected from $CR^x$; (3) when $R^2$ is selected from H or D and $R^3$ is selected from H or D, $X^4$ is selected from O, $X^3$ is selected from CH, - - - - - - - represents a double bond, v is selected from 1, and Y is selected from C, $R^1$ is not $C_{1-6}$ alkyl and halo $C_{1-6}$ alkyl.

The present invention provides a compound, or a stereoisomer, solvate, deuterated compound, or pharmaceutically acceptable salt thereof, wherein the selection of groups follows the rules of chemical bonding.

Specifically, the first technical solution of the present invention provides a compound as shown in formula (I), or a stereoisomer, solvate, deuterated compound, or pharmaceutically acceptable salt thereof,

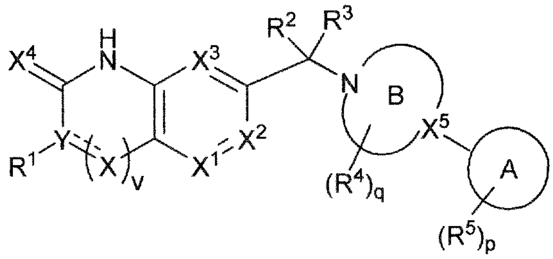

(I)

wherein X is selected from $CR^x$, $C(R^x)_2$, O, N or $NR^x$;

Y is selected from N, C or CH;

------- represents a single bond or a double bond;

v is selected from 1, 2 or 3;

$X^1$, $X^2$ and $X^3$ are each independently selected from N or $CR^x$;

provided that when ------- represents a double bond, and v is selected from 1, X, $X^1$, $X^2$ and $X^3$ are not all selected from $CR^x$;

$X^4$ is selected from O or S;

$X^5$ is selected from N or $CR^x$;

each $R^x$ is independently selected from H, D, halogen, cyano, amino, hydroxyl, $-SF_5$, $C_{1-6}$ alkyl, halo $C_{1-6}$ alkyl, halo $C_{1-6}$ alkoxy, deuterated $C_{1-6}$ alkyl, deuterated $C_{1-6}$ alkoxy, $C_{1-6}$ alkoxy, $C_{2-6}$ alkenyl, $C_{2-6}$ alkynyl, $C_{1-6}$ alkyl-O-$C_{1-6}$ alkyl, $-(CH_2)_r-C_{3-12}$ cycloalkyl or $-(CH_2)_r$-(3- to 12-membered heterocycloalkyl); or two $R^x$ on the same carbon atom together form =O;

$R^1$ is selected from halogen, nitro, cyano, amino, hydroxyl, $-SF_5$, $C_{1-6}$ alkyl, $C_{1-6}$ alkoxy, $C_{2-6}$ alkenyl, $C_{2-6}$ alkynyl, $C_{1-6}$ alkyl-O-$C_{1-6}$ alkyl, $-(CH_2)_r-C_{3-12}$ cycloalkyl or $-(CH_2)_r$-(3- to 12-membered heterocycloalkyl), wherein the alkyl, alkoxy, alkenyl, alkynyl, cycloalkyl or heterocycloalkyl is optionally further substituted with 1-3 groups selected from D, halogen, cyano, amino, hydroxyl, $C_{1-6}$ alkyl or $C_{1-6}$ alkoxy;

each r is independently selected from 0, 1, 2 or 3;

$R^2$ and $R^3$ are each independently selected from H, D, halogen, cyano, amino, hydroxyl, $C_{1-6}$ alkyl-O-$C_{1-6}$ alkyl, hydroxy $C_{1-6}$ alkyl, $C_{1-6}$ alkoxy, halo $C_{1-6}$ alkyl, halo $C_{1-6}$ alkoxy, deuterated $C_{1-6}$ alkyl, deuterated $C_{1-6}$ alkoxy or $C_{1-6}$ alkyl; or $R^2$ and $R^3$ together with the carbon atom to which they are attached form $C_{3-5}$ cycloalkyl or 4- to 5-membered heterocycloalkyl;

each $R^4$ is independently selected from D, halogen, cyano, amino, hydroxyl, - $SF_5$, $C_{1-6}$ alkyl, $C_{1-6}$ alkoxy, halo $C_{1-6}$ alkyl, halo $C_{1-6}$ alkoxy, deuterated $C_{1-6}$ alkyl or deuterated $C_{1-6}$ alkoxy; or two $R^4$ on the same carbon atom together with the carbon atom to which they are attached form =O;

each $R^5$ is independently selected from D, halogen, cyano, amino, hydroxyl, - $SF_5$, $C_{1-6}$ alkyl, $C_{1-6}$ alkoxy, halo $C_{1-6}$ alkyl, halo $C_{1-6}$ alkoxy, deuterated $C_{1-6}$ alkyl or deuterated $C_{1-6}$ alkoxy;

q is selected from 0, 1, 2 or 3; p is selected from 0, 1, 2 or 3;

ring B is 5- to 6-membered saturated monocyclic heterocycloalkane containing 1-2 nitrogen atoms, 5- to 6-membered partially unsaturated monocyclic heterocycloalkane containing 1-2 nitrogen atoms, 6- to 8-membered saturated bridged heterocycle containing 1-4 nitrogen atoms, 5- to 10-membered saturated fused heterocycle containing 1-4 nitrogen atoms, or 5- to 11-membered saturated spiro heterocycle containing 1-4 nitrogen atoms;

ring A is selected from 5- to 6-membered monocyclic heteroaromatic ring containing 1-5 nitrogen, oxygen or sulphur atoms, wherein the heteroaromatic ring is further substituted with 1-3 substituents selected from $R_a$; or

ring A is selected from 7- to 10-membered bicyclic heteroaromatic ring containing 1-5 nitrogen, oxygen or sulphur atoms, or 7- to 10-membered bicyclic aromatic ring, wherein the heteroaromatic ring or aromatic ring is optionally further substituted with 1-3 substituents selected from $R_b$;

$R_a$ is selected from $-C(O)N(R^{a1})_2$, $-NR^{a1}C(O)R^{a1}$, $-C(O)R^{a1}$, $-NR^{a1}$, - $NR^{a1}C(O)OR^{a1}$, $-NR^{a1}C(O)N(R^{a1})_2$, $-C(=S)N(R^{a1})_2$, $-S(O)_2N(R^{a1})_2$, 5- to 6-membered monocyclic heteroaryl containing 1-5 nitrogen, oxygen or sulphur atoms, 4- to 7-membered monocyclic heterocycloalkyl containing 1-4 nitrogen, oxygen or sulphur atoms, or 3- to 7-membered monocyclic cycloalkyl, wherein the heteroaryl, heterocycloalkyl, or cycloalkyl is optionally further substituted with 1-3 substituents selected from D, halogen, cyano, hydroxyl, amino, $-NHC_{1-6}$ alkyl, $-N(C_{1-6}$ alkyl$)_2$, $C_{1-6}$ alkyl, halo $C_{1-6}$ alkyl, $C_{1-6}$ alkoxy, halo $C_{1-6}$ alkoxy, deuterated $C_{1-6}$ alkyl, or deuterated $C_{1-6}$ alkoxy;

$R_b$ is selected from $-C(O)N(R^{a1})_2$, $-NR^{a1}C(O)OR^{a1}$, $-NR^{a1}C(O)N(R^{a1})_2$, - $C(=S)N(R^{a1})_2$, $-S(O)_2N(R^{a1})_2$, =O. D, halogen, cyano, hydroxyl, amino, $-NHC_{1-6}$ alkyl, $-N(C_{1-6}$ alkyl$)_2$, $C_{1-6}$ alkyl, $C_{3-12}$ cycloalkyl, 3- to 12-membered heterocycloalkyl, $C_{1-6}$ alkoxy, $C_{1-6}$ alkyl-O-$C_{1-6}$ alkyl, halo $C_{1-6}$ alkyl, halo $C_{1-6}$ alkoxy, deuterated $C_{1-6}$ alkyl or deuterated $C_{1-6}$ alkoxy;

each $R^{a1}$ is independently selected from H, D, $C_{1-6}$ alkyl, $C_{3-12}$ cycloalkyl, 3- to 12-membered heterocycloalkyl, 5- to 10-membered heteroaryl, $C_{1-6}$ alkoxy, $C_{1-6}$ alkyl-O-$C_{1-6}$ alkyl, $C_{1-6}$ alkyl-O-$C_{3-12}$ cycloalkyl, halo $C_{1-6}$ alkyl, halo $C_{1-6}$ alkoxy, deuterated $C_{1-6}$ alkyl, or deuterated $C_{1-6}$ alkoxy, wherein the alkyl, cycloalkyl, heterocycloalkyl, or heteroaryl is optionally further substituted with 1-3 substituents selected from D, halogen, cyano, hydroxyl, amino, $C_{1-6}$ alkyl, halo $C_{1-6}$ alkyl, deuterated $C_{1-6}$ alkyl, $C_{3-6}$ cycloalkyl or phenyl;
provided that:

(1) when ------- represents a single bond, $X^1$, $X^2$ and $X^3$ are all selected from $CR^x$, and v is selected from 1, X is selected from $C(R^x)_2$ or $NR^x$;
(2) when

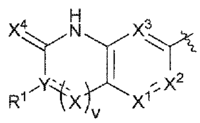

is selected from

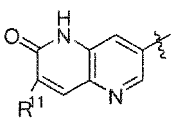,
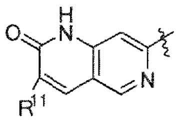,

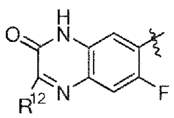 and 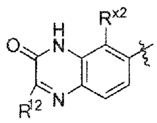,

wherein $R^{x2}$ is selected from H, $C_{1-6}$ alkyl or halogen, $R^{12}$ is selected from H, $C_{1-6}$ alkyl, halo $C_{1-6}$ alkyl, $C_{1-6}$ alkoxy or $C_{3-6}$ cycloalkyl, $R^{11}$ is selected from $C_{1-6}$ alkyl, and $R^2$ is selected from H or D, $R^3$ is not selected from H or D;

unless otherwise specified, the above-mentioned heterocycloalkane, heterocycloalkyl, heteroaryl, or heteroaromatic ring contains 1-5 heteroatoms selected from nitrogen, oxygen or sulphur.

The present invention provides a compound as shown in formula (I), or a stereoisomer, solvate, deuterated compound, or pharmaceutically acceptable salt thereof, wherein

wherein X is selected from CRX, $C(R^x)_2$, O, N or $NR^x$;
Y is selected from N, C or CH;
------- represents a single bond or a double bond;
v is selected from 1, 2 or 3;
$X^1$, $X^2$ and $X^3$ are each independently selected from N or $CR^x$;
provided that when ------- represents a double bond, and v is selected from 1, X, $X^1$, $X^2$ and $X^3$ are not all selected from $CR^x$;
$X^4$ is selected from O or S;
$X^5$ is selected from N or $CR^x$;
each $R^x$ is independently selected from H, D, halogen, cyano, amino, hydroxyl, $-SF_5$, $C_{1-6}$ alkyl, halo $C_{1-6}$ alkyl, halo $C_{1-6}$ alkoxy, deuterated $C_{1-6}$ alkyl, deuterated $C_{1-6}$ alkoxy, $C_{1-6}$ alkoxy, $C_{2-6}$ alkenyl, $C_{2-6}$ alkynyl, $C_{1-6}$ alkyl-O-$C_{1-6}$ alkyl, $-(CH_2)_r$ $C_{3-12}$ cycloalkyl or $-(CH_2)_r$-(3- to 12-membered heterocycloalkyl); or two $R^x$ on the same carbon atom together form =O;
$R^1$ is selected from halogen, nitro, cyano, amino, hydroxyl, $-SF_5$, $C_{1-6}$ alkyl, $C_{1-6}$ alkoxy, $C_{2-6}$ alkenyl, $C_{2-6}$ alkynyl, $C_{1-6}$ alkyl-O-$C_{1-6}$ alkyl, $-(CH_2)_r$-$C_{3-12}$ cycloalkyl or $-(CH_2)_r$-(3- to 12-membered heterocycloalkyl), wherein the alkyl, alkoxy, alkenyl, alkynyl, cycloalkyl or heterocycloalkyl is optionally further substituted with 1-3 groups selected from D, halogen, cyano, amino, hydroxyl, $C_{1-6}$ alkyl or $C_{1-6}$ alkoxy;
each r is independently selected from 0, 1, 2 or 3;

$R^2$ and $R^3$ are each independently selected from H, D, halogen, cyano, amino, hydroxyl, $C_{1-6}$ alkyl-O-$C_{1-6}$ alkyl, hydroxy $C_{1-6}$ alkyl, $C_{1-6}$ alkoxy, halo $C_{1-6}$ alkyl, halo $C_{1-6}$ alkoxy, deuterated $C_{1-6}$ alkyl, deuterated $C_{1-6}$ alkoxy or $C_{1-6}$ alkyl; or $R^2$ and $R^3$ together with the carbon atom to which they are attached form $C_{3-5}$ cycloalkyl or 4- to 5-membered heterocycloalkyl;

each $R^4$ is independently selected from D, halogen, cyano, amino, hydroxyl, - $SF_5$, $C_{1-6}$ alkyl, $C_{1-6}$ alkoxy, halo $C_{1-6}$ alkyl, halo $C_{1-6}$ alkoxy, deuterated $C_{1-6}$ alkyl or deuterated $C_{1-6}$ alkoxy; or two $R^4$ on the same carbon atom together with the carbon atom to which they are attached form =O;

each $R^5$ is independently selected from D, halogen, cyano, amino, hydroxyl, - $SF_5$, $C_{1-6}$ alkyl, $C_{1-6}$ alkoxy, halo $C_{1-6}$ alkyl, halo $C_{1-6}$ alkoxy, deuterated $C_{1-6}$ alkyl or deuterated $C_{1-6}$ alkoxy;

q is selected from 0, 1, 2 or 3; p is selected from 0, 1, 2 or 3;

ring B is 5- to 6-membered saturated monocyclic heterocycloalkane containing 1-2 nitrogen atoms, 5- to 6-membered partially unsaturated monocyclic heterocycloalkane containing 1-2 nitrogen atoms, 6- to 8-membered saturated bridged heterocycle containing 1-4 nitrogen atoms, 5- to 10-membered saturated fused heterocycle containing 1-4 nitrogen atoms, or 5- to 11-membered saturated spiro heterocycle containing 1-4 nitrogen atoms;

ring A is selected from 5- to 6-membered monocyclic heteroaromatic ring containing 1-5 nitrogen, oxygen or sulphur atoms, wherein the heteroaromatic ring is further substituted with 1-3 substituents selected from $R_a$; or

ring A is selected from 7- to 10-membered bicyclic heteroaromatic ring containing 1-5 nitrogen, oxygen or sulphur atoms, or 7- to 10-membered bicyclic aromatic ring, wherein the heteroaromatic ring or aromatic ring is optionally further substituted with 1-3 substituents selected from $R_b$;

$R_a$ is selected from $-C(O)N(R^{a1})_2$, $-NR^{a1}C(O)OR^{a1}$, $-NR^{a1}C(O)N(R^{a1})_2$, - $C(=S)N(R^{a1})_2$, $-S(O)_2N(R^{a1})_2$, 5- to 6-membered monocyclic heteroaryl containing 1-5 nitrogen, oxygen or sulphur atoms, 4- to 7-membered monocyclic heterocycloalkyl containing 1-4 nitrogen, oxygen or sulphur atoms, or 3- to 7-membered monocyclic cycloalkyl, wherein the heteroaryl, heterocycloalkyl, or cycloalkyl is optionally further substituted with 1-3 substituents selected from D, halogen, cyano, hydroxyl, amino, $-NHC_{1-6}$ alkyl, $-N(C_{1-6}\text{ alkyl})_2$, $C_{1-6}$ alkyl, halo $C_{1-6}$ alkyl, $C_{1-6}$ alkoxy, halo $C_{1-6}$ alkoxy, deuterated $C_{1-6}$ alkyl or deuterated $C_{1-6}$ alkoxy;

$R_b$ is selected from $-C(O)N(R^{a1})_2$, $-NR^{a1}C(O)OR^{a1}$, $-NR^{a1}C(O)N(R^{a1})_2$, - $C(=S)N(R^{a1})_2$, $-S(O)_2N(R^{a1})_2$, =O, D, halogen, cyano, hydroxyl, amino, $-NHC_{1-6}$ alkyl, $-N(C_{1-6}\text{ alkyl})_2$, $C_{1-6}$ alkyl, $C_{3-12}$ cycloalkyl, 3- to 12-membered heterocycloalkyl, $C_{1-6}$ alkoxy, $C_{1-6}$ alkyl-O-$C_{1-6}$ alkyl, halo $C_{1-6}$ alkyl, halo $C_{1-6}$ alkoxy, deuterated $C_{1-6}$ alkyl or deuterated $C_{1-6}$ alkoxy;

each $R^{a1}$ is independently selected from H, D, $C_{1-6}$ alkyl, $C_{3-12}$ cycloalkyl, 3- to 12-membered heterocycloalkyl, 5- to 10-membered heteroaryl, $C_{1-6}$ alkoxy, $C_{1-6}$ alkyl-O-$C_{1-6}$ alkyl, halo $C_{1-6}$ alkyl, halo $C_{1-6}$ alkoxy, deuterated $C_{1-6}$ alkyl, or deuterated $C_{1-6}$ alkoxy, wherein the alkyl, cycloalkyl, heterocycloalkyl, or heteroaryl is optionally further substituted with 1-3 substituents selected from D, halogen, cyano, hydroxyl, amino, $C_{1-6}$ alkyl, or $C_{3-6}$ cycloalkyl; or each $R^{a1}$ is independently selected from H, D, $C_{1-6}$ alkyl, $C_{3-12}$ cycloalkyl, 3- to 12-membered heterocycloalkyl, $C_{1-6}$ alkoxy, $C_{1-6}$ alkyl-O-$C_{1-6}$ alkyl, halo $C_{1-6}$ alkyl, halo $C_{1-6}$ alkoxy, deuterated $C_{1-6}$ alkyl or deuterated $C_{1-6}$ alkoxy;

provided that when - - - - - - - represents a single bond, $X^1$, $X^2$ and $X^3$ are all selected from $CR^x$, and v is selected from 1, X is selected from $C(R^x)_2$ or $NR^x$;

unless otherwise specified, the above-mentioned heterocycloalkane, heterocycloalkyl, heteroaryl, or heteroaromatic ring contains 1-5 heteroatoms selected from nitrogen, oxygen or sulphur.

The present invention provides a compound as shown in formula (I), or a stereoisomer, solvate, deuterated compound, or pharmaceutically acceptable salt thereof, wherein in some embodiments, the following conditions are also met: when

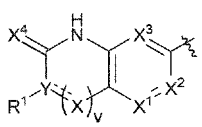

is selected from

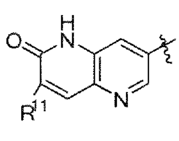, 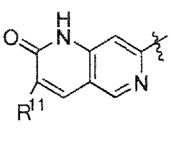, 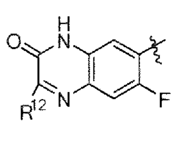

and

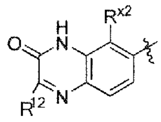,

wherein $R^{x2}$ is selected from H, $C_{1-6}$ alkyl or halogen, $R^{12}$ is selected from H, $C_{1-6}$ alkyl, halo $C_{1-6}$ alkyl, $C_{1-6}$ alkoxy or $C_{3-6}$ cycloalkyl, $R^{11}$ is selected from $C_{1-6}$ alkyl, and $R^2$ is selected from H or D, $R^3$ is not selected from H or D.

The second technical solution of the present invention relates to the compound as shown in formula (I), or the stereoisomer, solvate, deuterated compound, or pharmaceutically acceptable salt thereof according to the present invention, wherein

$R^2$ and $R^3$ are each independently selected from H, D, halogen, cyano, amino, hydroxyl, $C_{1-6}$ alkyl-O-$C_{1-6}$ alkyl, hydroxy $C_{1-6}$ alkyl, $C_{1-6}$ alkoxy, halo $C_{1-6}$ alkyl, halo $C_{1-6}$ alkoxy, deuterated $C_{1-6}$ alkyl, deuterated $C_{1-6}$ alkoxy or $C_{1-6}$ alkyl; or $R^2$ and $R^3$ together with the carbon atom to which they are attached form $C_{3-5}$ cycloalkyl or 4- to 5-membered heterocycloalkyl; provided that when $R^2$ is selected from H or D, $R^3$ is not selected from H or D;
other groups are consistent with those of any one of the preceding technical solutions.

The third technical solution of the present invention relates to the compound as shown in formula (I), or the stereoisomer, solvate, deuterated compound, or pharmaceutically acceptable salt thereof according to the present invention, having a structure of formula (II) or (II-a):

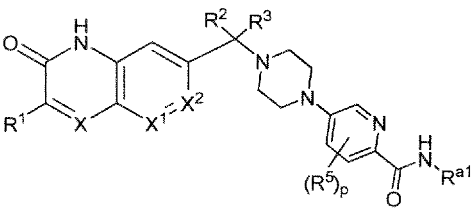 (II) 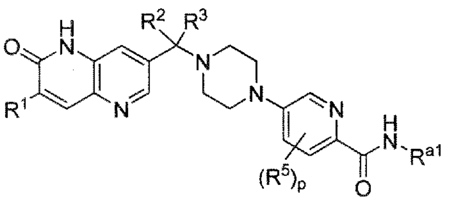 (II-a)

wherein X is selected from $CR^x$ or N;

$X^1$ and $X^2$ are each independently selected from N or $CR^x$;
p is selected from 0 or 1;
$R^x$ is independently selected from H, D, halogen, cyano, amino, hydroxyl, $C_{1-2}$ alkyl, $C_{1-2}$ alkoxy, $C_{2-4}$ alkenyl, $C_{2-4}$ alkynyl, $C_{1-2}$ alkyl-O-$C_{1-2}$ alkyl, $-(CH_2)_r-C_{3-5}$ cycloalkyl, $-(CH_2)_r-C_{5-9}$ bicyclic spiro cycloalkyl, $-(CH_2)_r$-(4- to 5-membered heterocycloalkyl), or $-(CH_2)_r$-(5- to 9-membered bicyclic spiro heterocycloalkyl);
$R^1$ is selected from $C_{1-6}$ alkyl or halo $C_{1-6}$ alkyl;
$R^5$ is selected from D, halogen, cyano, amino, hydroxyl, $C_{1-4}$ alkyl, $C_{1-4}$ alkoxy, halo $C_{1-4}$ alkyl, halo $C_{1-4}$ alkoxy, deuterated $C_{1-4}$ alkyl, or deuterated $C_{1-4}$ alkoxy;
each $R^{a1}$ is independently selected from $C_{1-4}$ alkyl, $C_{3-5}$ cycloalkyl, $C_{5-9}$ bicyclic spiro cycloalkyl, 4- to 6-membered heterocycloalkyl, 5- to 9-membered bicyclic spiro heterocycloalkyl, 5- to 10-membered heteroaryl. $C_{1-4}$ alkoxy, $C_{1-2}$ alkyl-O-$C_{1-2}$ alkyl, halo $C_{1-4}$ alkyl, halo $C_{1-4}$ alkoxy, deuterated $C_{1-4}$ alkyl, or deuterated $C_{1-4}$ alkoxy, wherein the alkyl, cycloalkyl, heterocycloalkyl, or heteroaryl is optionally further substituted with 1-3 substituents selected from D, halogen, cyano, hydroxyl, amino, $C_{1-2}$ alkyl, or $C_{3-4}$ cycloalkyl; or each $R^{a1}$ is independently selected from $C_{1-4}$ alkyl, $C_{3-5}$ cycloalkyl, $C_{5-9}$ bicyclic spiro cycloalkyl, 4- to 6-membered heterocycloalkyl, 5- to 9-membered bicyclic spiro heterocycloalkyl, $C_{1-4}$ alkoxy, $C_{1-2}$ alkyl-O-$C_{1-2}$ alkyl, halo $C_{1-4}$ alkyl, halo $C_{1-4}$ alkoxy, deuterated $C_{1-4}$ alkyl or deuterated $C_{1-4}$ alkoxy;
$R^2$ and $R^3$ are each independently selected from H, D, halogen, cyano, amino, hydroxyl, $C_{1-2}$ alkyl-O-$C_{1-2}$ alkyl, hydroxy $C_{1-3}$ alkyl, $C_{1-3}$ alkoxy, halo $C_{1-4}$ alkyl, halo $C_{1-4}$ alkoxy, deuterated $C_{1-4}$ alkyl, deuterated $C_{1-4}$ alkoxy or $C_{1-4}$ alkyl; or $R^2$ and $R^3$ together with the carbon atom to which they are attached form 3-membered cycloalkyl, 4-membered cycloalkyl, 5-membered cycloalkyl, 4-membered heterocycloalkyl, or 5-membered heterocycloalkyl, provided that when $R^2$ is selected from H or D, $R^3$ is not selected from H or D;
other groups are consistent with those mentioned above.

The fourth technical solution of the present invention relates to the compound as shown in formula (I), or the stereoisomer, solvate, deuterated compound, or pharmaceutically acceptable salt thereof according to the present invention, having a structure of formula (II) or (II-b):

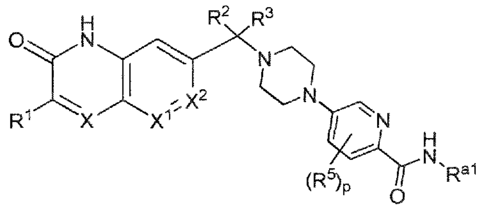

(II)

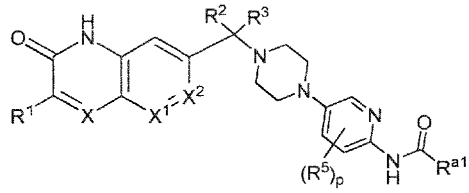

(II-b)

wherein X is selected from $CR^x$ or N;

$X^1$ and $X^2$ are each independently selected from N or $CR^x$;
p is selected from 0 or 1;
$R^x$ is independently selected from H, D, halogen, cyano, amino, hydroxyl, $C_{1-2}$ alkyl, $C_{1-2}$ alkoxy, $C_{2-4}$ alkenyl, $C_{2-4}$ alkynyl, $C_{1-2}$ alkyl-O-$C_{1-2}$ alkyl, -$(CH_2)_r$-$C_{3-5}$ cycloalkyl, -$(CH_2)_r$-$C_{5-9}$ bicyclic spiro cycloalkyl, -$(CH_2)_r$-(4- to 5-membered heterocycloalkyl), or -$(CH_2)_r$-(5- to 9-membered bicyclic spiro heterocycloalkyl);
$R^1$ is selected from $C_{1-6}$ alkyl or halo $C_{1-6}$ alkyl;
$R^5$ is selected from D, halogen, cyano, amino, hydroxyl, $C_{1-4}$ alkyl, $C_{1-4}$ alkoxy, halo $C_{1-4}$ alkyl, halo $C_{1-4}$ alkoxy, deuterated $C_{1-4}$ alkyl, or deuterated $C_{1-4}$ alkoxy;
each $R^{a1}$ is independently selected from $C_{1-4}$ alkyl, $C_{3-5}$ cycloalkyl, $C_{5-9}$ bicyclic spiro cycloalkyl, 4- to 6-membered heterocycloalkyl, 5- to 9-membered bicyclic spiro heterocycloalkyl, 5- to 10-membered heteroaryl, $C_{1-4}$ alkoxy, $C_{1-2}$ alkyl-O-$C_{1-2}$ alkyl, $C_{1-4}$ alkyl-O-$C_{3-5}$ cycloalkyl, halo $C_{1-4}$ alkyl, halo $C_{1-4}$ alkoxy, deuterated $C_{1-4}$ alkyl, or deuterated $C_{1-4}$ alkoxy, wherein the alkyl, cycloalkyl, heterocycloalkyl, or heteroaryl is optionally further substituted with 1-3 substituents selected from D, halogen, cyano, hydroxyl, amino, $C_{1-2}$ alkyl, halo $C_{1-2}$ alkyl, deuterated $C_{1-2}$ alkyl, $C_{3-4}$ cycloalkyl or phenyl;
$R^2$ and $R^3$ are each independently selected from H, D, halogen, cyano, amino, hydroxyl, $C_{1-2}$ alkyl-O-$C_{1-2}$ alkyl, hydroxy $C_{1-3}$ alkyl, $C_{1-3}$ alkoxy, halo $C_{1-4}$ alkyl, halo $C_{1-4}$ alkoxy, deuterated $C_{1-4}$ alkyl, deuterated $C_{1-4}$ alkoxy or $C_{1-4}$ alkyl; or $R^2$ and $R^3$ together with the carbon atom to which they are attached form 3-membered cycloalkyl, 4-membered cycloalkyl, 5-membered cycloalkyl, 4-membered heterocycloalkyl, or 5-membered heterocycloalkyl, provided that when $R^2$ is selected from H or D, $R^3$ is not selected from H or D.

The fifth technical solution of the present invention relates to the compound as shown in formula (I), or the stereoisomer, solvate, deuterated compound, or pharmaceutically acceptable salt thereof according to the present invention, having a structure of formula (III), (IV), (III-a), (III-b), (III-c), (IV-a), (IV-b) or (IV-c):

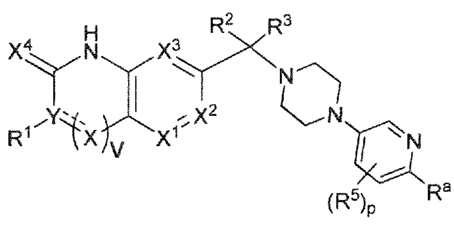

(III)

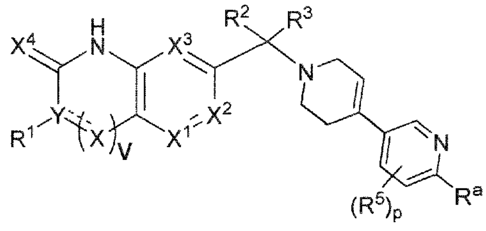

(IV)

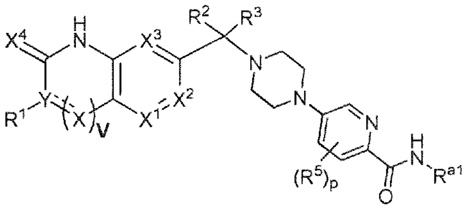 (III-a) 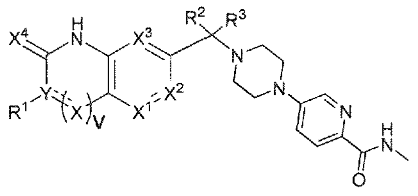 (III-b)

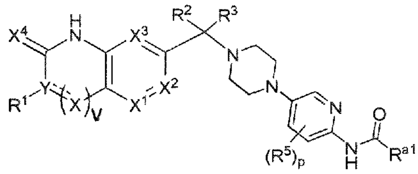 (III-c) 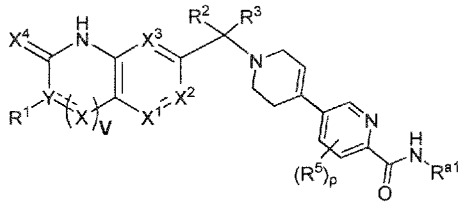 (IV-a)

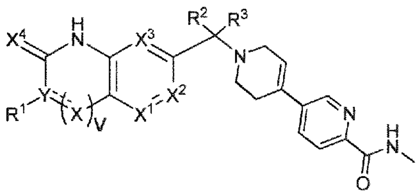 (IV-b) 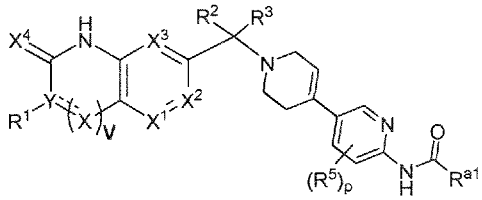 (IV-c)

wherein X is selected from $CR^x$, $C(R^x)_2$ or O;

$R_a$ is selected from $-C(O)N(R^{a1})_2$, $-NR^{a1}C(O)R^{a1}$, $-C(O)R^{a1}$, $-NR^{a1}$, - $NR^{a1}C(O)OR^{a1}$, $-NR^{a1}C(O)N(R^{a1})_2$, $-C(=S)N(R^{a1})_2$, $-S(O)_2N(R^{a1})_2$, 5- to 6-membered monocyclic heteroaryl containing 1-4 nitrogen, oxygen or sulphur atoms, 4- to 7-membered monocyclic heterocycloalkyl containing 1-4 nitrogen, oxygen or sulphur atoms, or 3- to 7-membered monocyclic cycloalkyl, wherein the heteroaryl, heterocycloalkyl, or cycloalkyl is optionally further substituted with 1-3 substituents selected from D, halogen, cyano, hydroxyl, amino, $-NHC_{1-2}$ alkyl, $-N(C_{1-2}$ alkyl$)_2$, $C_{1-4}$ alkyl, halo $C_{1-4}$ alkyl, $C_{1-4}$ alkoxy, halo $C_{1-4}$ alkoxy, deuterated $C_{1-4}$ alkyl, or deuterated $C_{1-4}$ alkoxy;

each $R^{a1}$ is independently selected from $C_{1-4}$ alkyl, $C_{3-5}$ cycloalkyl. $C_{5-9}$ bicyclic spiro cycloalkyl, 4- to 6-membered heterocycloalkyl, 5- to 9-membered bicyclic spiro heterocycloalkyl, 5- to 10-membered heteroaryl, $C_{1-4}$ alkoxy, $C_{1-2}$ alkyl-O-$C_{1-2}$ alkyl, $C_{1-4}$ alkyl-O-$C_{3-6}$ cycloalkyl, halo $C_{1-4}$ alkyl, halo $C_{1-4}$ alkoxy, deuterated $C_{1-4}$ alkyl, or deuterated $C_{1-4}$ alkoxy, wherein the alkyl, cycloalkyl, heterocycloalkyl, or heteroaryl is optionally further substituted with 1-3 substituents selected from D, F, Cl, cyano, hydroxyl, amino, $C_{1-2}$ alkyl, halo $C_{1-2}$ alkyl, deuterated $C_{1-2}$ alkyl, $C_{3-4}$ cycloalkyl or phenyl;

$R^1$ is selected from halogen, nitro, cyano, amino, hydroxyl, $-SF_5$, $C_{1-6}$ alkyl, $C_{1-6}$ alkoxy, $C_{2-6}$ alkenyl, $C_{2-6}$ alkynyl, $C_{1-6}$ alkyl-O-$C_{1-6}$ alkyl, $-(CH_2)_r-C_{3-12}$ cycloalkyl or $-(CH_2)_r$-(3- to 12-membered heterocycloalkyl), wherein the alkyl, alkoxy, alkenyl, alkynyl, cycloalkyl or heterocycloalkyl is optionally further substituted with 1-3 groups selected from D, halogen, cyano, amino, hydroxyl, $C_{1-6}$ alkyl or $C_{1-6}$ alkoxy;

each r is independently selected from 0, 1 or 2;

provided that when $R^2$ is selected from H or D and $R^3$ is selected from H or D, $X^4$ is selected from O, $X^3$ is selected from CH, - - - - - - - represents a double bond, v is selected from 1, and Y is selected from C, $R^1$ is not $C_{1-6}$ alkyl and halo $C_{1-6}$ alkyl.

The sixth technical solution of the present invention relates to the compound as shown in formula (I), or the stereoisomer, solvate, deuterated compound, or pharmaceutically acceptable salt thereof according to the present invention, having a structure of formula (III), (III-a) or (111-b):

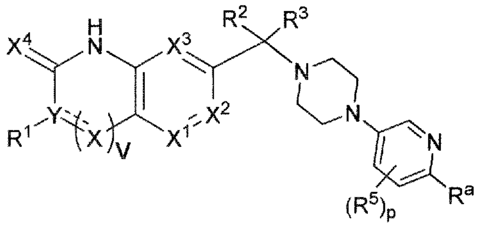

(III)

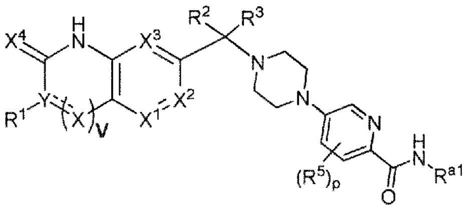

(III-a)

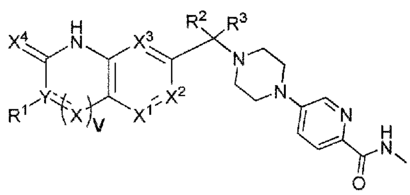

(III-b)

wherein X is selected from $CR^x$, $C(R^x)_2$ or O;

$R_a$ is selected from $-C(O)N(R^{a1})_2$, $-NR^{a1}C(O)OR^{a1}$, $-NR^{a1}C(O)N(R^{a1})_2$, - $C(=S)N(R^{a1})_2$, $-S(O)_2N(R^{a1})_2$, 5- to 6-membered monocyclic heteroaryl containing 1-4 nitrogen, oxygen or sulphur atoms, 4- to 7-membered monocyclic heterocycloalkyl containing 1-4 nitrogen, oxygen or sulphur atoms, or 3- to 7-membered monocyclic cycloalkyl, wherein the heteroaryl, heterocycloalkyl, or cycloalkyl is optionally further substituted with 1-3 substituents selected from D, halogen, cyano, hydroxyl, amino, $-NHC_{1-2}$ alkyl, $-N(C_{1-2}$ alkyl$)_2$, $C_{1-4}$ alkyl, halo $C_{1-4}$ alkyl, $C_{1-4}$ alkoxy, halo $C_{1-4}$ alkoxy, deuterated $C_{1-4}$ alkyl or deuterated $C_{1-4}$ alkoxy:

each $R^{a1}$ is independently selected from $C_{1-4}$ alkyl, $C_{3-5}$ cycloalkyl, $C_{5-9}$ bicyclic spiro cycloalkyl, 4- to 6-membered heterocycloalkyl, 5- to 9-membered bicyclic spiro heterocycloalkyl, 5- to 10-membered heteroaryl, $C_{1-4}$ alkoxy, $C_{1-2}$ alkyl-O-$C_{1-2}$ alkyl, halo $C_{1-4}$ alkyl, halo $C_{1-4}$ alkoxy, deuterated $C_{1-4}$ alkyl, or deuterated $C_{1-4}$ alkoxy, wherein the alkyl, cycloalkyl, heterocycloalkyl, or heteroaryl is optionally further substituted with 1-3 substituents selected from D, F, Cl, cyano, hydroxyl, amino, $C_{1-2}$ alkyl, or $C_{3-4}$ cycloalkyl; or each $R^{a1}$ is independently selected from $C_{1-4}$ alkyl, $C_{3-5}$ cycloalkyl, $C_{5-9}$ bicyclic spiro cycloalkyl, 4- to 6-membered heterocycloalkyl, 5- to 9-membered bicyclic spiro heterocycloalkyl, $C_{1-4}$ alkoxy, $C_{1-2}$ alkyl-O-$C_{1-2}$ alkyl, halo $C_{1-4}$ alkyl, halo $C_{1-4}$ alkoxy, deuterated $C_{1-4}$ alkyl or deuterated $C_{1-4}$ alkoxy;

$R^1$ is selected from halogen, nitro, cyano, amino, hydroxyl, $-SF_5$, $C_{1-6}$ alkyl, $C_{1-6}$ alkoxy, $C_{2-6}$ alkenyl, $C_{2-6}$ alkynyl, $C_{1-6}$ alkyl-O-$C_{1-6}$ alkyl, $-(CH_2)_r-C_{3-12}$ cycloalkyl or $-(CH_2)_r-$(3- to 12-membered heterocycloalkyl), wherein the alkyl, alkoxy, alkenyl, alkynyl, cycloalkyl or heterocycloalkyl is optionally further substituted with 1-3 groups selected from D, halogen, cyano, amino, hydroxyl, $C_{1-6}$ alkyl or $C_{1-6}$ alkoxy;

each r is independently selected from 0, 1 or 2;

provided that when $R^2$ is selected from H or D and $R^3$ is selected from H or D, $X^4$ is selected from O, $X^3$ is selected from CH, - - - - - - - represents a double bond, v is selected from 1, and Y is selected from C, $R^1$ is not $C_{1-6}$ alkyl and halo $C_{1-6}$ alkyl;

other groups are consistent with those mentioned above.

The seventh technical solution of the present invention relates to the compound as shown in formula (I), (II), (II-a), (II-b), (III), (III-a), (III-b), (III-c), (IV), (IV-a), (IV-b) or (IV-c), or the stereoisomer, solvate, deuterated compound, or pharmaceutically acceptable salt thereof according to the present invention, wherein

X is selected from $CR^x$, $C(R^x)_2$ or O;

v is selected from 1 or 2;

p is selected from 0 or 1;

each $R^x$ is independently selected from H, D, halogen, cyano, amino, hydroxyl, $C_{1-4}$ alkyl, $C_{1-4}$ alkoxy, $C_{2-4}$ alkenyl, $C_{2-4}$ alkynyl, $C_{1-4}$ alkyl-O-$C_{1-4}$ alkyl, $-(CH_2)_r-C_{3-6}$ monocyclic cycloalkyl or $-(CH_2)_r-$(4- to 6-membered monocyclic heterocycloalkyl); or two $R^x$ on the same carbon atom together form =O;

each $R^5$ is independently selected from D, halogen, cyano, amino, hydroxyl, $C_{1-4}$ alkyl, $C_{1-4}$ alkoxy, halo $C_{1-4}$ alkyl, halo $C_{1-4}$ alkoxy, deuterated $C_{1-4}$ alkyl, or deuterated $C_{1-4}$ alkoxy;

$R^{a1}$ is selected from $C_{1-4}$ alkyl, $C_{3-5}$ cycloalkyl, $C_{5-9}$ bicyclic spiro cycloalkyl, 4- to 6-membered heterocycloalkyl, 5- to 9-membered bicyclic spiro heterocycloalkyl, 5- to 6-membered heteroaryl, $C_{1-4}$ alkoxy, $C_{1-2}$ alkyl-O-$C_{1-2}$ alkyl,

$C_{1-2}$ alkyl-O-$C_{3-5}$ cycloalkyl, halo $C_{1-4}$ alkyl, halo $C_{1-4}$ alkoxy, deuterated $C_{1-4}$ alkyl, or deuterated $C_{1-4}$ alkoxy, wherein the alkyl, cycloalkyl, heterocycloalkyl, or heteroaryl is optionally further substituted with 1-3 substituents selected from D, halogen, cyano, hydroxyl, amino, $C_{1-2}$ alkyl, halo $C_{1-2}$ alkyl, deuterated $C_{1-2}$ alkyl, $C_{3-5}$ cycloalkyl or phenyl; in some embodiments, $R^{a1}$ is selected from $C_{1-4}$ alkyl, $C_{3-5}$ cycloalkyl, $C_{5-9}$ bicyclic spiro cycloalkyl, 4- to 6-membered heterocycloalkyl, 5-to 9-membered bicyclic spiro heterocycloalkyl, $C_{1-4}$ alkoxy, $C_{1-2}$ alkyl-O-$C_{1-2}$ alkyl, halo $C_{1-4}$ alkyl, halo $C_{1-4}$ alkoxy, deuterated $C_{1-4}$ alkyl or deuterated $C_{1-4}$ alkoxy;
$R^2$ and $R^3$ are each independently selected from H, D, halogen, cyano, amino, hydroxyl, $C_{1-2}$ alkyl-O-$C_{1-2}$ alkyl, hydroxy $C_{1-3}$ alkyl, $C_{1-3}$ alkoxy, halo $C_{1-4}$ alkyl, halo $C_{1-4}$ alkoxy, deuterated $C_{1-4}$ alkyl, deuterated $C_{1-4}$ alkoxy or $C_{1-4}$ alkyl; or $R^2$ and $R^3$ together with the carbon atom to which they are attached form 3-membered cycloalkyl, 4-membered cycloalkyl, 5-membered cycloalkyl, 4-membered heterocycloalkyl, or 5-membered heterocycloalkyl; the definitions of other groups are consistent with those mentioned above.

The eighth technical solution of the present invention relates to the compound as shown in formula (I), (II), (II-a), (II-b), (III), (III-a), (III-b), (III-c), (IV), (IV-a), (IV-b) or (IV-c), or the stereoisomer, solvate, deuterated compound, or pharmaceutically acceptable salt thereof according to the present invention, wherein
$R^1$ is selected from halogen, nitro, cyano, amino, hydroxyl, $-SF_5$, $C_{1-4}$ alkyl, $C_{1-4}$ alkoxy, $C_{2-4}$ alkenyl, $C_{2-4}$ alkynyl, $C_{1-4}$ alkyl-O-$C_{1-4}$ alkyl, $-(CH_2)_r$-$C_{3-6}$ monocyclic cycloalkyl, $-(CH_2)_r$-$C_{5-9}$ bicyclic spiro cycloalkyl, $-(CH_2)_r$-(4- to 6-membered monocyclic heterocycloalkyl) or $-(CH_2)_r$-(5- to 9-membered bicyclic spiro heterocycloalkyl), wherein the alkyl, alkoxy, alkenyl, alkynyl, cycloalkyl, or heterocycloalkyl is optionally further substituted with 1-3 groups selected from D, F, Cl, cyano, amino, hydroxyl, $C_{1-3}$ alkyl or $C_{1-3}$ alkoxy.

The ninth technical solution of the present invention relates to the compound as shown in formula (I), (II), (II-a), (II-b), (III), (III-a), (III-b), (III-c), (IV), (IV-a), (IV-b), (IV-c), (V), (VI), (VII), (VIII) or (IX), or the stereoisomer, solvate, deuterated compound, or pharmaceutically acceptable salt thereof according to the present invention, wherein

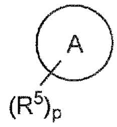

is selected from

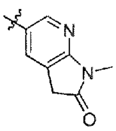 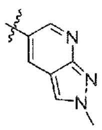 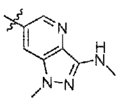 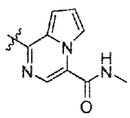 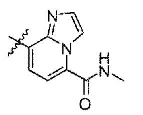 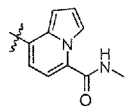 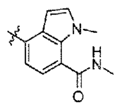

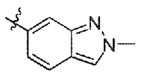 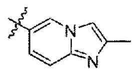 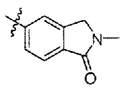 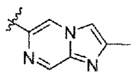 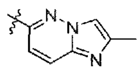 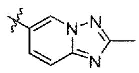 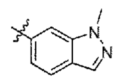

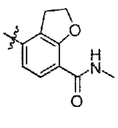 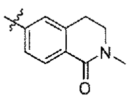 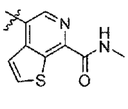 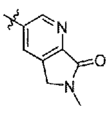 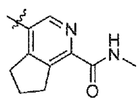 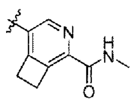 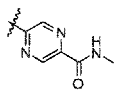

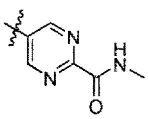 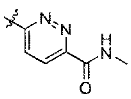 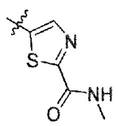 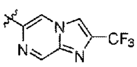 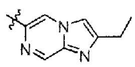 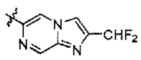 ;

or

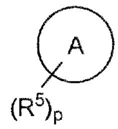

is selected from

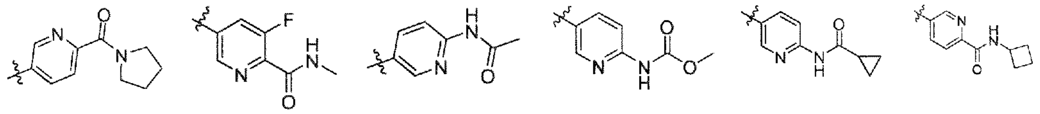

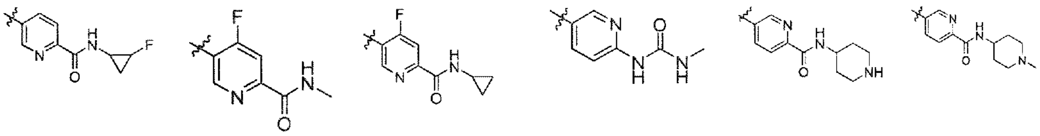

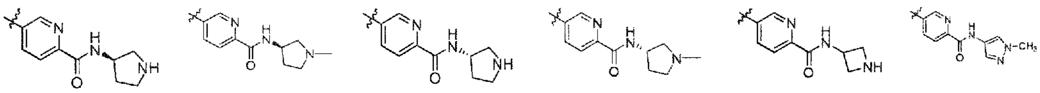

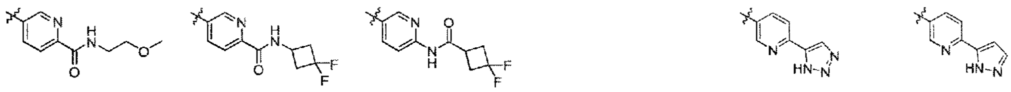

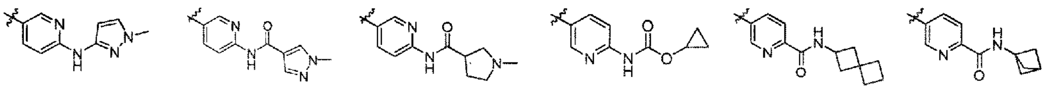

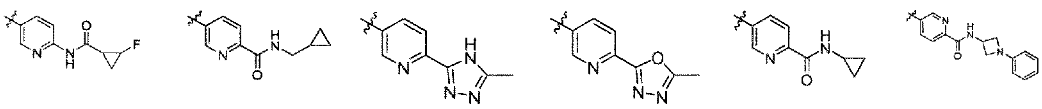

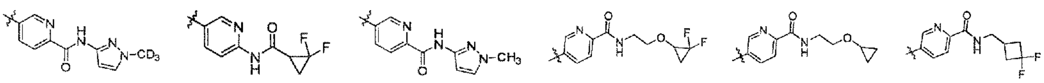

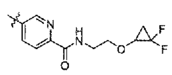 .

The tenth technical solution of the present invention relates to the compound as shown in formula (I), (II), (II-a), (II-b), (III), (III-a), (III-b), (III-c), (IV), (IV-a), (IV-b), (IV-c), (V), (VI), (VII), (VIII) or (IX), or the stereoisomer, solvate, deuterated compound, or pharmaceutically acceptable salt thereof according to the present invention, wherein ring B is selected from piperazinyl, piperidyl or

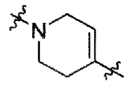 ;

in some embodiments, ring B is selected from piperazinyl or

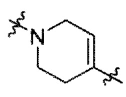 ;

in some embodiments, ring B is selected from piperazinyl; in some embodiments, ring B is selected from

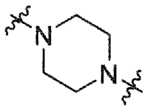 or 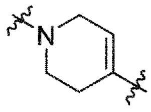.

The eleventh technical solution of the present invention relates to the compound as shown in formula (I), (II), (II-a), (II-b), (III), (III-a), (III-b), (III-c), (IV), (IV-a), (IV-b), (IV-c), (V), (VI), (VII), (VIII) or (IX), or the stereoisomer, solvate, deuterated compound, or pharmaceutically acceptable salt thereof according to the present invention, wherein

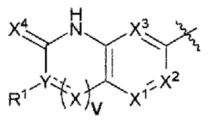

is selected from

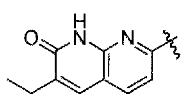, 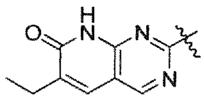,

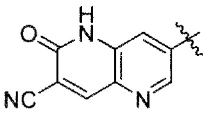, 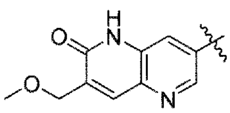, 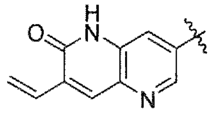, 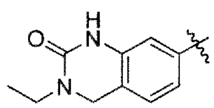,

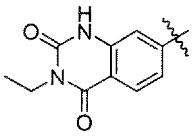, 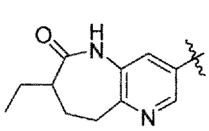, 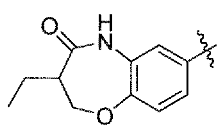, 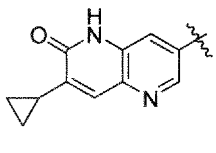

or

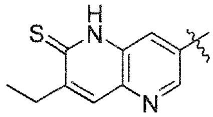 ;

in some embodiments,

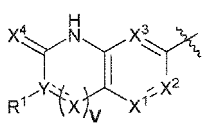

is selected from

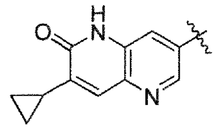

The twelfth technical solution of the present invention relates to the compound as shown in formula (1), (II), (II-a) or (II-b), or the stereoisomer, solvate, deuterated compound, or pharmaceutically acceptable salt thereof according to the present invention, wherein

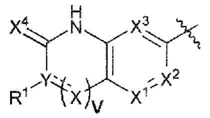

is selected from

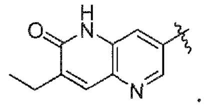

The thirteenth technical solution of the present invention relates to the compound as shown in formula (I), (II), (II-a), (II-b), (III), (III-a), (III-b), (III-c), (IV), (IV-a), (IV-b), (IV-c), (V), (VI), (VII), (VIII) or (IX), or the stereoisomer, solvate, deuterated compound, or pharmaceutically acceptable salt thereof, or the stereoisomer, solvate, deuterated compound, or pharmaceutically acceptable salt thereof according to the present invention, wherein

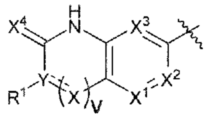

is selected from

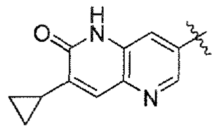

The fourteenth technical solution of the present invention relates to the compound as shown in formula (I), or the stereoisomer, solvate, deuterated compound, or pharmaceutically acceptable salt thereof according to the present invention, having a structure of formula (V):

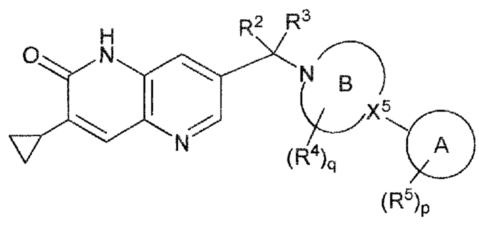

(V)

wherein $R^2$ and $R^3$ are each independently selected from H, D, halogen, cyano, amino, hydroxyl, $C_{1-2}$ alkyl-O-$C_{1-2}$ alkyl, hydroxy $C_{1-2}$ alkyl, $C_{1-2}$ alkoxy, halo $C_{1-2}$ alkyl, halo $C_{1-2}$ alkoxy, deuterated $C_{1-2}$ alkyl, deuterated $C_{1-2}$ alkoxy or $C_{1-2}$ alkyl; or $R^2$ and $R^3$ together with the carbon atom to which they are attached form $C_{3-5}$ cycloalkyl or 4- to 5-membered heterocycloalkyl; in some embodiments, $R^2$ and $R^3$ are each independently selected from H, D, halogen, cyano, amino, hydroxyl, or halo $C_{1-2}$ alkyl; in some embodiments, $R^2$ and $R^3$ are each independently selected from

H, D, or $C_{1-2}$ alkyl; or $R^2$ and $R^3$ together with the carbon atom to which they are attached form $C_{3-4}$ cycloalkyl; in some embodiments, $R^2$ and $R^3$ are each independently selected from H or D;

each $R^4$ is independently selected from D, halogen, cyano, amino, hydroxyl, - $SF_5$, $C_{1-2}$ alkyl, $C_{1-2}$ alkoxy, halo $C_{1-2}$ alkyl, halo $C_{1-2}$ alkoxy, deuterated $C_{1-2}$ alkyl or deuterated $C_{1-2}$ alkoxy; or two $R^4$ on the same carbon atom together with the carbon atom to which they are attached form =O; in some embodiments, each $R^4$ is independently selected from D, F, Cl, cyano, amino, hydroxyl, $C_{1-2}$ alkyl, $C_{1-2}$ alkoxy, halo $C_{1-2}$ alkyl, halo $C_{1-2}$ alkoxy, deuterated $C_{1-2}$ alkyl, or deuterated $C_{1-2}$ alkoxy; or two $R^4$ on the same carbon atom together with the carbon atom to which they are attached form =O; in some embodiments, each $R^4$ is independently selected from D, F, Cl, methyl, ethyl, methoxy, ethoxy, $-CH_2F$, $-CHF_2$, $-CF_3$, $-CH_2CH_2F$, $-CH_2CHF_2$, $-CH_2CF_3$, $-CHFCH_2F$, $-CHFCHF_2$, $-CHFCF_3$, $-CF_2CH_2F$, $-CF_2CHF_2$, $-CF_2CF_3$, $- CH_2D$, $-CHD_2$, $-CD_3$, $-CH_2CH_2D$, $-CH_2CHD_2$, $-CH_2CD_3$, $-CHDCH_2D$, $-CHDCHD_2$, $-CHDCD_3$, $-CD_2CH_2D$, $-CD_2CHD_2$, or $-CD_2CD_3$; or two $R^4$ on the same carbon atom together with the carbon atom to which they are attached form =O; in some embodiments, each $R^4$ is independently selected from D, F, Cl, or cyano;

each $R^5$ is independently selected from D, halogen, cyano, amino, hydroxyl, - $SF_5$, $C_{1-2}$ alkyl, $C_{1-2}$ alkoxy, halo $C_{1-2}$ alkyl, halo $C_{1-2}$ alkoxy, deuterated $C_{1-2}$ alkyl or deuterated $C_{1-2}$ alkoxy; in some embodiments, each $R^5$ is independently selected from D, F, Cl, $C_{1-2}$ alkyl, halo $C_{1-2}$ alkyl, or deuterated $C_{1-2}$ alkyl; in some embodiments, each $R^5$ is independently selected from D, F, Cl, methyl, ethyl, $-CH_2F$, $-CHF_2$, $-CF_3$, $-CH_2CH_2F$, $-CH_2CHF_2$, $-CH_2CF_3$, $-CHFCH_2F$, $-CHFCHF_2$, $-CHFCF_3$, $-CF_2CH_2F$, $-CF_2CHF_2$, $-CF_2CF_3$, $-CH_2D$, $-CHD_2$, $-CD_3$, $-CH_2CH_2D$, $-CH_2CHD_2$, $- CH_2CD_3$, $-CHDCH_2D$, $-CHDCHD_2$, $-CHDCD_3$, $-CD_2CH_2D$, $-CD_2CHD_2$, or $- CD_2CD_3$;

q is selected from 0 or 1; in some embodiments, q is selected from 0;

p is selected from 0 or 1; in some embodiments, p is selected from 0;

ring B is selected from

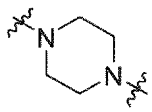 or 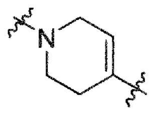 ;

in some embodiments, ring B is selected from

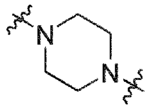 ;

ring A is selected from

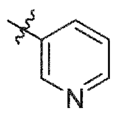 ,

which is further substituted with 1-3 substituents selected from $R_a$;

or

ring A is selected from 7- to 10-membered bicyclic heteroaromatic ring containing 1-5 nitrogen, oxygen or sulphur atoms, or 7- to 10-membered bicyclic aromatic ring, wherein the heteroaromatic ring or aromatic ring is optionally further substituted with 1-3 substituents selected from $R_b$; in some embodiments, ring A is selected from

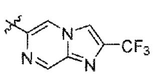 , 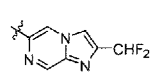 , 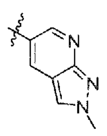 , 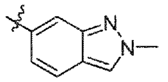 , 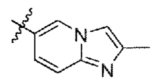 ,

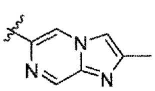 , 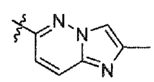 , 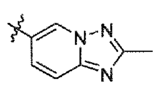 , 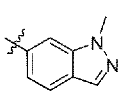 or 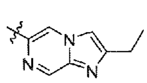 ;

in some embodiments, ring A is selected from

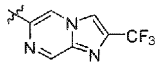 or 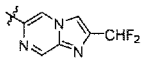;

$R_a$ is selected from $-C(O)N(R^{a1})_2$, $-NR^{a1}C(O)R^{a1}$ or $-C(O)R^{a1}$; in some embodiments, $R_a$ is selected from $-C(O)N(R^{a1})_2$ or $-NR^{a1}C(O)R^{a1}$; in some embodiments, $R_a$ is selected from $-C(O)N(R^{a1})_2$;

$R_b$ is selected from D, halogen, cyano, hydroxyl, amino, $C_{1-2}$ alkyl, $C_{3-5}$ cycloalkyl, 3- to 5-membered heterocycloalkyl, $C_{1-2}$ alkoxy, $C_{1-2}$ alkyl-O-$C_{1-2}$ alkyl, halo $C_{1-2}$ alkyl, halo $C_{1-2}$ alkoxy, deuterated $C_{1-2}$ alkyl, or deuterated $C_{1-2}$ alkoxy; in some embodiments, $R_b$ is selected from $-C(O)N(R^{a1})_2$, =O, D, halogen, cyano, hydroxyl, amino, $-NHCH_3$, $-NHCH_2CH_3$, $-N(CH_3)_2$, methyl, ethyl, $-CH_2F$, $-CHF_2$, $-CF_3$, $-CH_2CH_2F$, $-CH_2CHF_2$, $-CH_2CF_3$, $-CHFCH_2F$, $-CHFCHF_2$, or $-CHFCF_3$; in some embodiments, $R_b$ is selected from D, halogen, cyano, hydroxyl, amino, methyl, ethyl, $-CH_2F$, $-CHF_2$, $-CF_3$, $-CH_2CH_2F$, $-CH_2CHF_2$, $-CH_2CF_3$, $-CHFCH_2F$, $-CHFCHF_2$, or $-CHFCF_3$;

each $R^{a1}$ is independently selected from H, D, $C_{1-2}$ alkyl, $C_{3-5}$ cycloalkyl, 3- to 5-membered heterocycloalkyl, 5- to 6-membered heteroaryl, halo $C_{1-2}$ alkyl, halo $C_{1-2}$ alkoxy, deuterated $C_{1-2}$ alkyl, or deuterated $C_{1-2}$ alkoxy, wherein the cycloalkyl, heterocycloalkyl, or heteroaryl is optionally further substituted with 1-3 substituents selected from D, halogen, cyano, hydroxyl, amino, $C_{1-2}$ alkyl, halo $C_{1-2}$ alkyl, deuterated $C_{1-2}$ alkyl, or $C_{3-4}$ cycloalkyl; in some embodiments, each $R^{a1}$ is independently selected from $C_{1-2}$ alkyl, $C_{3-5}$ cycloalkyl, 5- to 6-membered heteroaryl, halo $C_{1-2}$ alkyl, halo $C_{1-2}$ alkoxy, deuterated $C_{1-2}$ alkyl, or deuterated $C_{1-2}$ alkoxy, wherein the cycloalkyl or heteroaryl is optionally further substituted with 1-3 substituents selected from D, halogen, cyano, hydroxyl, amino, $C_{1-2}$ alkyl, halo $C_{1-2}$ alkyl, or deuterated $C_{1-2}$ alkyl; in some embodiments, each $R^{a1}$ is independently selected from $C_{1-2}$ alkyl, $C_{3-5}$ cycloalkyl, or 5- to 6-membered heteroaryl, wherein the cycloalkyl or heteroaryl is optionally further substituted with 1-3 substituents selected from D, halogen, cyano, hydroxyl, amino, $C_{1-2}$ alkyl, halo $C_{1-2}$ alkyl, or deuterated $C_{1-2}$ alkyl; in some embodiments, each $R^{a1}$ is independently selected from methyl, ethyl, cyclopropyl, cyclobutyl, $-CH_2F$, $-CHF_2$, $-CF_3$, $-CH_2CH_2F$, $-CH_2CHF_2$, $-CH_2CF_3$, $-CHFCH_2F$, $-CHFCHF_2$, $-CHFCF_3$, $-CF_2CH_2F$, $-CF_2CHF_2$, $-CF_2CF_3$, $-CH_2CH_2CH_2F$, $-CH_2CH_2CHF_2$, $-CH_2CH_2CF_3$, or

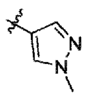.

The fifteenth technical solution of the present invention relates to the compound as shown in formula (I), or the stereoisomer, solvate, deuterated compound, or pharmaceutically acceptable salt thereof according to the present invention, having a structure of formula (VI), (VII), (VIII) or (IX):

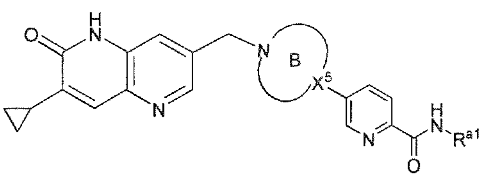 (VI)

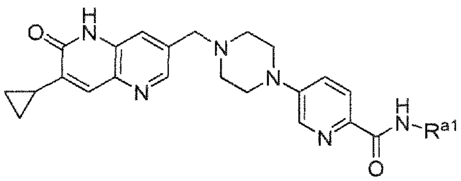 (VII)

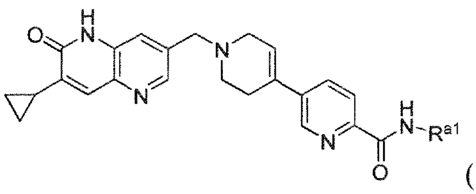 (VIII)

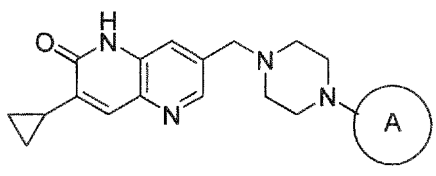 (IX)

wherein ring B is selected from

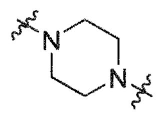 or 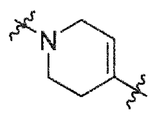 ;

in some embodiments, ring B is selected from

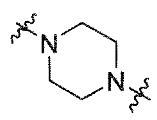 ;

$X^5$ is selected from N or CH; in some embodiments, $X^5$ is selected from N;

each $R^{a1}$ is independently selected from $C_{1-4}$ alkyl, $C_{3-5}$ cycloalkyl, or 5- to 6-membered heteroaryl, wherein the alkyl, cycloalkyl, or heteroaryl is optionally further substituted with 1, 2 or 3 substituents selected from D, F, Cl, cyano, hydroxyl, amino, $C_{1-2}$ alkyl, halo $C_{1-2}$ alkyl, or deuterated $C_{1-2}$ alkyl; in some embodiments, each $R^{a1}$ is independently selected from methyl, ethyl, propyl, isopropyl, tert-butyl, cyclopropyl, cyclobutyl, $-CH_2F$, $-CHF_2$, $-CF_3$, $-CH_2CH_2F$, $-CH_2CHF_2$, $-CH_2CF_3$, - $CHFCH_2F$, $-CHFCHF_2$, $-CHFCF_3$, $-CF_2CH_2F$, $-CF_2CHF_2$, $-CF_2CF_3$, $-CH_2CH_2CH_2F$, $-CH_2CH_2CHF_2$, $-CH_2CH_2CF_3$,

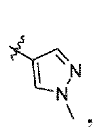 , 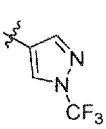 or 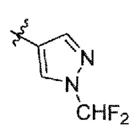 ;

ring A is selected from 8- to 10-membered bicyclic heteroaromatic ring containing 1-5 nitrogen, oxygen or sulphur atoms, wherein the heteroaromatic ring is optionally further substituted with 1, 2 or 3 substituents selected from -$C(O)N(R^{a1})_2$, -$NHC_{1-2}$ alkyl, -$N(C_{1-2}$ alkyl$)_2$, $C_{1-2}$ alkyl, =O, $C_{1-2}$ alkyl, $C_{3-5}$ cycloalkyl, halo $C_{1-2}$ alkyl, halo $C_{1-2}$ alkoxy, deuterated $C_{1-2}$ alkyl, or deuterated $C_{1-2}$ alkoxy; in some embodiments, ring A is selected from

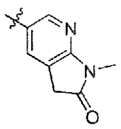 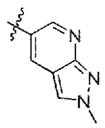 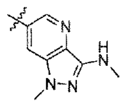 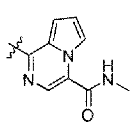 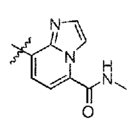 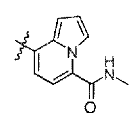 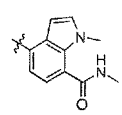

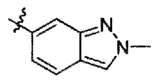 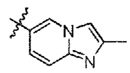 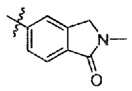 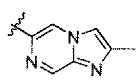 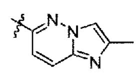 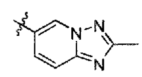 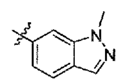

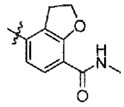 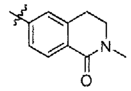 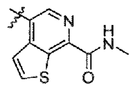 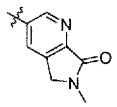 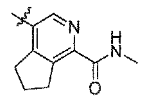 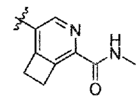 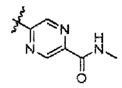

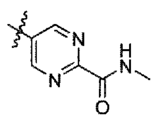 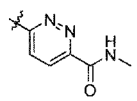 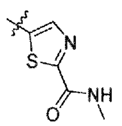 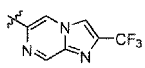 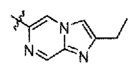 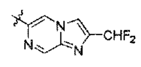 ;

in some embodiments, ring A is selected from 8- to 10-membered bicyclic heteroaromatic ring containing 1-5 nitrogen, oxygen or sulphur atoms, wherein the heteroaromatic ring is optionally further substituted with 1, 2 or 3 substituents selected from $C_{1-2}$ alkyl, $C_{3-5}$ cycloalkyl, halo $C_{1-2}$ alkyl, halo $C_{1-2}$ alkoxy, deuterated $C_{1-2}$ alkyl, or deuterated $C_{1-2}$ alkoxy; in some embodiments, ring A is selected from 8-, 9- or 10-membered bicyclic heteroaromatic ring containing 1, 2, 3, 4 or 5 nitrogen, oxygen or sulphur atoms, wherein the heteroaromatic ring is optionally further substituted with 1, 2 or 3 substituents selected from $C_{1-2}$ alkyl, $C_{3-5}$ cycloalkyl, halo $C_{1-2}$ alkyl, halo $C_{1-2}$ alkoxy, deuterated $C_{1-2}$ alkyl, or deuterated $C_{1-2}$ alkoxy; in some embodiments, ring A is selected from

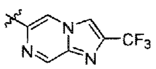, 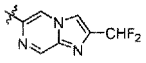, 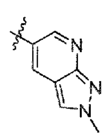,

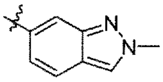, 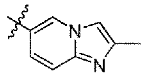, 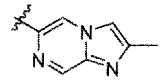, 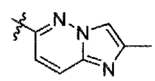, 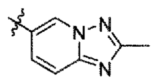, 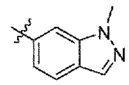

or

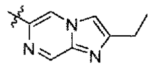;

in some embodiments, ring A is selected from

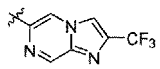

or

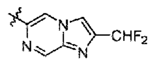.

The sixteenth technical solution of the present invention relates to the compound as shown in formula (I), (II), (II-a), (II-b), (III), (III-a), (III-b), (III-c), (IV), (IV-a), (IV-b), (IV-c), (V), (VI), (VII), (VIII) or (IX), or the stereoisomer, solvate, deuterated compound, or pharmaceutically acceptable salt thereof according to the present invention, wherein the compound is selected from one of the following structures:

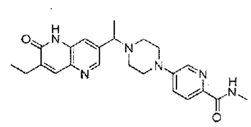 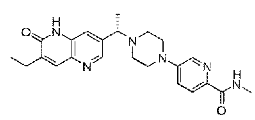 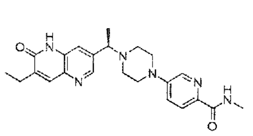 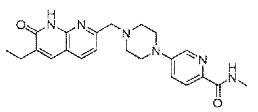

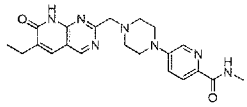 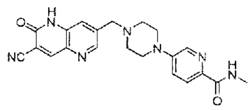 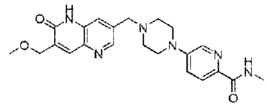 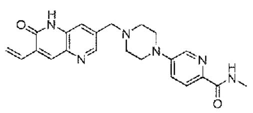

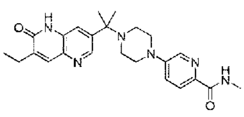 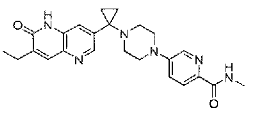 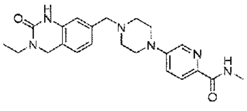 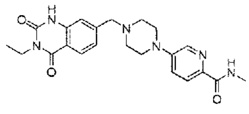

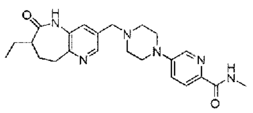 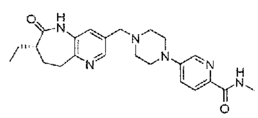 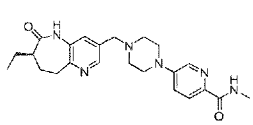 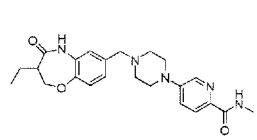

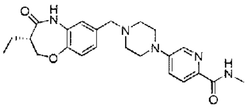 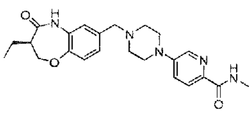 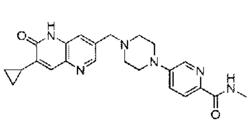 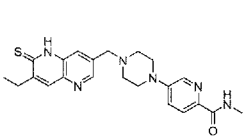

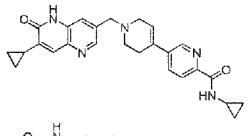 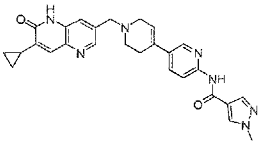 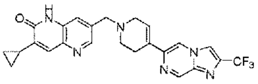 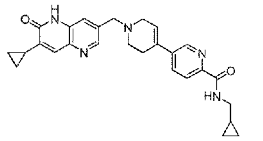

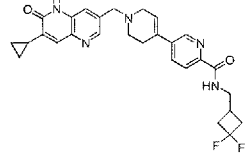 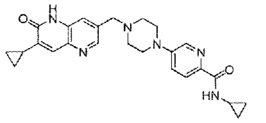 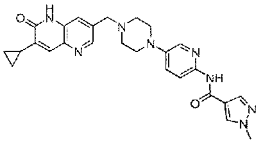 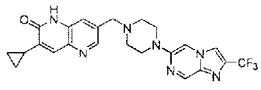

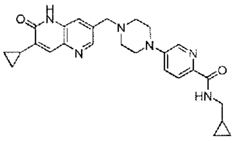 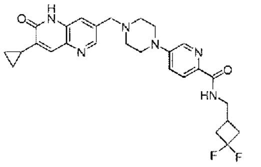

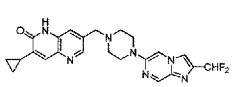 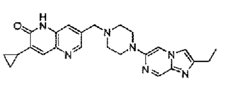 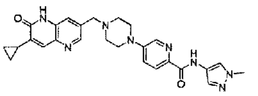 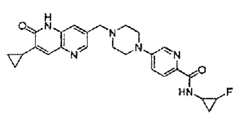

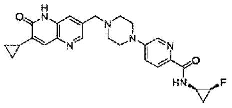 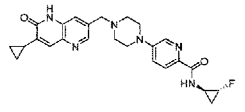 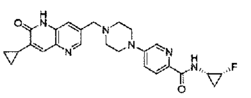 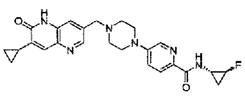

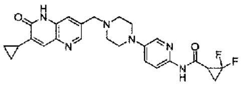 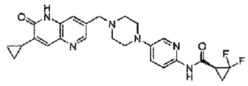 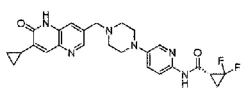 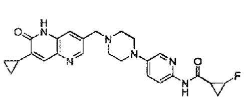

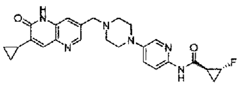 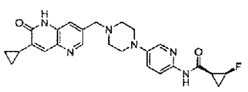 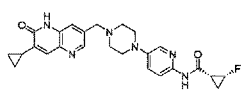 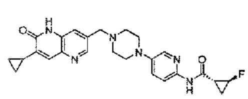

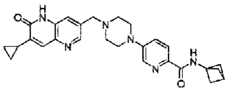 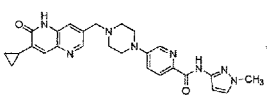 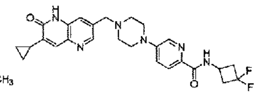 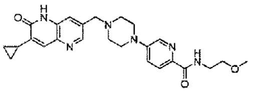

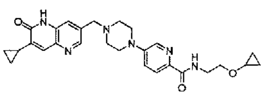 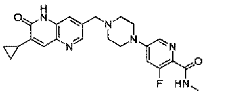 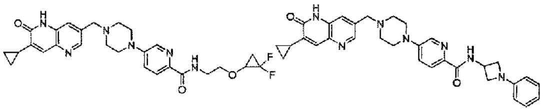

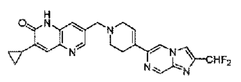 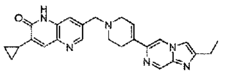 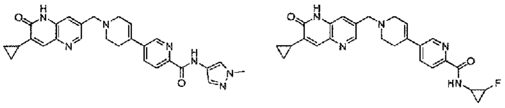

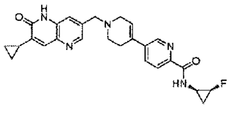 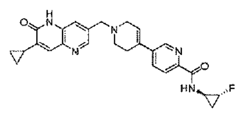 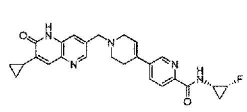 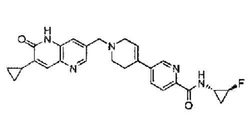

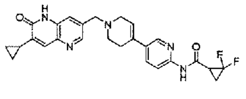 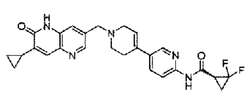 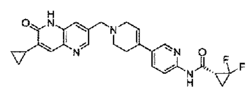 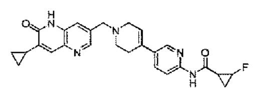

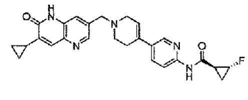 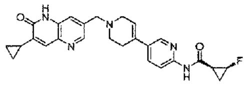 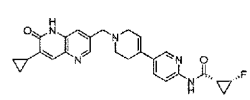 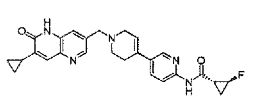

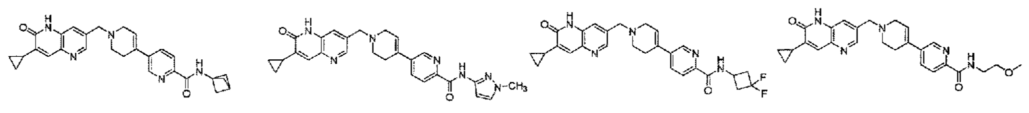

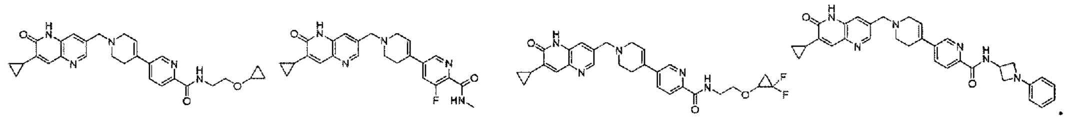.

Secondly, the present invention also provides a pharmaceutical composition, comprising the compound, or the stereoisomer, solvate, deuterated compound, or pharmaceutically acceptable salt thereof according to any one of the preceding technical solutions, and a pharmaceutically acceptable excipient and/or carrier.

Further, the pharmaceutical composition or pharmaceutical preparation comprises 1-1440 mg of the compound, or the stereoisomer, deuterated compound, solvate, or pharmaceutically acceptable salt thereof according to any one of the preceding first to fourteenth technical solutions, and a pharmaceutically acceptable excipient and/or carrier.

Further, the present invention also provides the use of the compound, or the stereoisomer, solvate, deuterated compound, or pharmaceutically acceptable salt thereof, or the pharmaceutical composition according to any one of the preceding embodiments in the preparation of a drug for treating/preventing a PARP-1-mediated disease. Further, the PARP-1-mediated disease includes, but is not limited to cancer.

The present invention also provides a method for treating a disease in a mammal, comprising administering to a subject a therapeutically effective amount of the compound, or the stereoisomer, deuterated compound, solvate, or pharmaceutically acceptable salt thereof according to any one of the preceding first to fourteenth technical solutions, and a pharmaceutically acceptable excipient and/or carrier, wherein the therapeutically effective amount is preferably 1-1440 mg, and the disease is preferably cancer.

The present invention also provides a method for treating a disease in a mammal, comprising administering to the mammal a therapeutically effective amount of the compound, or the stereoisomer, deuterated compound, solvate, or pharmaceutically acceptable salt thereof, or the pharmaceutical composition according to the present invention. In some embodiments, the mammal according to the present invention comprises humans.

The "effective amount" or "therapeutically effective amount" as described in the present application refers to administration of a sufficient amount of the compound disclosed in the present application that will alleviate to some extent one or more symptoms of the diseases or conditions being treated. In some embodiments, the outcome is the reduction and/or remission of signs, symptoms or causes of the disease, or any other desired change in the biological system. For example, an "effective amount" in terms of the therapeutic use is an amount of the composition comprising the compound disclosed in the present application that is required to provide clinically significant reduction of the symptoms of the disease. Examples of the therapeutically effective amount include, but are not limited to 1-1440 mg, 1-1400 mg, 1-1300 mg, 1-1200 mg, 1-1000 mg, 1-900 mg, 1-800 mg, 1-700 mg, 1-600 mg, 1-500 mg, 1-400 mg, 1-300 mg, 1-250 mg, 1-200 mg, 1-150 mg, 1-125 mg, 1-100 mg, 1-80 mg, 1-60 mg, 1-50 mg, 1-40 mg, 1-25 mg, 1-20 mg, 5-1000 mg, 5-900 mg, 5-800 mg, 5-700 mg, 5-600 mg, 5-500 mg, 5-400 mg, 5-300 mg, 5-250 mg, 5-200 mg, 5-150 mg, 5-125 mg, 5-100 mg, 5-90 mg, 5-70 mg, 5-80 mg, 5-60 mg, 5-50 mg, 5-40 mg, 5-30 mg, 5-25 mg, 5-20 mg, 10-1000 mg, 10-900 mg, 10-800 mg, 10-700 mg, 10-600 mg, 10-500 mg, 10-450 mg, 10-400 mg, 10-300 mg, 10-250 mg, 10-200 mg, 10-150 mg, 10-125 mg, 10-100 mg, 10-90 mg, 10-80 mg, 10-70 mg, 10-60 mg, 10-50 mg, 10-40 mg, 10-30 mg, 10-20 mg; 20-1000 mg, 20-900 mg, 20-800 mg, 20-700 mg, 20-600 mg, 20-500 mg, 20-400 mg, 20-350 mg, 20-300

mg, 20-250 mg, 20-200 mg, 20-150 mg, 20-125 mg, 20-100 mg, 20-90 mg, 20-80 mg, 20-70 mg, 20-60 mg, 20-50 mg, 20-40 mg, 20-30 mg; 50-1000 mg, 50-900 mg, 50-800 mg, 50-700 mg, 50-600 mg, 50-500 mg, 50-400 mg, 50-300 mg, 50-250 mg, 50-200 mg, 50-150 mg, 50-125 mg, 50-100 mg; 100-1000 mg, 100-900 mg, 100-800 mg, 100-700 mg, 100-600 mg, 100-500 mg, 100-400 mg, 100-300 mg, 100-250 mg, or 100-200 mg.

The present invention relates to a pharmaceutical composition or pharmaceutical preparation, comprising a therapeutically effective amount of the compound, or the stereoisomer, deuterated compound, solvate, or pharmaceutically acceptable salt thereof according to the present invention, and an excipient and/or carrier. The pharmaceutical composition can be in a unit preparation form (the amount of the drug substance in the unit preparation is also referred to as the "preparation strength"). In some embodiments, the pharmaceutical composition comprises the compound, or the stereoisomer, deuterated compound, solvate, or pharmaceutically acceptable salt thereof according to the present invention in an amount including but not limited to 1-1440 mg, 5-1000 mg, 10-800 mg, 20-600 mg, 25-500 mg, 40-200 mg, 50-100 mg, 1 mg, 1.25 mg, 2.5 mg, 5 mg, 10 mg, 12.5 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 110 mg, 120 mg, 125 mg, 130 mg, 140 mg, 150 mg, 160 mg, 170 mg, 180 mg, 190 mg, 200 mg, 210 mg, 220 mg, 230 mg, 240 mg, 250 mg, 275 mg, 300 mg, 325 mg, 350 mg, 375 mg, 400 mg, 425 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 850 mg, 900 mg, 950 mg, 1000 mg, 1100 mg, 1200 mg, 1300 mg, 1400 mg, or 1440 mg.

The present invention also provides a method for treating a disease in a mammal, comprising administering to a subject a therapeutically effective amount of the compound, or the stereoisomer, deuterated compound, solvate, or pharmaceutically acceptable salt thereof according to the present invention, and a pharmaceutically acceptable excipient and/or carrier, wherein the therapeutically effective amount is preferably 1-1440 mg, and the disease is preferably cancer.

The present invention also provides a method for treating a disease in a mammal, comprising administering to a subject a drug, i.e., the compound, or the stereoisomer, deuterated material, solvate, or pharmaceutically acceptable salt thereof according to the present invention, and a pharmaceutically acceptable excipient and/or carrier in a daily dose of 1-1440 mg/day, wherein the daily dose can be a single dose or divided doses; in some embodiments, the daily dose includes, but is not limited to 10-1440 mg/day, 20-1440 mg/day, 25-1440 mg/day, 50-1440 mg/day, 75-1440 mg/day, 100-1440 mg/day, 200-1440 mg/day, 10-1000 mg/day, 20-1000 mg/day, 25-1000 mg/day, 50-1000 mg/day, 75-1000 mg/day, 100-1000 mg/day, 200-1000 mg/day, 25-800 mg/day, 50-800 mg/day, 100-800 mg/day, 200-800 mg/day, 25-400 mg/day, 50-400 mg/day, 100-400 mg/day, or 200-400 mg/day, in some embodiments, the daily dose includes, but is not limited to 1 mg/day, 5 mg/day, 10 mg/day, 20 mg/day, 25 mg/day, 50 mg/day, 75 mg/day, 100 mg/day, 125 mg/day, 150 mg/day, 200 mg/day, 400 mg/day, 600 mg/day, 800 mg/day, 1000 mg/day, 1200 mg/day, 1400 mg/day, or 1440 mg/day.

The present invention relates to a kit, which may include a composition in single-dose or multiple-dose form, wherein the kit comprises the compound, or the stereoisomer, deuterated compound, solvate, or pharmaceutically acceptable salt thereof according to the present invention, and the amount of the compound, or the stereoisomer, deuterated compound, solvate, or pharmaceutically acceptable salt thereof according to the present invention is the same as that in the above-mentioned pharmaceutical composition.

In the present invention, the amount of the compound, or the stereoisomer, deuterated compound, solvate, or pharmaceutically acceptable salt thereof according to the present invention is calculated in the form of a free base in each case.

The term "preparation strength" refers to the weight of the drug substance contained in each vial, tablet or other unit preparation.

Synthetic route

Patent document WO 2021013735 A1 introduces a method for preparing a PARP-1 inhibitor, and those skilled in the art would have been able to prepare the compounds of the present invention by means of combining the document and known organic synthesis techniques, wherein the starting materials used therein are commercially available chemicals and (or) compounds described in chemical documents. "Commercially available chemicals" are obtained from regular commercial sources, and suppliers include: Titan Technology Co., Ltd., Energy Chemical Co., Ltd., Shanghai Demo Co., Ltd., Chengdu Kelong Chemical Co., Ltd., Accela ChemBio Co., Ltd., PharmaBlock Sciences (Nanjing), Inc., WuXi Apptec Co., Ltd., J&K Scientific Co., Ltd., etc.

References and monographs in the art introduce in detail the synthesis of reactants that can be used to prepare the compounds described herein, or provide articles describing the preparation method for reference. The references and monographs include: "Synthetic Organic Chemistry", John Wiley & Sons, Inc., New York; S. R. Sandler et al., "Organic Functional Group Preparations," 2nd Ed., Academic Press, New York, 1983; H. O. House, "Modern Synthetic Reactions", 2nd Ed., W. A. Benjamin, Inc. Menlo Park, Calif. 1972; T. L. Gilchrist, "Heterocyclic Chemistry", 2nd Ed., John Wiley & Sons, New York, 1992; J. March, "Advanced Organic Chemistry: Reactions, Mechanisms and Structure", 4th Ed., Wiley-Interscience, New York, 1992; Fuhrhop, J. and Penzlin G. "Organic Synthesis: Concepts, Methods,

Starting Materials", Second, Revised and Enlarged Edition (1994) John Wiley & Sons ISBN: 3 527-29074-5; Hoffman, R.V. "Organic Chemistry, An Intermediate Text" (1996) Oxford University Press, ISBN 0-19-509618-5; Larock, R. C. "Comprehensive Organic Transformations: A Guide to Functional Group Preparations" 2nd Edition (1999) Wiley-VCH, ISBN: 0-471-19031-4; March, J. "Advanced Organic Chemistry: Reactions, Mechanisms, and Structure" 4th Edition (1992) John Wiley & Sons, ISBN: 0-471-60180-2; Otera, J. (editor) "Modern Carbonyl Chemistry" (2000) Wiley-VCH, ISBN: 3-527-29871-1; Patai, S. "Patai's 1992 Guide to the Chemistry of Functional Groups" (1992) Interscience ISBN: 0-471-93022-9; Solomons, T. W. G. "Organic Chemistry" 7th Edition (2000) John Wiley & Sons, ISBN: 0-471-19095-0; Stowell, J.C., "Intermediate Organic Chemistry" 2nd Edition (1993) Wiley-Interscience, ISBN: 0-471-57456-2; "Industrial Organic Chemicals: Starting Materials and Intermediates: An Ullmann's Encyclopaedia" (1999) John Wiley & Sons, ISBN: 3-527-29645-X, in 8 volumes; "Organic Reactions" (1942-2000) John Wiley & Sons, in over 55 volumes; and "Chemistry of Functional Groups", John Wiley & Sons, in 73 volumes.

Specific and similar reactants can be selectively identified by the indexes of known chemicals prepared by the Chemical Abstracts Service of the American Chemical Society, wherein the indexes are available in most public libraries or university libraries and online. Chemicals that are known but not commercially available in the catalogue are optionally prepared by custom chemical synthesis plants, wherein many of standard chemical supply plants (such as those listed above) provide custom synthesis services. Reference document for the preparation and selection of the pharmaceutically acceptable salts of the compounds described herein is P. H. Stahl & C. G. Wermuth "Handbook of Pharmaceutical Salts", Verlag Helvetica Chimica Acta, Zurich, 2002.

## Term

Unless otherwise specified, the terms of the present invention have the following meanings.

The carbon, hydrogen, oxygen, sulphur, nitrogen and halogen involved in the groups and compounds of the present invention all comprise isotopes thereof, and are optionally further substituted with one or more of the corresponding isotopes thereof, wherein the isotopes of carbon comprise $^{12}C$, $^{13}C$ and $^{14}C$; the isotopes of hydrogen comprise protium (H), deuterium (deuterium, also known as heavy hydrogen), and tritium (T, also known as superheavy hydrogen); the isotopes of oxygen comprise $^{16}O$, $^{17}O$ and $^{18}O$; the isotopes of sulphur comprise $^{32}S$, $^{33}S$, $^{34}S$ and $^{36}S$; the isotopes of nitrogen comprise $^{14}N$ and $^{15}N$; the isotope of fluorine comprises $^{19}F$; the isotopes of chlorine comprise $^{35}Cl$ and $^{37}Cl$; and the isotopes of bromine comprise $^{79}Br$ and $^{81}Br$.

The term "halogen" herein refers to F, Cl, Br, I, or isotopes thereof.

The term "halo" or "substituted with halogen" refers to being substituted with one or more groups selected from F, Cl, Br, I, or isotopes thereof, wherein the upper limit of the number of halogen substituents is equal to the sum of the number of hydrogens that can be substituted in the group to be substituted. Without particular limitation, the number of halogen substituents is any integer between 1 and the upper limit, and when the number of halogen substituents is greater than 1, the group to be substituted can be substituted with the same or different halogen. Generally, the circumstances of being substituted with 1-5 halogen, 1-3 halogen, 1-2 halogen, and 1 halogen are included.

The term "deuterium" refers to the isotope deuterium of hydrogen (H).

The term "deuterated" or "deuterated material" refers to the case where a hydrogen atom on a group, such as alkyl, cycloalkyl, alkylene, aryl, heteroaryl, mercapto, heterocycloalkyl, alkenyl and alkynyl is substituted with at least one deuterium atom, wherein the upper limit of the number of deuterium substituents is equal to the sum of the number of hydrogens that can be substituted in the group to be substituted. Without particular limitation, the number of deuterium substituents is any integer between 1 and the upper limit, for example, 1-20 deuterium atoms, 1-10 deuterium atoms, 1-6 deuterium atoms, 1-3 deuterium atoms, 1-2 deuterium atoms or 1 deuterium atom.

Group "$C_{x-y}$" refers to a group comprising x to y carbon atoms, for example, "$C_{1-6}$ alkyl" refers to alkyl comprising 1-6 carbon atoms.

The term "alkyl" refers to a monovalent straight or branched saturated aliphatic hydrocarbon group, usually an alkyl group with 1 to 20 carbon atoms, or an alkyl group with 1 to 8 carbon atoms, or an alkyl group with 1 to 6 carbon atoms, or an alkyl group with 1 to 4 carbon atoms, such as "$C_{1-6}$ alkyl", "$C_{1-5}$ alkyl", "$C_{1-4}$ alkyl", "$C_{1-3}$ alkyl", "$C_{1-2}$ alkyl", "$C_{2-6}$ alkyl", "$C_{2-5}$ alkyl", "$C_{2-4}$ alkyl", "$C_{2-3}$ alkyl", "$C_{3-6}$ alkyl", "$C_{3-5}$ alkyl", "$C_{3-4}$ alkyl", "$C_{4-6}$ alkyl", "$C_{4-5}$ alkyl", and "$C_{5-6}$ alkyl". Non-limiting examples of alkyl include methyl, ethyl, n-propyl, isopropyl, n-butyl, isobutyl, sec-butyl, tert-butyl, n-pentyl, isopentyl, 2-methylbutyl, neopentyl, 1-ethylpropyl, n-hexyl, isohexyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, 1,2-dimethylpropyl, etc. The alkyl can be further substituted with any substituent.

The term "alkylene" refers to a bivalent straight or branched saturated alkyl. Examples of alkylene include, but are not limited to, methylene, ethylidene, *etc.* The alkylene can be optionally further substituted with a substituent.

The term "haloalkyl" refers to an alkyl group in which one or more hydrogens are substituted with one or more halogen atoms (e.g., fluorine, chlorine, bromine, iodine, or isotopes thereof), wherein the upper limit of the number of halogen substituents is equal to the sum of the number of hydrogens that can be substituted in the alkyl group. Without

particular limitation, the number of halogen substituents is any integer between 1 and the upper limit. Generally, the alkyl group is substituted with 1-5 halogen, 1-3 halogen, 1-2 halogen or 1 halogen; and when the number of halogen substituents is greater than 1, the group to be substituted can be substituted with the same or different halogen. Specific examples include, but are not limited to, $-CF_3$, $-CH_2Cl$, $-CH_2CF_3$, $-CCl_2$, $CF_3$, etc.

The term "alkoxy" or "alkyloxy" refers to -O-alkyl, such as -O-$C_{1-8}$ alkyl, -O-$C_{1-6}$ alkyl, -O-$C_{1-4}$ alkyl or -O-$C_{1-2}$ alkyl. Non-limiting and specific examples of alkoxy or alkyloxy include methoxy, ethoxy, n-propoxy, isopropoxy, n-butoxy, sec-butoxy, tert-butoxy, n-pentoxy, n-hexyloxy, cyclopropoxy, cyclobutoxy, etc. The alkoxy may be optionally substituted with a substituent.

The term "haloalkoxy" refers to -O-haloalkyl, such as -O-halo $C_{1-8}$ alkyl, -O-halo $C_{1-6}$ alkyl, -O-halo $C_{1-4}$ alkyl or -O-halo $C_{1-2}$ alkyl; the upper limit of the number of halogen substituents is equal to the sum of the number of hydrogens that can be substituted in the group to be substituted. Without particular limitation, the number of halogen substituents is any integer between 1 and the upper limit, preferably 1-5 halogen, 1 -3 halogen, 1-2 halogen, and 1 halogen; and when the number of halogen substituents is greater than 1, the group to be substituted can be substituted with the same or different halogen. Non-limiting examples of haloalkoxy include monofluoromethoxy, difluoromethoxy, trifluoromethoxy, difluoroethyloxy, *etc.*

The term "alkenyl" refers to a straight or branched hydrocarbon group comprising at least one carbon-carbon double bond (C=C) and generally comprises 2 to 18 carbon atoms, such as 2 to 8 carbon atoms, further such as 2 to 6 carbon atoms, and more further such as 2 to 4 carbon atoms. Examples of alkenyl include, but are not limited to vinyl, allyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 2-methyl-1-butenyl, 2-methyl-3-butenyl, 1-hexenyl, 2-hexenyl, 3-hexenyl, 4-hexenyl, 5-hexenyl, 1-methyl-1-pentenyl, 2-methyl-1-pentenyl, 1-heptenyl, 2-heptenyl, 3-heptenyl, 4-heptenyl, 1-octenyl, 3-octenyl, 1-nonenyl, 3-nonenyl, 1-decenyl, 4-decenyl, 1,3-butadiene, 1,3-pentadiene, 1,4-pentadiene, 1,4-hexadiene, etc.; and the alkenyl may further be optionally substituted with a substituent.

The term "alkenylene" refers to a straight or branched divalent unsaturated hydrocarbon group containing at least one carbon-carbon double bond (C=C). Unless otherwise specified, the alkynylene contains 2-6 carbon atoms, preferably 2-4 carbon atoms. Non-limiting examples of alkynylene include ethynylene, and the alkenylene may be optionally substituted with a substituent.

The term "alkynyl" refers to a straight or branched hydrocarbon group containing at least one carbon-carbon triple bond (C=C) and generally comprises 2 to 18 carbon atoms; further, alkynyl comprises 2 to 8 carbon atoms; further, alkynyl comprises 2 to 6 carbon atoms, and more further, alkynyl comprises 2 to 4 carbon atoms. Examples of alkynyl include, but are not limited to ethynyl, 1-propynyl, 2-propynyl, butynyl, 2-butynyl, 3-butynyl, 1-methyl-2-propynyl, 4-pentynyl, 3-pentynyl, 1-methyl-2-butynyl, 2-hexynyl, 3-hexynyl, 2-heptynyl, 3-heptynyl, 4-heptynyl, 3-octynyl, 3-nonynyl, 4-decynyl, etc.; and the alkynyl may be optionally substituted with a substituent.

The term "alkynylene" refers to a straight or branched, divalent unsaturated hydrocarbon group containing a carbon-carbon triple bond (C=C) and generally comprises 2-6 carbon atoms, and further comprises 2-4 carbon atoms. Non-limiting examples of alkynylene include ethynylene, propynylene, and butynylene, and the alkynylene may be optionally substituted with a substituent.

The term "cycloalkyl" refers to a saturated or partially unsaturated, non-aromatic carbocyclic hydrocarbon group containing no ring heteroatoms. The cycloalkyl may be monocyclic, bicyclic or polycyclic; the bicyclic or polycyclic cycloalkyl may be in the form of a fused ring, a spiro ring, a bridged ring or a combination thereof, and may comprise one or more aromatic rings, but the ring system is non-aromatic as a whole; and the connection site is on a non-aromatic ring. Generally, the cycloalkyl contains 3 to 20 carbon atoms; further, the cycloalkyl contains 3-8 carbon atoms; and still further, the cycloalkyl contains 3-6 carbon atoms; when the cycloalkyl is monocyclic cycloalkyl, the cycloalkyl contains 3-15 carbon atoms, or 3-10 carbon atoms, or 3-8 carbon atoms, or 3-6 carbon atoms; when the cycloalkyl is bicyclic or polycyclic cycloalkyl, the cycloalkyl contains 5-12 carbon atoms, or 5-11 carbon atoms, or 6-10 carbon atoms. Non-limiting examples of cycloalkyl include cyclopropyl, cyclobutyl, cyclopentyl, cyclohexyl, cycloheptyl, butenyl, cyclopentenyl, cyclohexenyl,

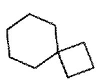, 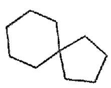, 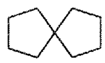, 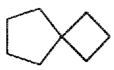,

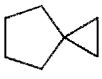, 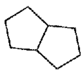, 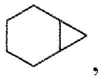, 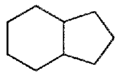, 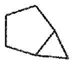, 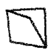, 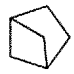, 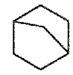, 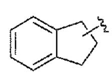,

etc., and the cycloalkyl may be optionally substituted with a substituent.

The term "cycloalkylene" refers to a divalent group of cycloalkyl.

The term "aryl" refers to a substituted or unsubstituted aromatic 5- to 15-membered carbocycle, and includes monocyclic aryl and fused aryl. Aryl is preferably a 5- to 10-membered aromatic ring, further preferably a 5- to 9-membered aromatic ring, and further preferably a 5- to 8-membered aromatic ring. Aryl ring can be fused to an aryl ring and a non-aryl ring (such as a heteroaryl, heterocycloalkyl, or cycloalkyl ring), wherein the aryl ring is the connection site. Non-limiting examples of aryl include phenyl, naphthyl, anthryl, phenanthryl and

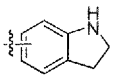, 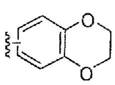, 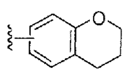, 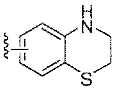, 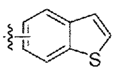, 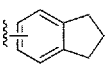, 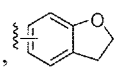, 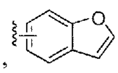,

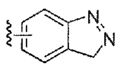, 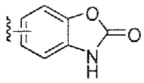, 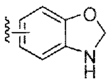, 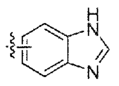, 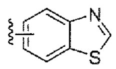, 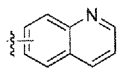 and 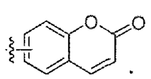. ,

and the aryl may be optionally further substituted with any substituent.

The term "heterocycloalkyl" refers to a saturated or partially unsaturated, non-aromatic carbocycle comprising 1, 2, 3, 4 or 5 heteroatoms selected from N, S, O, P or Si. The heterocycloalkyl may be monocyclic, bicyclic or polycyclic; the bicyclic or polycyclic heterocycloalkyl may be in the form of a bridged ring, a fused ring, a spiro ring or a combination thereof, and may comprise one or more aromatic rings or heteroaromatic rings, but the ring system is non-aromatic as a whole; and the connecting site is on a non-aromatic ring. Usually, the heterocycloalkyl is a 3-20-membered ring. When the heterocycloalkyl is monocyclic heterocycloalkyl, the heterocycloalkyl is usually a 3- to 15-membered ring, or a 3- to 10-membered ring, or a 3-8-membered ring, or a 3-6-membered ring; when the heterocycloalkyl is bicyclic or polycyclic heterocycloalkyl, the heterocycloalkyl is usually a 5- to 12-membered ring, or a 5- to 11-membered ring, or a 6- to 9-membered ring. The heteroatoms N, S and P include their oxidation states C=O, N-O, S=O, $S(=O)_2$, P=O, and $P(=O)_2$. When heterocycloalkyl is a bicyclic or polycyclic ring, at least one ring contains at least one heteroatom, and the heterocycloalkyl can be a bicyclic or polycyclic ring formed by a ring containing the heteroatom(s) and a ring containing no heteroatom, or a bicyclic or polycyclic ring formed by a ring containing the heteroatom(s) and a ring containing the heteroatom(s). When heterocycloalkyl is connected to other groups, the connection site can be at a heteroatom or a carbon atom. Non-limiting examples of heterocycloalkyl include azetidinyl, morpholinyl, piperazinyl, piperidyl, tetrahydropyranyl, oxetanyl, pyranyl, azacyclopentenyl, azacyclohexenyl, oxacyclopentenyl, oxacyclohexenyl, etc., and the heterocycloalkyl may be optionally substituted with a substituent.

The term "heteroaromatic ring" or "heteroaryl", unless otherwise specified, refers to an aromatic ring containing 1 to 5 heteroatoms selected from N, O, S, P, Si and their oxidation states, which can be monocyclic, bicyclic, or polycyclic, wherein the bicyclic or polycyclic heteroaromatic ring or heteroaryl can be bridged rings, fused rings, spiro rings and combinations thereof. Bicyclic or polycyclic heteroaromatic ring or heteroaryl can be formed by fusion of heteroaryl to aryl, of heteroaryl to heteroaryl, or of heteroaryl to cycloalkyl or heterocycloalkyl, wherein the heteroaryl is the connection site. Non-limiting examples of heteroaromatic ring or heteroaryl include furyl, thienyl, pyrrolyl, oxazolyl, thiazolyl, imidazolyl, pyrazolyl, pyridyl, pyrimidinyl, pyridazinyl, pyrazinyl, indolyl, purinyl,

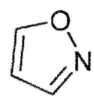,

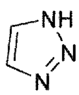, 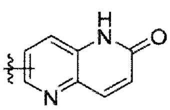, 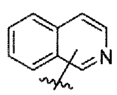, 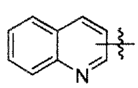,

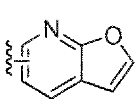, 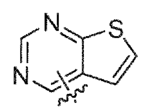, 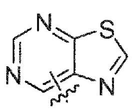, 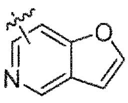,

etc. The heteroaryl may be optionally substituted with a substituent.

The term "aromatic ring" refers to an aromatic ring system containing or containing no N, S, O, P, Si and other heteroatoms, and its definition includes aryl and heteroaryl. The aromatic ring may be optionally substituted with a

substituent.

The term "heterocycle" or "heterocyclyl" refers to a saturated or unsaturated, aromatic or non-aromatic ring containing 1 to 5 heteroatoms selected from N, O, S, P, Si and their oxidation states, and its meaning includes heteroaryl and heterocycloalkyl. Heterocycles include monocyclic heterocycles, bicyclic bridged heterocycles, bicyclic fused heterocycles and bicyclic spiro heterocycles or combinations thereof. The heterocycle is usually a 3- to 12-membered heterocycle, or a 5- to 12-membered heterocycle, or a 5- to 7-membered heterocycle. Heterocyclyl can be connected to a heteroatom or a carbon atom. Non-limiting examples of heterocyclyl include oxiranyl, azacyclopropyl, oxetanyl, azetidinyl, 1,3-dioxolanyl, 1,4-dioxolanyl, 1,3-dioxanyl, piperazinyl, azacycloheptyl, pyridyl, furyl, thienyl, pyranyl, N-alkylpyrrolyl, pyrimidyl, pyrazinyl, pyrazolyl, pyridazinyl, imidazolyl, piperidyl, piperadinyl, morpholinyl, thiomorpholinyl, 1,3-dithianyl, dihydrofuryl, dihydropyranyl, dithiolanyl, tetrahydrofuryl, tetrahydropyrrolyl, tetrahydroimidazolyl, oxazolyl, dihydrooxazolyl, tetrahydrooxazolyl, tetrahydrothiazolyl, tetrahydropyranyl, benzoimidazolyl, benzopyridyl, pyrrolopyridyl, benzodihydrofuryl, azabicyclo[3.2.1]octanyl, azabicyclo[5.2.0]nonanyl, oxatricyclo[5.3.1.1]dodecyl, azaadamantyl and oxaspiro[3.3]heptanyl.

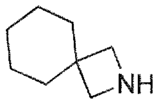, 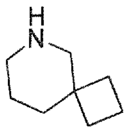, 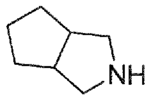, 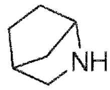, 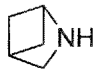,

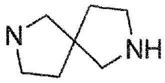, 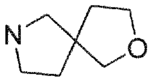, 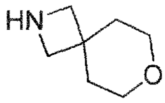, 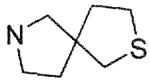,

etc., and the heterocycle may be optionally substituted with a substituent.

The term "spiro ring" refers to a polycyclic group sharing one carbon atom (referred to as a spiro atom) between rings, which may contain 0 or at least 1 double or triple bond, and may contain 0 to 5 heteroatoms selected from N, O, S, P, Si and their oxidation states. Generally, a spiro ring is a 5- to 14-membered ring, or a 5- to 12-membered ring, or a 5- to 10-membered ring. Generally, a spiro ring is a spiro ring formed by a three-membered ring and a three-membered ring, a three-membered ring and a four-membered ring, a three-membered ring and a five-membered ring, a three-membered ring and a six-membered ring, a four-membered ring and a four-membered ring, a four-membered ring and a five-membered ring, a four-membered ring and a six-membered ring, a five-membered ring and a five-membered ring or a five-membered ring and a six-membered ring. The spiro ring may be spirocyclic, non-limiting examples of which include

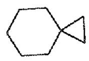 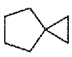 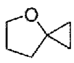 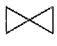 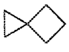 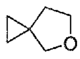 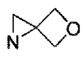 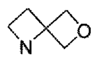 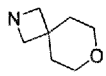

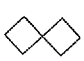 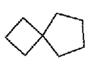 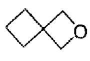 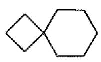 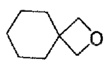 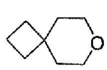 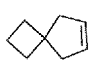 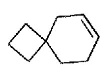

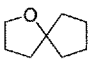 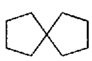 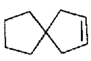 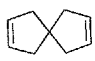 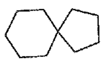 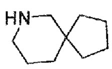 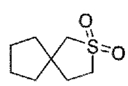 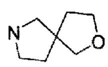,

and the spiro ring may be optionally substituted with a substituent.

The term "bicyclic spiro cycloalkyl" means that both rings forming the spiro ring are cycloalkyl.

The term "bicyclic spiro heterocycloalkyl" means that at least one of the two rings forming the spiro ring is heterocycloalkyl.

The term "bicyclic heteroaromatic ring" refers to an aromatic bicyclic system as a whole, and at least one of the rings in the bicyclic system is a ring containing a heteroatom.

The term "fused ring" refers to a polycyclic group in which the rings share two adjacent ring atoms and one chemical bond, which may contain one or more double or triple bonds, and may contain 0 to 5 heteroatoms selected from N, S, O, P, Si and their oxidation states. Generally, a fused ring is a 5- to 20-membered ring, or a 5- to 14-membered ring, or a 5- to 12-membered ring or a 5- to 10-membered ring. Generally, a fused ring is in the form of a three-membered

ring fused a four-membered ring (indicating a fused ring formed by a three-membered ring and a four-membered ring, and either the three-membered ring or the four-membered ring may be possibly used as the basic ring according to the IUPC nomenclature; similarly hereinafter), a three-membered ring fused a tive-membered ring, a three-membered ring fused a six-membered ring, a four-membered ring fused a four-membered ring, a four-membered ring fused a five-membered ring, a four-membered ring fused a six-membered ring, a five-membered ring fused a five-membered ring, a five-membered ring fused a six-membered ring, and a six-membered ring fused a six-membered ring. Non-limiting examples of fused ring include purine, quinoline, isoquinoline, benzopyran, benzofuran, benzothiophene, and

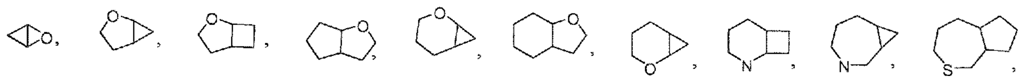

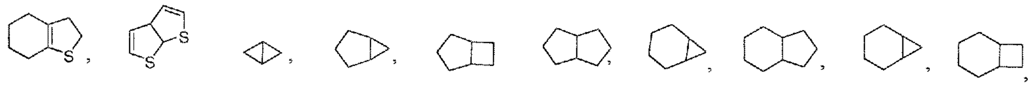

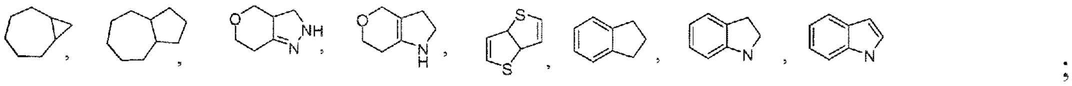

and the fused ring may be optionally substituted with a substituent.

The term "bridged ring" refers to a ring system in which two non-adjacent ring atoms are shared between two rings and a bridged ring may contain 1 or more double or triple bonds. The bridged ring may contain 0 to 5 heteroatoms selected from N, S, O, P, Si and their oxidation states. Generally, the bridged ring has 5 to 20, or 5 to 14, or 5 to 12, or 5 to 10 ring atoms. Non-limiting examples of bridged ring include adamantane,

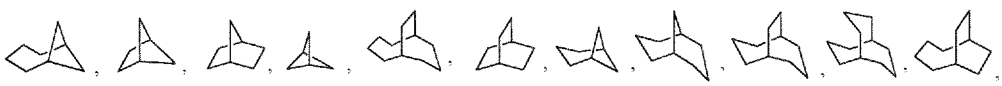

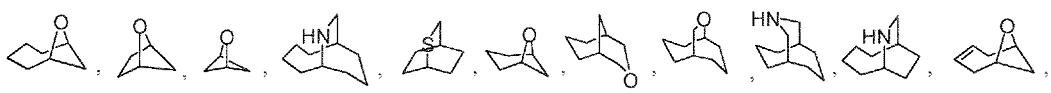

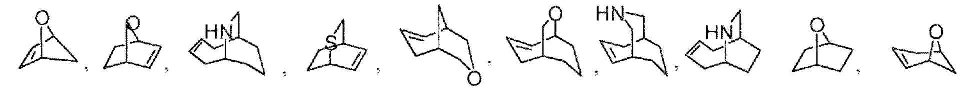

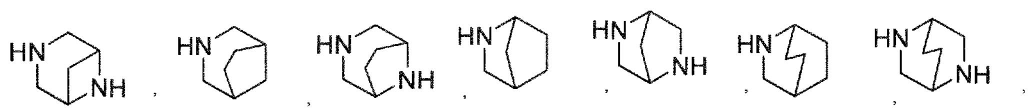

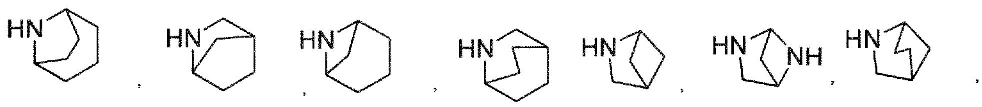

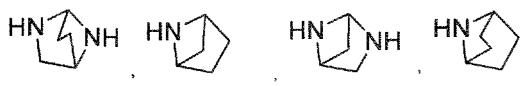

Unless otherwise specified, the term "substitute" or "substituent" refers to any substitution at a position allowed by chemical theory, and the number of substituents conforms to the rules of chemical bonding. Exemplary substituents include, but are not limited to: $C_{1-6}$ alkyl, $C_{2-6}$ alkenyl, $C_{2-6}$ alkynyl, $C_{3-8}$ heteroalkyl, $C_{5-12}$ aryl, 5- to 12-membered heteroaryl, hydroxyl, $C_{1-6}$ alkoxy, $C_{5-12}$ aryloxy, thiol, $C_{1-6}$ alkylthio, cyano, halogen, $C_{1-6}$ alkylthiocarbonyl, $C_{1-6}$ alkylcarbamoyl, N-carbamoyl, nitro, silyl, sulfinyl, sulfonyl, sulfoxide, halo $C_{1-6}$ alkyl, halo $C_{1-6}$ alkoxy, amino, phosphonic acid, $-CO_2(C_{1-6}\,alkyl)$, $-OC(=O)(C_{1-6}\,alkyl)$, $-OCO_2(C_{1-6}\,alkyl)$, $-C(=O)NH_2$, $-C(=O)N(C_{1-6}\,alkyl)_2$, $-OC(=O)NH(C_{1-6}\,alkyl)$,

$-NHC(=O)(C_{1-6}$ alkyl), $-N(C_{1-6}$ alkyl)$C(=O)(C_{1-6}$ alkyl), $-NHCO_2(C_{1-6}$ alkyl), $-NHC(=O)N(C_{1-6}$ alkyl$)_2$, $-NHC(=O)NH(C_{1-6}$ alkyl), $-NHC(=O)NH_2$, $-NHSO_2(C_{1-6}$ alkyl), $-SO_2N(C_{1-6}$ alkyl$)_2$, $-SO_2NH(C_{1-6}$ alkyl), $-SO_2NH_2$, $-SO_2C_{1-6}$ alkyl, etc.

The term "optional" or "optionally" refers to that the events or circumstances subsequently described may but not necessarily occur, and the description includes the occasions where the events or circumstances occur or do not occur. For example, "alkyl optionally substituted with F" means that the alkyl may but not necessarily be substituted with F, and the description includes the case where the alkyl is substituted with F and the case where the alkyl is not substituted with F.

The term "pharmaceutically acceptable salt" refers to a salt of the compound of the present invention, which salt maintains the biological effectiveness and characteristics of a free acid or a free base and is obtained by reacting the free acid with a non-toxic inorganic base or organic base, or reacting the free base with a non-toxic inorganic acid or organic acid.

The term "pharmaceutical composition" represents a mixture of one or more compounds described herein or the stereoisomers, solvates, or pharmaceutically acceptable salts thereof and other components comprising physiologically/pharmaceutically acceptable carriers and/or excipients.

The term "carrier" refers to: a system that does not cause significant irritation to the organism and does not eliminate the biological activity and characteristics of the administered compound, and can change the way the drug enters the human body and the distribution of the drug in the body, control the release rate of the drug and delivery the drug to targeted organs. Non-limiting examples of the carrier include microcapsule, microsphere, nanoparticle, liposome, etc.

The term "excipient" refers to: a substance that is not a therapeutic agent per se, but used as a diluent, adjuvant, adhesive and/or vehicle for addition to a pharmaceutical composition, thereby improving the disposal or storage properties thereof, or allowing to or promoting the formation of a compound or a pharmaceutical composition into a unit dosage form for administration. As is known to those skilled in the art, a pharmaceutically acceptable excipient can provide various functions and can be described as a wetting agent, a buffer, a suspending agent, a lubricant, an emulsifier, a disintegrating agent, an absorbent, a preservative, a surfactant, a colorant, a flavouring agent and a sweetening agent. Examples of pharmaceutically acceptable excipients include, but are not limited to: (1) sugars, such as lactose, glucose and sucrose; (2) starch, such as corn starch and potato starch; (3) cellulose and its derivatives, such as sodium carboxymethyl cellulose, ethyl cellulose, cellulose acetate, hydroxypropyl methylcellulose, hydroxypropyl cellulose, microcrystalline cellulose and croscarmellose (such as croscarmellose sodium); (4) tragacanth powder; (5) malt; (6) gelatine; (7) talc; (8) excipients, such as cocoa butter or suppository wax; (9) oils, such as peanut oil, cottonseed oil, safflower oil, sesame oil, olive oil, corn oil and soybean oil; (10) diols, such as propylene glycol; (11) polyols, such as glycerine, sorbitol, mannitol and polyethylene glycol; (12) esters, such as ethyl oleate and ethyl laurate; (13) agar; (14) buffers, such as magnesium hydroxide and aluminium hydroxide; (15) alginic acid; (16) pyrogen-free water; (17) isotonic saline; (18) Ringer's solution; (19) ethanol; (20) pH buffered solution; (21) polyester, polycarbonate and/or polyanhydride; and (22) other non-toxic compatible substances used in a pharmaceutical preparation.

The term "stereoisomer" refers to an isomer produced as a result of different spatial arrangement of atoms in molecules, including cis-trans isomers, enantiomers and conformational isomers.

The compounds of the present invention also include tautomers thereof, for example, when the present invention describes the left side compound in which the pyrimidine ring is substituted with OH, the right side tautomer compound is also included.

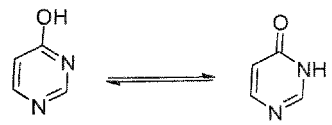

The term "solvate" refers to a substance formed by the compound of the present invention or the salt thereof and a stoichiometric or non-stoichiometric solvent bound by intermolecular non-covalent forces. When the solvent is water, the solvate is a hydrate.

The term "cocrystal" refers to a crystal formed by the combination of active pharmaceutical ingredient (API) and co-crystal former (CCF) under the action of hydrogen bonds or other non-covalent bonds. The pure state of API and CCF are both solid at room temperature, and there is a fixed stoichiometric ratio among various components. The cocrystal is a multi-component crystal, which includes both a binary cocrystal formed between two neutral solids and a multi-element cocrystal formed between a neutral solid and a salt or solvate.

Detailed Description of Embodiments

The content of the present invention is described in detail with the following examples. If a specific condition

is not indicated in the examples, a conventional condition is used in an experimental method. The listed examples are intended to better illustrate the content of the present invention, but should not be construed as limiting the content of the present invention. According to the above-mentioned content of the invention, those skilled in the art can make unsubstantial modifications and adjustments to the embodiments, which still fall within the protection scope of the present invention.

Detection method

The structure of the compound is determined by nuclear magnetic resonance (NMR) or (and) mass spectrometry (MS). The NMR shift (δ) is given in the unit of 10-6 (ppm). NMR is determined with Bruker Avance III 400 and Bruker Avance 300; the solvents for determination are deuterated dimethyl sulfoxide (DMSO-d6), deuterated chloroform (CDCl3) and deuterated methanol (CD3OD); and the internal standard is tetramethylsilane (TMS);

MS is determined with Agilent 6120B (ESI) and Agilent 6120B (APCI);
HPLC is determined with Agilent 1260DAD high pressure liquid chromatograph (Zorbax SB-C18 100 × 4.6 mm, 3.5 μM);
Yantai Huanghai HSGF254 or Qingdao GF254 silica gel plate is used as a thin layer chromatography silica plate, and the silica gel plate for the thin layer chromatography (TLC) is of the specification of 0.15 mm-0.20 mm, and the specification when separating and purifying a product by thin layer chromatography is 0.4 mm - 0.5 mm;
and for the column chromatography, Yantai Huanghai silica gel of 200-300 mesh silica gel is generally used as a carrier.

Example 1:

N-methyl-5-(4-((6-oxo-7-vinyl-5,6-dihydro-1,5-naphthyridin-3-yl)methyl)piperazin-1-yl)picolinamide (Compound 1)

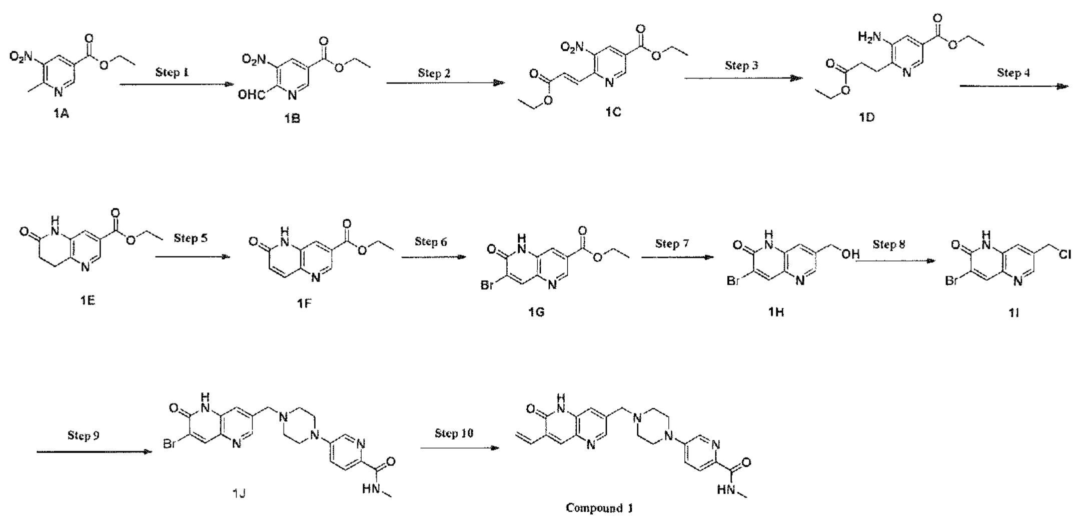


Step 1:

Compound 1A (20 g, 95.21 mmol) and selenium dioxide (21.13 g, 190.42 mmol) were added to 1,4-dioxane solvent (200 mL), and the mixture was heated to reflux, stirred for 3 hours, cooled, filtered, and directly purified and separated by silica gel column chromatography (petroleum ether: ethyl acetate (v/v) = 1 : 0-5 : 1), to afford the title compound (20 g, yield: 94%).

LCMS m/z =225.1 $[M+1]^+$
$^1H$ NMR (400 MHz, $CDCl_3$) δ 10.31 (s, 1H), 9.50 (d, 1H), 8.80 (d, 1H), 4.52 (q, 2H), 1.47 (t, 3H).

Step 2:

Under nitrogen atmosphere, sodium hydride (8.9 g, 223.23 mmol) was slowly added to anhydrous tetrahydrofuran solvent, and the mixture was cooled to 0°C in an ice bath. Ethyl 2-(diethoxyphosphoryl)acetate (50 g, 223.23 mmol) was then slowly added dropwise to the above-mentioned solution, and the mixture was reacted for half an hour. Subsequently, the solution was cooled to -78°C, reagent 1B (20 g, 89.29 mmol) dissolved in anhydrous tetrahydrofuran was slowly added dropwise to the above-mentioned reaction liquid, and the mixture was reacted for 1 hour. After the reaction was completed, the reaction liquid was quenched with saturated ammonium chloride solution, extracted with ethyl acetate, dried over anhydrous sodium sulphate and filtered, and the filtrate was concentrated under reduced pressure and separated by silica gel column chromatography (petroleum ether: ethyl acetate (v/v) = 1 : 0-3 : 1), to afford the title compound (15 g, yield: 57%).

LCMS m/z =295.1 $[M+1]^+$
$^1$H NMR (400 MHz, DMSO-*d6*) δ 9.32 (d, 1H), 8.79 (d, 1H), 8.07 (d, 1H), 7.08 (d, 1H), 4.42 (q, 2H), 4.25 (q, 2H), 1.37 (t, 3H), 1.28 (t, 3H).

Step 3:

Reagent 1C (15 g, 51.02 mmol) was added to ethanol (200 mL), palladium on carbon was added, and the mixture was subjected to hydrogen replacement and reacted at room temperature under hydrogen atmosphere for 16 hours. After the reaction was completed, the reaction liquid was filtered with diatomaceous earth, to afford the title compound (12 g, yield: 92%).

LCMS m/z =267.1 $[M+1]^+$
$^1$H NMR (400 MHz, DMSO-*d6*) δ 8.21 (d, 1H), 7.45 (d, 1H), 5.44 (s, 2H), 4.29 (d, 2H), 4.05 (d, 2H), 2.86 (d, 2H), 2.75 (d, 2H), 1.30 (t, 3H), 1.16 (t, 3H).

Step 4:

Reagent 1D (12 g, 45.11 mmol) was added to hydrogen chloride/1,4-dioxane solution (200 mL), and the mixture was reacted at room temperature for 1 hour. After the reaction was completed, the reaction liquid was directly concentrated, to afford the title compound (9 g, yield: 91%).
LCMS m/z =221.1 $[M+1]^+$

Step 5:

Reagent 1E (9 g, 40.91 mmol) and dichlorodicyanobenzoquinone (11.2 g, 49.09 mmol) were added to 1,4-dioxane (200 mL), and the mixture was reacted at reflux for 2 hours. After the reaction was completed, saturated aqueous sodium bicarbonate solution was added, and the mixture was stirred for half an hour, filtered, and washed with saturated aqueous sodium bicarbonate solution, water and ether to afford a solid, which was spun to dryness to afford the title compound (8 g, yield: 90%).

LCMS m/z =219.1 $[M+1]^+$
$^1$H NMR (400 MHz, DMSO-*d6*) δ 12.03 (s, 1H), 8.90 (d, 1H), 8.18 (d, 1H), 7.99 (d, 1H), 6.88 (d, 1H), 4.39 (q, 2H), 1.36 (t, 3H).

Step 6:

Reagent 1F (4 g, 18.18 mmol) and sodium bromate (5.49 g, 36.36 mmol) were added to a solution of hydrogen bromide in acetic acid (50 mL), and the mixture was reacted at 100°C for 16 hours. After the reaction was completed, the reaction liquid was concentrated, washed with saturated sodium bicarbonate, extracted and concentrated, to afford a crude product (7 g).

LCMS m/z =297.0 $[M+1]^+$
$^1$H NMR (400 MHz, DMSO-*d6*) δ 11.04 (s, 1H), 8.94 (d, 1H), 8.58 (s, 1H), 8.21 (d, 1H), 4.39 (q, 2H), 1.36 (t, 3H).

Step 7:

Reagent 1G (2 g, crude) was added to tetrahydrofuran solvent (70 mL), diisobutylaluminium hydride (32 ml, 1.5 mol/L in toluene) was slowly added in an ice bath, and the mixture was reacted at this temperature for 2 hours. After the reaction was completed, the reaction liquid was quenched with water and concentrated. The crude product was eluted with dichloromethane/methanol = 10 : 1, concentrated, and purified by reverse-phase HPLC ($H_2O$:$CH_3CN$=1:0-10:1), to afford the title compound (400 mg, 33%).
LCMS m/z =255.0 $[M+1]^+$

Step 8:

Reagent 1H (400 mg) was added to dry dichloromethane solvent (70 mL), thionyl chloride (32 ml, 1.5 mol/L in toluene) was slowly added in an ice bath, 2 drops of DMF was added dropwise, and the mixture was stirred overnight at room temperature. After the reaction was completed, the reaction liquid was concentrated to dryness, to afford the title compound (420 mg, 100%).
LCMS m/z =273.0 $[M+1]^+$

Step 9:

Raw material 1I (150 mg, 0.58 mmol), N-methyl-5-(piperazin-1-yl)picolinamide (158 mg, 0.58 mmol), DIPEA (0.48 mL, 2.9 mmol) and potassium iodide (190 mg, 1.16 mmol) were added to DMF (6 mL) solvent, and then the mixture was heated to 70°C and reacted for 6 hours. The reaction liquid was filtered, and the filtrate was concentrated under reduced pressure to dryness and purified by preparative TLC (DCM:MeOH=10:1), to afford a target compound (100 mg, yield: 38%).
LCMS m/z =457.1 $[M+1]^+$

Step 10:

Reagent 1J (50 mg, 0.11 mmol), tributylvinyltin (67 mg, 0.22 mmol), potassium carbonate (15 mg, 0.11 mmol), tetraethylammonium chloride (24 mg, 0.22 mmol), and bis(triphenylphosphine)palladium(II) dichloride (77 mg, 0.11 mmol) were added to dry DMF solvent (10 mL), and under nitrogen protection, the mixture was heated to 100°C, stirred for 5 hours, cooled and filtered. The filtrate was concentrated under reduced pressure and purified by preparative TLC (DCM:MeOH=10:1), to afford the title compound (5 mg, 11%).

LCMS m/z=405.2 $[M+1]^+$
$^1H$ NMR (400 MHz, $CD_3OD$) δ8.48 (d, 1H), 8.18 (d, 1H), 7.80 (d, 1H), 7.67 (s, 1H), 7.47-7.54 (m, 1H), 7.25-7.28 (m, 1H), 6.87 (t, 1H), 6.10 (d, 1H), 5.60 (d, 1H), 3.66 (s, 2H), 3.30-3.32 (m, 4H), 2.83 (s, 3H),2.58-2.60 (m, 4H).

Example 2:

5-(4-((7-cyclopropyl-6-oxo-5,6-dihydro-1,5-naphthyridin-3-yl)methyl)piperazin-1-yl)-N-methylpicolinamide (Compound 2)

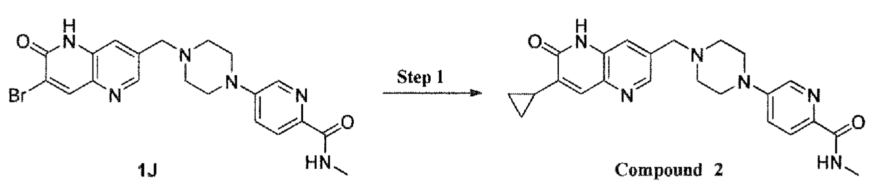


To a reaction flask were added 1J (300 mg, 0.66 mmol), cyclopropylboronic acid (70 mg, 0.81 mmol), caesium carbonate (430 mg, 1.32 mmol) and dioxane (6 mL), the reaction mixture was subjected to nitrogen replacement for protection, and then Pd(dppf)$Cl_2$ (90 mg, 0.12 mmol) was added. After the addition was completed, the mixture was heated to 100°C and reacted for 4 h. After the reaction was completed, to the reaction liquid was added water (20 mL), and then the mixture was extracted with ethyl acetate (50 mL). The organic phase was separated, and the aqueous phase was extracted with ethyl acetate (50 mL×2). The organic phases were combined, washed with saturated brine

(50 mL×2), dried over anhydrous sodium sulphate, filtered and concentrated, to afford a crude, which was purified by column chromatography (eluent: dichloromethane: methanol = 10 : 1), to afford a product (60 mg). The product was further purified by preparative HPLC (Preparation method: instrument: waters 2767 preparative liquid phase chromatographic instrument); chromatographic column: SunFire@ Prep C18 (19 mm×250 mm); the sample was dissolved in DMF and filtered with a 0.45 μm filter to prepare a sample liquid; Preparative chromatography conditions: a. composition of mobile phases A and B: mobile phase A: acetonitrile, and mobile phase B: water (containing 5 mM ammonia water); b. gradient elution, mobile phase A: 25%-85%; c. flow rate: 15 mL/min; d. elution time: 15 min; retention time: 6.0 min), to afford compound 2 (18 mg).

LCMS m/z = 419.3 $[M+1]^+$
$^1$H NMR (400 MHz, $CD_3OD$) δ 8.47 (d, 1H), 8.29 (d, 1H), 7.91 (d, 1H), 7.77 (s, 1H), 7.52 (s, 1H), 7.40 - 7.36 (m, 1H), 3.75 (s, 2H), 3.43 - 3.41 (m, 4H), 2.95 (s, 3H), 2.71 - 2.68 (m, 4H), 2.23 - 2.19 (m, 1H), 1.11 - 1.07 (m, 2H), 0.86 - 0.84 (m, 2H).

Example 3

N-cyclopropyl-1'-((7-cyclopropyl-6-oxo-5,6-dihydro-1,5-naphthyridin-3-yl)methyl)-1',2',3',6'-tetrahydro-[3,4'-bipyridine]-6-carboxamide (Compound 3)

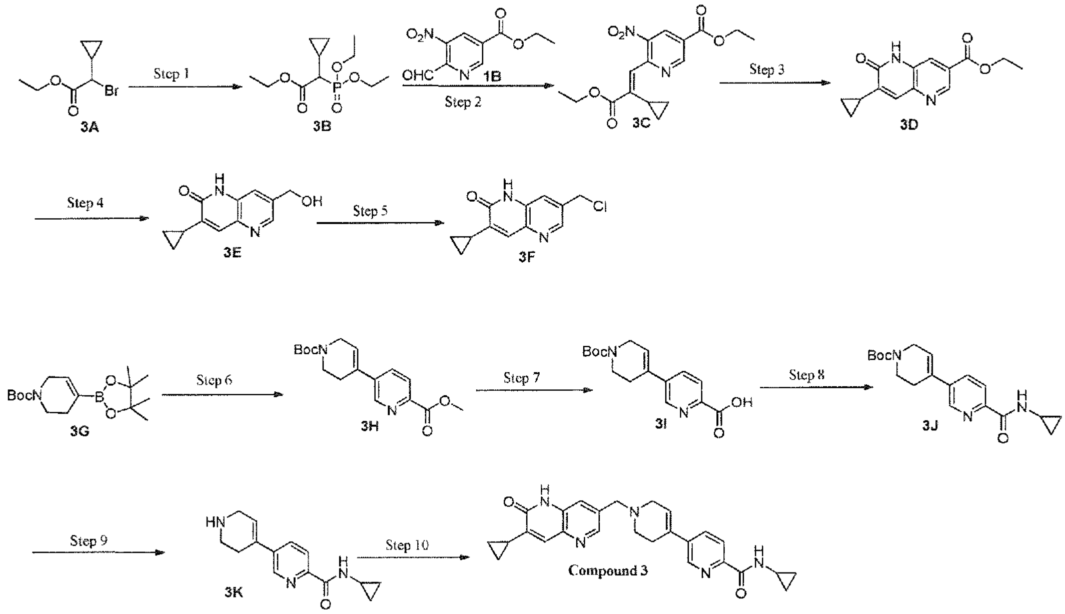


Step 1:

Ethyl 2-bromo-2-cyclopropylacetate 3A (30.0 g, 144.89 g) was weighed and dissolved in triethyl phosphite (150 mL), and then the mixture was heated to 130°C in a sealed tube and reacted for 20 h. After the reaction was completed, the reaction liquid was cooled to room temperature and subjected to rotary evaporation to remove excess triethyl phosphite, to afford crude target compound 3B (38.29 g, yield: 100.00%), which was directly used in the subsequent reaction. LC-MS (ESI): m/z= 265.2 $[M+H]^+$.

Step 2:

NaH (9.28 g, 231.96 mmol, 60%) was weighed and dissolved in ultra-dry tetrahydrofuran (300 mL), and then the mixture was cooled to about -10°C. 3B (38.29 g, 144.97 mmol) was weighed and dissolved in tetrahydrofuran (50 mL), and then the solution of 3B in THF was slowly added to the reaction liquid. After the dropwise addition was completed, the mixture was slowly naturally warmed to room temperature and stirred for 30 min. After the reaction was completed, the solution was cooled to -70°C, 1B (13.0 g, 57.99 mmol) was then dissolved in ultra-dry tetrahydrofuran (50 mL), and the solution was slowly added dropwise to the reaction liquid. After the dropwise addition was completed, the reaction

process was monitored by TLC spot plate (ethyl acetate:petroleum ether = 1 : 8). After the reaction was completed, to the reaction liquid were added saturated aqueous ammonium chloride solution (50 mL) and water (50 mL), and the mixture was extracted with ethyl acetate (400 mL). The organic phase was separated, and the aqueous phase was extracted once with ethyl acetate (200 mL). The organic phases were combined and concentrated to afford a crude, which was purified by column chromatography (eluent: ethyl acetate:petroleum ether = 1 : 8), and then the eluent was concentrated, to afford target compound 3C (12.9 g, 66.54%).
LC-MS (ESI): m/z= 334.2 $[M+H]^+$.

Step 3:

Compound 3C (12.9 g, 38.59 mmol) was weighed and dissolved in a mixed solution of anhydrous ethanol (260 mL) and water (40 mL), and then ammonium chloride (30.96 g, 578.85 mmol) and reduced iron powder (21.55 g, 385.9 mmol) were added. After the addition was completed, the mixture was heated to 80°C and reacted for 2 h. After the reaction was completed, the reaction liquid was cooled to room temperature, to the reaction liquid were added saturated aqueous sodium bicarbonate solution and ethyl acetate (400 mL), and the mixture was filtered with diatomaceous earth. The filter cake was washed with ethyl acetate (100 mL), and the organic phase in the filtrate was separated, dried over anhydrous sodium sulphate and concentrated to afford a crude, which was slurried with a mixed solvent (30 mL, ethyl acetate:petroleum ether = 10 : 1), filtered and dried, to afford target compound 3D (4.67 g, 46.86%).

LC-MS (ESI): m/z= 259.1 $[M+H]^+$.
$^1$H NMR (400 MHz, DMSO-*d6*) δ 12.03 (s, 1H), 8.87 - 8.86 (m, 1H), 8.14 (s, 1H), 7.48 (s, 1H), 4.40 - 4.35 (m, 2H), 2.23 - 2.16 (m, 1H), 1.37 - 1.33 (m, 3H), 1.05 - 1.01 (m, 2H), 0.92 - 0.90 (m, 2H).

Step 4:

Compound 3D (4.6 g, 17.81 mmol) was weighed and dissolved in ultra-dry tetrahydrofuran (230 mL), and then the mixture was cooled to -60°C with dry-ice ethanol. Lithium aluminium hydride (2.7 g, 71.24 mmol) was weighed and slowly added to the reaction liquid. After the addition was completed, the mixture was slowly warmed to about -40°C and reacted for another 2 h. After the reaction was completed as monitored by LCMS, to the reaction liquid was slowly added a small amount of water (20 mL), the mixture was filtered to afford a solid, which was dried, heated to reflux with a mixed solution of dichloromethane and methanol (dichloromethane : MeOH = 1 : 2, 300 mL), and filtered to afford a filtrate, and the filtrate was concentrated to afford an off-white solid. This process was repeated four times as such, and the resulting products were combined and concentrated, to afford crude target compound 3E (4.6 g), which was directly used in the subsequent reaction.
LC-MS (ESI): m/z= 217.1 $[M+H]^+$.

Step 5:

Compound 3E (4.6 g, 21.27 mmol) was weighed and dissolved in a mixed solution of dichloromethane (70 mL) and tetrahydrofuran (70 mL), and then DMF (2 mL) was added. After the addition was completed, the mixture was cooled to below 0°C, and then thionyl chloride (9 mL) was slowly added. After the addition was completed, the mixture was kept at this temperature and reacted for 10 min. After the reaction was completed as monitored by TLC spot plate (dichloromethane:methanol = 15 : 1), to the reaction liquid was added saturated aqueous sodium bicarbonate solution. After the addition was completed, the solution was alkaline (pH = 8-9) and then was extracted with ethyl acetate (400 mL×3). The organic phases were combined, washed with saturated brine, dried over anhydrous sodium sulphate and concentrated, to afford crude target compound 3F (0.95 g, yield: 19.03%).
LC-MS (ESI): m/z= 235.1 $[M+H]^+$.

Step 6:

Methyl 5-bromopyridine-2-carboxylate (0.7 g, 3.24 mmol), 3G (1.0 g, 3.24 mmol), chloro[2-(dicyclohexylphosphino)-3,6-dimethoxy-2',4',6'-triisopropyl-1,1'-biphenyl][2-(2-aminoethyl)phenyl]palladium (II) (RuPhos-Pd-G2, 0.26 mg, 0.32 mmol), and potassium carbonate (0.60 g, 4.44 mmol) were dissolved in anhydrous DMF (30 mL), and the mixture was reacted at 100°C under nitrogen protection for 3 hours. After the reaction was completed as monitored by LCMS, the reaction liquid was spun to dryness to remove the solvent, purified by column chromatography (eluent: petroleum ether : ethyl acetate = 1 : 3) and concentrated, to afford target compound 3H (1.00 g, yield: 97.08%).
LC-MS (ESI): m/z= 319.2 $[M+H]^+$.

Step 7:

3H (0.3 g, 0.94 mmol) was weighed and dissolved in dioxane (5 ml) and water (1 mL), sodium hydroxide (0.5 g, 12.5 mmol) was added, and the mixture was stirred and reacted at 60°C for 2 hours. After the reaction was completed as monitored by LCMS, 0.5M hydrochloric acid was added to adjust to pH = 3, and the mixture was extracted twice with ethyl acetate and concentrated, to afford crude target compound 31 (0.30 g), which was directly used in the next reaction.
LC-MS (ESI): m/z= 305.1 $[M+H]^+$.

Step 8:

31 (0.3 g, 0.99 mmol) was dissolved in N,N-dimethylformamide (10 mL), diisopropylethylamine (0.38 g, 2.97 mmol) and 2-(7-aza-1H-benzotriazol-1-yl)-1,1,3,3-tetramethyluronium hexafluorophosphate (0.75 g, 1.97 mmol) were then added, and the mixture was stirred at room temperature for half an hour. Cyclopropylamine (0.14 g, 2.46 mmol) was added, and the mixture was stirred for another 1 hour. Water was added for dilution, and the mixture was extracted three times with ethyl acetate (10 ml × 3). The organic phase was washed with saturated brine (50 mL × 3), dried over anhydrous sodium sulphate, concentrated and purified by column chromatography (eluent: ethyl acetate:petroleum ether = 10 : 1), to afford target compound 3J (0.29 g, 85.20%).
LCMS m/z =344.3 $[M+1]^+$

Step 10:

Compound 3J (0.29 g, 0.85 mmol) was weighed and dissolved in dichloromethane (8 mL), a solution of hydrogen chloride in dioxane (1 mL, 4 mmol/L) was then added, and the mixture was stirred at room temperature for 20 h. After the reaction was completed, the reaction liquid was concentrated under reduced pressure, to afford target compound 3K hydrochloride (0.27 g), which was directly used in the next reaction.
LCMS m/z =244.1 $[M+1]^+$

Step 11:

Compound 3K hydrochloride (0.10 g, 0.36 mmol) and compound 3F (0.076 g, 0.33 mmol) were weighed and dissolved in anhydrous DMF (5 mL), diisopropylethylamine (0.5 mL) and potassium iodide (0.12 g, 0.72 mmol) were then added, and the mixture was heated to 80°C, stirred and reacted for 1 h. After the reaction was completed as monitored by LCMS, to the reaction liquid were added water (10 mL) and ethyl acetate (50 mL). The organic phase was separated, and the aqueous phase was extracted twice with ethyl acetate (30 mL×2). The organic phases were combined, washed successively with water (50 mL) and saturated brine (50 mL), dried over anhydrous sodium sulphate, filtered and concentrated to afford a crude, which was purified by preparative TLC (developing agent: dichloromethane : methanol = 10 : 1), to afford target compound 3 (0.048 g, 31.54%).
LC-MS (ESI): m/z= 442.50 $[M+H]^+$.
$^1$H NMR (400 MHz, DMSO-$d_6$) δ 11.84 (s, 1H), 8.66 - 8.63 (m, 2H), 8.39(s, 1H), 8.01 - 7.95 (m, 2H), 7.62 (s, 1H), 7.42 (s, 1H), 6.40 (s, 1H), 3.71 (s, 2H), 3.15 - 3.14 (m, 2H), 2.90 - 2.89 (m, 1H), 2.72 - 2.68 (m, 2H), 2.54 (s, 2H), 2.17 - 2.11 (m, 1H), 0.98 - 0.96 (m, 2H), 0.83 -0.82 (m, 2H), 0.70 - 0.66 (m, 4H).

Example 4

5-(4-((7-cyclopropyl-6-oxo-5,6-dihydro-1,5-naphthyridin-3-yl)methyl)piperazin-1-yl)-N-(cyclopropylmethyl)picolinamide (Compound 4)

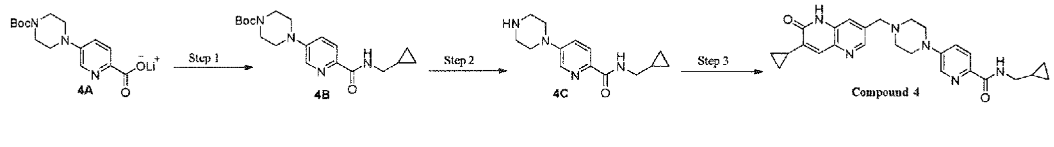


Step 1:

Compound 4A (0.313 g, 1.0 mmol) (synthesized according to the synthesis method in patent US 2018141923A1) was weighed and dissolved in DMF (10 mL), HATU (570 mg, 1.5 mmol) was then added under stirring, and the mixture

was stirred at room temperature. After the solid was completely dissolved, DIEPA (1 mL) was added, cyclopropylmethylamine (0.14 g, 2 mmol) was finally added, and the mixture was stirred overnight at room temperature. After the reaction was completed as monitored by LCMS, to the system was added water (50 mL) under stirring, at which time a large amount of white solid was observed to be precipitated out, and the mixture was stirred for another 10 min, filtered and dried under vacuum, to afford target compound 4B (0.332 g, 92.2%).

LC-MS (ESI): m/z = 361.2 $[M+H]^+$.

Step 2:

4B (0.332 g, 0.92 mmol) was dissolved in methanol (5 mL), and hydrogen chloride/1,4-dioxane (5 mL, 4M) solution was added. The mixture was reacted at room temperature for 4 hours and spun to dryness, to afford target compound 4C (0.28 g, crude), which was directly used in the subsequent reaction.

LC-MS (ESI): m/z= 261.2 $[M+H]^+$.

Step 3:

Compound 4C (0.10 g, 0.35 mmol) and compound 3F (0.076 g, 0.33 mmol) were weighed and dissolved in anhydrous DMF (5 mL), diisopropylethylamine (0.5 mL) and potassium iodide (0.12 g, 0.72 mmol) were then added, and the mixture was heated to 80°C, stirred and reacted for 1 h. After the reaction was completed as monitored by LCMS, to the reaction liquid were added water (10 mL) and ethyl acetate (50 mL). The organic phase was separated, and the aqueous phase was extracted twice with ethyl acetate (30 mL×2). The organic phases were combined, washed successively with water (50 mL) and saturated brine (50 mL), dried over anhydrous sodium sulphate, filtered and concentrated to afford a crude, which was purified by preparative TLC (developing agent : dichloromethane:methanol = 10 : 1), to afford target compound 4 (0.047 g, 29.01%).

LC-MS (ESI): m/z= 459.70 $[M+H]^+$.

$^1$H NMR (400 MHz, DMSO-$d_6$) δ 11.86 (s, 1H), 8.43 - 8.38 (m, 2H), 8.28 - 8.27 (m, 1H), 7.85 - 7.83 (m, 1H), 7.61 (s, 1H), 7.42 - 7.38 (m, 2H), 3.64 (s, 2H), 3.34 - 3.33 (m, 4H), 3.15 - 3.12 (m, 2H), 2.57 - 2.55 (m, 4H), 2.16 - 2.12 (m, 1H), 1.05 - 1.01 (m, 1H), 0.99 - 0.95 (m, 2H), 0.84 - 0.80 (m, 2H), 0.43 - 0.38 (m, 2H), 0.24 - 0.22 (m, 2H).

Example 5

N-cyclopropyl-5-(4-((7-cyclopropyl-6-oxo-5,6-dihydro-1,5-naphthyridin-3-yl)methyl)piperazin-1-yl)picolinamide (Compound 5)

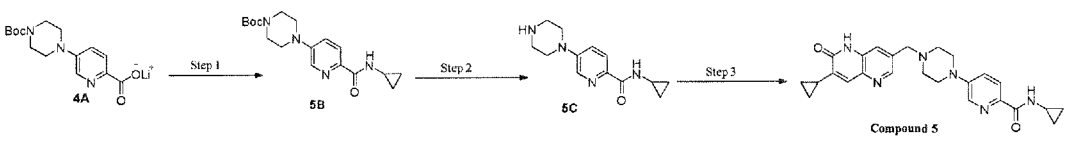


Step 1:

Compound 4A (0.313 g, 1.0 mmol) was weighed and dissolved in DMF (10 mL), HATU (570 mg, 1.5 mmol) was then added under stirring, and the mixture was stirred at room temperature. After the solid was completely dissolved, DIEPA (1 mL) was added, cyclopropylamine (0.12 g, 2 mmol) was finally added, and the mixture was stirred overnight at room temperature. After the reaction was completed as monitored by LCMS, to the system was added water (50 mL) under stirring, at which time a large amount of white solid was observed to be precipitated out, and the mixture was stirred for another 10 min, filtered and dried under vacuum, to afford target compound 5B (0.309 g, 89.2%).

LC-MS (ESI): m/z = 347.2 $[M+H]^+$.

Step 2:

5B (0.309 g, 0.89 mmol) was dissolved in methanol (5 mL), and hydrogen chloride/1,4-dioxane (5 mL, 4M) solution was added. The mixture was reacted at room temperature for 4 hours and spun to dryness, to afford target compound 5C (0.27 g, crude), which was directly used in the subsequent reaction.

LC-MS (ESI): m/z= 247.2 $[M+H]^+$.

Step 3:

Compound 5C (0.10 g, crude) and compound 3F (0.076 g, 0.33 mmol) were weighed and dissolved in anhydrous DMF (5 mL), diisopropylethylamine (0.5 mL) and potassium iodide (0.12 g, 0.72 mmol) were then added, and the mixture was heated to 80°C, stirred and reacted for 1 h. After the reaction was completed as monitored by LCMS, to the reaction liquid were added water (10 mL) and ethyl acetate (50 mL). The organic phase was separated, and the aqueous phase was extracted twice with ethyl acetate (30 mL×2). The organic phases were combined, washed successively with water (50 mL) and saturated brine (50 mL), dried over anhydrous sodium sulphate, filtered and concentrated to afford a crude, which was purified by preparative TLC (developing agent : dichloromethane:methanol = 10 : 1), to afford compound 5 (0.054 g, 37.2%).

LC-MS (ESI): m/z= 445.1 $[M+H]^+$.

$^1H$ NMR (400 MHz, DMSO-$d_6$) δ 11.85 (s, 1H), 8.68 - 8.63 (m, 1H), 8.41 - 8.35 (m, 1H), 8.29 - 8.21 (m, 1H), 7.86 - 7.79 (m, 1H), 7.61 (s, 1H), 7.44 - 7.35 (m, 2H), 3.64 (s, 2H), 3.41 - 3.36 (m, 4H), 2.90 -2.78 (m, 1H), 2.57 - 2.55 (m, 4H), 2.44 - 2.28 (m, 1H), 2.20 - 2.09 (m, 2H), 1.00 - 0.93 (m, 2H), 0.89 - 0.75 (m, 2H), 0.71 - 0.56 (m, 2H).

Example 6

5-(4-((7-cyclopropyl-6-oxo-5,6-dihydro-1,5-naphthyridin-3-yl)methyl)piperazin-1-yl)-N-(1-methyl-1H-pyrazol-4-yl)picolinamide (Compound 6)

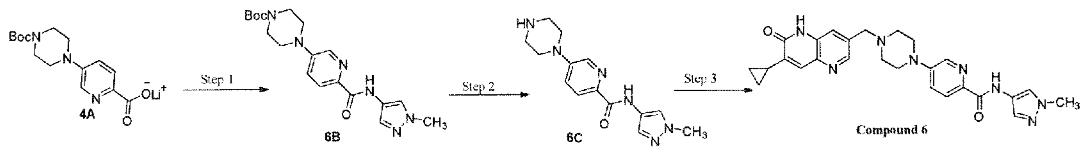


Step 1:

Compound 4A (0.313 g, 1.0 mmol) was weighed and dissolved in DMF (10 mL), HATU (570 mg, 1.5 mmol) was then added under stirring, and the mixture was stirred at room temperature. After the solid was completely dissolved, DIEPA (1 mL) was added, 1-methyl-1H-pyrazol-4-amine (0.19 g, 2 mmol) was finally added, and the mixture was stirred overnight at room temperature. After the reaction was completed as monitored by LCMS, to the system was added water (50 mL) under stirring, at which time a large amount of white solid was observed to be precipitated out, and the mixture was stirred for another 10 min, filtered and dried under vacuum, to afford target compound 6B (0.342 g, 88.5%).

LC-MS (ESI): m/z = 387.2 $[M+H]^+$.

Step 2:

6B (0.342 g, 0.88 mmol) was dissolved in methanol (5 mL), and hydrogen chloride/1,4-dioxane (5 mL, 4M) solution was added. The mixture was reacted at room temperature for 4 hours and spun to dryness, to afford target compound 6C (0.29 g, crude), which was directly used in the subsequent reaction.

LC-MS (ESI): m/z= 287.2 $[M+H]^+$.

Step 3:

Compound 6C (0.12 g, crude) and compound 3F (0.076 g, 0.33 mmol) were weighed and dissolved in anhydrous DMF (5 mL), diisopropylethylamine (0.5 mL) and potassium iodide (0.12 g, 0.72 mmol) were then added, and the mixture was heated to 80°C, stirred and reacted for 1 h. After the reaction was completed as monitored by LCMS, to the reaction liquid were added water (10 mL) and ethyl acetate (50 mL). The organic phase was separated, and the aqueous phase was extracted twice with ethyl acetate (30 mL×2). The organic phases were combined, washed successively with water (50 mL) and saturated brine (50 mL), dried over anhydrous sodium sulphate, filtered and concentrated to afford a crude, which was purified by preparative TLC (developing agent : dichloromethane:methanol = 10 : 1), to afford compound 6 (0.077 g, 48.1%).

LC-MS (ESI): m/z= 485.2 $[M+H]^+$.

$^1H$ NMR (400 MHz, DMSO-$d_6$) δ 11.85 (s, 1H), 10.49 (s, 1H), 8.40 - 8.37 (m, 1H), 8.33 - 8.29 (m, 1H), 8.02 (s, 1H), 7.94 - 7.88 (m, 1H), 7.70 - 7.67 (m, 1H), 7.63 - 7.59 (m, 1H), 7.66 - 7.40 (m, 2H), 3.81 (s, 3H), 3.65 (s, 2H), 3.41 -

3.33 (m, 4H), 2.61 - 2.53 (m, 4H), 2.20 - 2.09 (m, 1H), 1.01 - 0.93 (m, 2H), 0.86 - 0.78 (m, 2H).

Example 7

5-(4-((7-cyclopropyl-6-oxo-5,6-dihydro-1,5-naphthyridin-3-yl)methyl)piperazin-1-yl)-N-((3,3-difluorocyclobutyl)methyl)picolinamide (Compound 7)

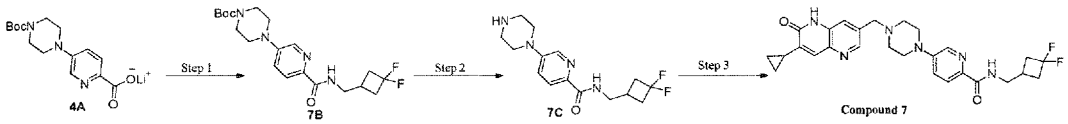


4A 7B 7C Compound 7

Step 1:

Compound 4A (0.313 g, 1.0 mmol) was weighed and dissolved in DMF (10 mL), HATU (570 mg, 1.5 mmol) was then added under stirring, and the mixture was stirred at room temperature. After the solid was completely dissolved, DIEPA (1 mL) was added, 3,3-difluorocyclobutylmethylamine (0.24 g, 2 mmol) was finally added, and the mixture was stirred overnight at room temperature. After the reaction was completed as monitored by LCMS, to the system was added water (50 mL) under stirring, at which time a large amount of white solid was observed to be precipitated out, and the mixture was stirred for another 10 min, filtered and dried under vacuum, to afford target compound 7B (0.374 g, 91.3%).

LC-MS (ESI): m/z = 411.2 $[M+H]^+$.

Step 2:

7B (0.374 g, 0.88 mmol) was dissolved in methanol (5 mL), and hydrogen chloride/1,4-dioxane (5 mL, 4M) solution was added. The mixture was reacted at room temperature for 4 hours and spun to dryness, to afford target compound 7C (0.31 g, crude), which was directly used in the subsequent reaction.

LC-MS (ESI): m/z= 311.2 $[M+H]^+$.

Step 3:

Compound 7C (0.15 g, crude) and compound 3F (0.076 g, 0.33 mmol) were weighed and dissolved in anhydrous DMF (5 mL), diisopropylethylamine (0.5 mL) and potassium iodide (0.12 g, 0.72 mmol) were then added, and the mixture was heated to 80°C, stirred and reacted for 1 h. After the reaction was completed as monitored by LCMS, to the reaction liquid were added water (10 mL) and ethyl acetate (50 mL). The organic phase was separated, and the aqueous phase was extracted twice with ethyl acetate (30 mL×2). The organic phases were combined, washed successively with water (50 mL) and saturated brine (50 mL), dried over anhydrous sodium sulphate, filtered and concentrated to afford a crude, which was purified by preparative TLC (developing agent: dichloromethane:methanol = 10 : 1), to afford compound 7 (0.067 g, 40.1%).

LC-MS (ESI): m/z= 509.2 $[M+H]^+$.

$^1$H NMR (400 MHz, DMSO-$d_6$) δ 11.85 (s, 1H), 8.68 - 8.60 (m, 1H), 8.39 - 8.36 (m, 1H), 8.28 - 8.25 (m, 1H), 7.86 - 7.81 (m, 1H), 7.63 - 7.59 (m, 1H), 7.47 - 7.32 (m, 2H), 3.64 (s, 2H), 3.43 - 3.31 (m, 6H), 2.64 - 2.52 (m, 6H), 2.45 - 2.28 (m, 3H), 2.19 - 2.10 (m, 1H), 1.01 - 0.94 (m, 2H), 0.86 - 0.79 (m, 2H).

Example 8

3-cyclopropyl-7-((4-(2-(trifluoromethyl)imidazo[1,2-a]pyrazin-6-yl)piperazin-1-yl)methyl)-1,5-naphthyridin-2(1H)-one (Compound 8)

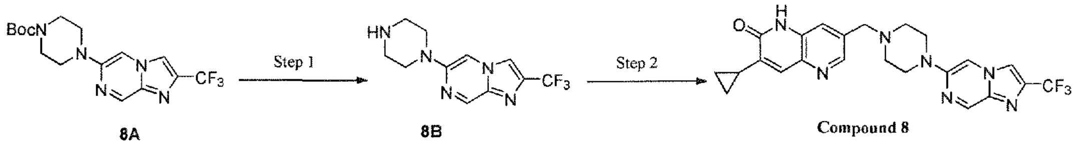


8A 8B Compound 8

Step 1:

8A (0.371 g, 1 mmol) was dissolved in methanol (5 mL), and hydrogen chloride/1,4-dioxane (5 mL, 4M) solution was added. The mixture was reacted at room temperature for 4 hours and spun to dryness, to afford target compound 8B (0.29 g, crude), which was directly used in the subsequent reaction.

LC-MS (ESI): m/z= 272.2 $[M+H]^+$.

Step 2:

Compound 8B (0.15 g, crude) and compound 3F (0.076 g, 0.33 mmol) were weighed and dissolved in anhydrous DMF (5 mL), diisopropylethylamine (0.5 mL) and potassium iodide (0.12 g, 0.72 mmol) were then added, and the mixture was heated to 80°C, stirred and reacted for 1 h. After the reaction was completed as monitored by LCMS, to the reaction liquid were added water (10 mL) and ethyl acetate (50 mL). The organic phase was separated, and the aqueous phase was extracted twice with ethyl acetate (30 mL×2). The organic phases were combined, washed successively with water (50 mL) and saturated brine (50 mL), dried over anhydrous sodium sulphate, filtered and concentrated to afford a crude, which was purified by preparative TLC (developing agent: dichloromethane:methanol = 10 : 1), to afford compound 8 (0.034 g, 21.9%).

LC-MS (ESI): m/z= 470.1 $[M+H]^+$.

$^1H$ NMR (400 MHz, DMSO-$d_6$) $\delta$ 11.85 (s, 1H), 8.99 (s, 1H), 8.46 (s, 1H), 8.42 - 8.38 (m, 1H), 7.92 (s, 1H), 7.62 (s, 1H), 7.42 (s, 1H), 3.65 (s, 2H), 3.42 - 3.34 (m, 4H), 2.62 - 2.53 (m, 4H), 2.20 - 2.10 (m, 1H), 1.01 - 0.94 (m, 2H), 0.87 - 0.77 (m, 2H).

Biological test

1. PARP-1 Enzyme Activity Test Experiment

PARP-1 chemical fluorescence detection kit was purchased from BPS Bioscience. The histone solution in the kit was diluted 5X with 1X PBS, and 25 μL of the diluted histone solution was added to a microwell plate and incubated overnight at 4°C. After the incubation, the plate was washed three times with PBST (0.05% Tween-20). 100 μL of the blocking solution was added to the microwell plate and incubated at 25°C for 90 minutes. After the incubation, the plate was washed three times with PBST. 2.5 μL of compounds at different concentrations diluted in test buffer and 12.5 μL of substrate mixed solution (1.25 μL 10X PARP test buffer; 1.25 μL 10X PARP test mixed solution; 2.5 μL Activated DNA, 7.5 μL double-distilled water) were added to the microwell plate. The PARP-1 enzyme was diluted to 2 ng/μL, 10 μL of the diluent was added to the microwell plate, and the reaction system was incubated at 25°C for 60 minutes.

After the incubation, the plate was washed three times with PBST. Streptavidin-HRP was diluted 50X with a blocking solution, and 25 μL of the diluent was added to the microwell plate and incubated at 25°C for 30 minutes. After the incubation, the plate was washed three times with PBST. ELISA ECL substrate A and substrate B were mixed at a ratio of 1:1 (v/v), 50 μL of the mixture was added to the microwell plate, and the chemiluminescence value was read.

The inhibition rate was calculated according to formula 1, where RLUsample was the readout of the compound well, RLUmax was the readout of the solvent control well, and RLUmin was the readout of the control well without the PARP-1 enzyme. Curve fitting was performed by four parameters (log(inhibitor) vs. response -- Variable slope) using GraphPad Prism software, and the IC50 value was calculated.

$$\text{Inhibition\%} = (1-(\text{RLUsample}-\text{RLUmin})/(\text{RLUmax}-\text{RLUmin})) \times 100\%$$

(formula 1)

Experimental results: the compound of the present invention had a significant inhibitory effect on PARP-1 enzyme activity in vitro, and the $IC_{50}$ value of the example compounds on PARP-1 enzyme activity was less than 100 μM. $IC_{50}$ values are represented by grades A, B, C, and D, where A represents $IC_{50} \leq 1$ nM, B represents $IC_{50} \leq 10$ nM, C represents $IC_{50} \leq 50$ nM, and D represents $IC_{50} \leq 100$ nM. The test results of some examples were shown in Table 1.

Table 1 PARP-1 enzyme activity

| Compound | $IC_{50}$ (nM) |
|---|---|
| 2 | A |
| 3 | A |
| 5 | A |
| 6 | A |

Conclusion: the compounds of the present invention have significant inhibitory effects on PARP-1 enzyme activity in vitro, especially compounds 2, 3, 5, 6 and 8, the $IC_{50}$ values of which on PARP-1 are 0.9 nM, 0.6 nM, 0.74 nM, 0.54 nM and 1.2 nM, respectively.

2. MDA-MB-436 Cell Activity Test Experiment

Breast tumour cells MDA-MB-436 were purchased from ATCC, the culture medium was Leibovitz's L-15 + 10% FBS, and the cells were cultured in a $CO_2$-free incubator at 37°C. On day 1, the cells in the exponential growth phase were collected, and the cell suspension was adjusted to 4000 cells/135 μL with the culture medium. 135 μL of the cell suspension was added to each well of a 96-well cell culture plate and incubated overnight. On day 2, compounds at different concentrations were added, and the plate was placed in the incubator and incubated for 7 days. After the incubation was completed, according to operation instructions for a CellTiter-Glo kit (Promega, G7573), 75 μL of CTG solution, which was already pre-melted and equilibrated to room temperature, was added to each well, and the mixture was uniformly mixed for 2 min using a microplate shaker. The plate was placed at room temperature for 10 min, and then fluorescence signal values were measured using an Envision2104 plate reader (PerkinElmer). The inhibition rate was calculated using formula (1), where $RLU_{compound}$ was the readout of the drug treated group, $RLU_{control}$ was the average value of the vehicle control group, and $RLU_{blank}$ was the average value of the cell-free well. The $IC_{50}$ value was calculated using GraphPad Prism software.

$$Inh.\% = (1-(RLU_{compound} - RLU_{blank})/(RLU_{control} - RLU_{blank})) \times 100\% \text{ (formula 1)}$$

Test results: the compounds of the present invention had a significant inhibitory effect on MDA-MB-436 cells, the IC50 value of the compounds on MDA-MB-436 cells was less than 100 nM, the $IC_{50}$ value of some excellent compounds on MDA-MB-436 cells was less than 10 nM, and the $IC_{50}$ value of more excellent compounds on MDA-MB-436 cells was less than 1 nM; and the inhibitory rate of the compounds on breast tumour cells MDA-MB-436 was greater than 70%, the inhibition rate of some excellent compounds was greater than 85%, and the inhibition rate of more excellent compounds was greater than 90%. The results of some specific compounds were as shown in Table 2.

Table 2 MDA-MB-436 cell activity

| Compound | $IC_{50}$ (nM) | Max inh.% 10 μM |
|---|---|---|
| 2 | 5.1 | 86.3 |
| 3 | 4.5 | 82.1 |
| 5 | 5.6 | 80.7 |
| 6 | 2.8 | 85.4 |
| 8 | 1.1 | 97.4 |

Conclusion: the compound of the present invention has good inhibitory activity on breast tumour cells MDA-MB-436.

3. Pharmacokinetic test in mice

3.1 Experimental animals: male ICR mice, 20-25 g, 18 mice/compound. purchased from Chengdu Ddossy Experimental Animals Co., Ltd.

3.2 Experimental design: on the day of the test, 18 ICR mice were randomly grouped according to their body weights. the animals were fasted with water available for 12 to 14 h one day before the administration, and were fed 4 h after the administration.

Table 3. Administration information

| Group | Quantity | Administration information | | | | | |
|---|---|---|---|---|---|---|---|
| | Male | Test compound | Administration dosage (mg/kg) | Administration concentration (mg/mL) | Administration volume (mL/kg) | Collected samples | Mode of administration |
| G3 | 9 | Compound 3 | 2.5 | 0.2 | 5 | Plasma | Intravenously |
| G4 | 9 | | 10 | 1 | 10 | Plasma | Intragastrically |

Notes: Solvent for intravenous administration: 5% DMA+5% Solutol+90% Saline; Solvent for intragastric administration: 5% DMSO+30% PEG400+65% (20% SBE-CD) (DMA: dimethylacetamide; Solutol: polyethylene glycol-15-hydroxystearate; Saline: physiological saline; MC: methylcellulose)

Before and after the administration, 0.06 mL of blood was taken from the orbit of the animals under isoflurane anaesthesia and placed in an EDTAK2 centrifuge tube. The blood was centrifuged at 5000 rpm and 4°C for 10 min to collect plasma. The blood collection time points for the intravenous group and intragastric administration group were: 0 min, 5 min, 15 min, 30 min, 1 h, 2 h, 4 h, 6 h, 8 h, and 24 h. Before analysis and detection, all samples were stored at -80°C, and a quantitative analysis of samples was performed using LC-MS/MS.
The test results were shown in Table 4.

Table 4 Pharmacokinetic parameters of test compounds in plasma of mice

| Test compound | Mode of administration | CL (mL/min/kg) | $Vd_{ss}$ (L/kg) | $AUC_{0-t}$ (hr*ng/mL) | F (%) |
|---|---|---|---|---|---|
| Compound 3 | i.v. (2.5 mg/kg) | 0.346 | 0.207 | 107581 | - |
| | i.g. (10 mg/kg) | - | - | 707389 | 164 |
| -: not applicable. | | | | | |

Conclusion: compound 3 has good pharmacokinetic characteristics in mice.

4. Pharmacokinetic Test in Rats

1.1 Experimental animals: male SD rats, about 220 g, 6-8 weeks old, 6 rats/compound, purchased from Chengdu Ddossy Experimental Animals Co., Ltd.
1.2 Experimental design: on the day of the test, 6 SD rats were randomly grouped according to their body weight; the animals were fasted with water available for 12 to 14 h one day before the administration, and were fed 4 h after the administration.

Table 5. Administration information

| Group | Quantity | Administration information | | | | | |
|---|---|---|---|---|---|---|---|
| | Male | Test compound | Administration dosage (mg/kg) | Administration concentration (mg/mL) | Administration volume (mL/kg) | Collected samples | Mode of administration |
| G1 | 3 | Compound 2 | 2.5 | 0.5 | 5 | Plasma | Intravenously |
| G2 | 3 | | 10 | 1 | 10 | Plasma | Intragastrically |
| Notes: Solvent for intravenous administration: 5% DMA+5% Solutol+90% Saline; Solvent for intragastric administration: 5% DMSO+30% PEG400+65% (20% SBE-CD) (DMA: dimethylacetamide; Solutol: polyethylene glycol-15-hydroxystearate; Saline: physiological saline; MC: methylcellulose) | | | | | | | |

Before and after the administration, 0.15 ml of blood was taken from the orbit of the animals under isoflurane anaesthesia and placed in an EDTAK2 centrifuge tube. The blood was centrifuged at 5000 rpm and 4°C for 10 min to collect plasma. The blood collection time points for the intravenous administration group and intragastric administration group were: 0, 5 min, 15 min, 30 min, 1 h, 2 h, 4 h, 6 h, 8 h, and 24 h. Before analysis and detection, all samples were stored at -80°C, and a quantitative analysis of samples was performed using LC-MS/MS.

The test results were shown in Table 6.

Table 6 Pharmacokinetic parameters of test compounds in plasma of rats

| Test compound | Mode of administration | CL (mL/min/kg) | $Vd_{ss}$ (L/kg) | $AUC_{0-t}$ (hr*ng/mL) | F (%) |
|---|---|---|---|---|---|
| Compound 2 | i.v. (2.5 mg/kg) | 0.585 | 0.169 | 70806 | - |
| | i.g. (10 mg/kg) | - | - | 205512 | 72.6 |
| -: not applicable. | | | | | |

Conclusion: compound 2 has good pharmacokinetic characteristics in rats.

## Claims

1. A compound as shown in formula (I), or a stereoisomer, solvate, deuterated compound, or pharmaceutically acceptable salt thereof,

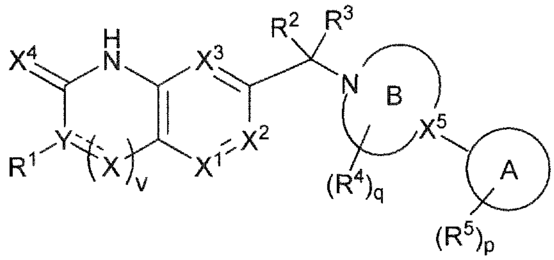

(I)

**characterized in that** X is selected from $CR^x$, $C(R^x)_2$, O, N or $NR^x$;
Y is selected from N, C or CH;
------- represents a single bond or a double bond;
v is selected from 1, 2 or 3;
$X^1$, $X^2$ and $X^3$ are each independently selected from N or $CR^x$;
provided that when = represents a double bond, and v is selected from 1, X, $X^1$, $X^2$ and $X^3$ are not all selected from $CR^x$,
$X^4$ is selected from O or S;
$X^5$ is selected from N or $CR^x$;
each $R^x$ is independently selected from H, D, halogen, cyano, amino, hydroxyl, $-SF_5$, $C_{1-6}$ alkyl, halo $C_{1-6}$ alkyl, halo $C_{1-6}$ alkoxy, deuterated $C_{1-6}$ alkyl, deuterated $C_{1-6}$ alkoxy, $C_{1-6}$ alkoxy, $C_{2-6}$ alkenyl, $C_{2-6}$ alkynyl, $C_{1-6}$ alkyl-O-$C_{1-6}$ alkyl, $-(CH_2)_r-C_{3-12}$ cycloalkyl or $-(CH_2)_r$-(3- to 12-membered heterocycloalkyl); or two $R^x$ on the same carbon atom together form =O;
$R^1$ is selected from halogen, nitro, cyano, amino, hydroxyl, $-SF_5$, $C_{1-6}$ alkyl, $C_{1-6}$ alkoxy, $C_{2-6}$ alkenyl, $C_{2-6}$ alkynyl, $C_{1-6}$ alkyl-O-$C_{1-6}$ alkyl, $-(CH_2)_r-C_{3-12}$ cycloalkyl or $-(CH_2)_r$-(3- to 12-membered heterocycloalkyl), wherein the alkyl, alkoxy, alkenyl, alkynyl, cycloalkyl or heterocycloalkyl is optionally further substituted with 1-3 groups selected from D, halogen, cyano, amino, hydroxyl, $C_{1-6}$ alkyl or $C_{1-6}$ alkoxy;
each r is independently selected from 0, 1, 2 or 3;
$R^2$ and $R^3$ are each independently selected from H, D, halogen, cyano, amino, hydroxyl, $C_{1-6}$ alkyl-O-$C_{1-6}$ alkyl, hydroxy $C_{1-6}$ alkyl, $C_{1-6}$ alkoxy, halo $C_{1-6}$ alkyl, halo $C_{1-6}$ alkoxy, deuterated $C_{1-6}$ alkyl, deuterated $C_{1-6}$ alkoxy or $C_{1-6}$ alkyl; or $R^2$ and $R^3$ together with the carbon atom to which they are attached form $C_{3-5}$ cycloalkyl or 4- to 5-membered heterocycloalkyl;
each $R^4$ is independently selected from D, halogen, cyano, amino, hydroxyl, $-SF_5$, $C_{1-6}$ alkyl, $C_{1-6}$ alkoxy, halo $C_{1-6}$ alkyl, halo $C_{1-6}$ alkoxy, deuterated $C_{1-6}$ alkyl or deuterated $C_{1-6}$ alkoxy; or two $R^4$ on the same carbon atom

together with the carbon atom to which they are attached form =O;
each $R^5$ is independently selected from D, halogen, cyano, amino, hydroxyl, - $SF_5$, $C_{1-6}$ alkyl, $C_{1-6}$ alkoxy, halo $C_{1-6}$ alkyl, halo $C_{1-6}$ alkoxy, deuterated $C_{1-6}$ alkyl or deuterated $C_{1-6}$ alkoxy;
q is selected from 0, 1, 2 or 3; p is selected from 0, 1, 2 or 3;
ring B is 5- to 6-membered saturated monocyclic heterocycloalkane containing 1-2 nitrogen atoms, 5- to 6-membered partially unsaturated monocyclic heterocycloalkane containing 1-2 nitrogen atoms, 6- to 8-membered saturated bridged heterocycle containing 1-4 nitrogen atoms, 5- to 10-membered saturated fused heterocycle containing 1-4 nitrogen atoms, or 5- to 11-membered saturated spiro heterocycle containing 1-4 nitrogen atoms;
ring A is selected from 5- to 6-membered monocyclic heteroaromatic ring containing 1-5 nitrogen, oxygen or sulphur atoms, wherein the heteroaromatic ring is further substituted with 1-3 substituents selected from $R_a$; or ring A is selected from 7- to 10-membered bicyclic heteroaromatic ring containing 1-5 nitrogen, oxygen or sulphur atoms, or 7- to 10-membered bicyclic aromatic ring, wherein the heteroaromatic ring or aromatic ring is optionally further substituted with 1-3 substituents selected from $R_b$;
$R_a$ is selected from $-C(O)N(R^{a1})_2$, $-NR^{a1}C(O)R^{a1}$, $-C(O)R^{a1}$, $-NR^{a1}$, - $NR^{a1}C(O)OR^{a1}$, $-NR^{a1}C(O)N(R^{a1})_2$, $-C(=S)N(R^{a1})_2$, $-S(O)_2N(R^{a1})_2$, 5- to 6-membered monocyclic heteroaryl containing 1-5 nitrogen, oxygen or sulphur atoms, 4- to 7-membered monocyclic heterocycloalkyl containing 1-4 nitrogen, oxygen or sulphur atoms, or 3- to 7-membered monocyclic cycloalkyl, wherein the heteroaryl, heterocycloalkyl, or cycloalkyl is optionally further substituted with 1-3 substituents selected from D, halogen, cyano, hydroxyl, amino, $-NHC_{1-6}$ alkyl, $-N(C_{1-6}$ alkyl$)_2$, $C_{1-6}$ alkyl, halo $C_{1-6}$ alkyl, $C_{1-6}$ alkoxy, halo $C_{1-6}$ alkoxy, deuterated $C_{1-6}$ alkyl, or deuterated $C_{1-6}$ alkoxy;
$R_b$ is selected from $-C(O)N(R^{a1})_2$, $-NR^{a1}C(O)OR^{a1}$, $-NR^{a1}C(O)N(R^{a1})_2$, - $C(=S)N(R^{a1})_2$, $-S(O)_2N(R^{a1})_2$, =O, D, halogen, cyano, hydroxyl, amino, $-NHC_{1-6}$ alkyl, $-N(C_{1-6}$ alkyl$)_2$, $C_{1-6}$ alkyl, $C_{3-12}$ cycloalkyl, 3- to 12-membered heterocycloalkyl, $C_{1-6}$ alkoxy, $C_{1-6}$ alkyl-O-$C_{1-6}$ alkyl, halo $C_{1-6}$ alkyl, halo $C_{1-6}$ alkoxy, deuterated $C_{1-6}$ alkyl or deuterated $C_{1-6}$ alkoxy;
each $R^{a1}$ is independently selected from H, D, $C_{1-6}$ alkyl, $C_{3-12}$ cycloalkyl, 3- to 12-membered heterocycloalkyl, 5- to 10-membered heteroaryl, $C_{1-6}$ alkoxy, $C_{1-6}$ alkyl-O-$C_{1-6}$ alkyl, $C_{1-6}$ alkyl-O-$C_{3-12}$ cycloalkyl, halo $C_{1-6}$ alkyl, halo $C_{1-6}$ alkoxy, deuterated $C_{1-6}$ alkyl, or deuterated $C_{1-6}$ alkoxy, wherein the alkyl, cycloalkyl, heterocycloalkyl, or heteroaryl is optionally further substituted with 1-3 substituents selected from D, halogen, cyano, hydroxyl, amino, $C_{1-6}$ alkyl, halo $C_{1-6}$ alkyl, deuterated $C_{1-6}$ alkyl, $C_{3-6}$ cycloalkyl or phenyl;
provided that:

(3) when - - - - - represents a single bond, $X^1$, $X^2$ and $X^3$ are all selected from $CR^x$, and v is selected from 1, X is selected from $C(R^x)_2$ or $NR^x$;
(4) when

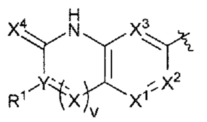

is selected from

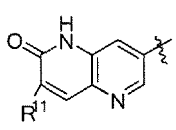, 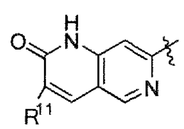,

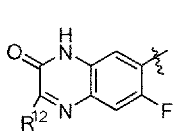 and 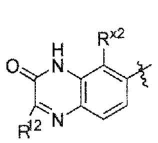,

wherein $R^{x2}$ is selected from H, $C_{1-6}$ alkyl or halogen, $R^{12}$ is selected from H, $C_{1-6}$ alkyl, halo $C_{1-6}$ alkyl, $C_{1-6}$ alkoxy or $C_{3-6}$ cycloalkyl, $R^{11}$ is selected from $C_{1-6}$ alkyl, and $R^2$ is selected from H or D, $R^3$ is not selected from H or D;

unless otherwise specified, the above-mentioned heterocycloalkane, heterocycloalkyl, heteroaryl, or heteroaromatic ring contains 1-5 heteroatoms selected from nitrogen, oxygen or sulphur.

2. The compound, or the stereoisomer, solvate, deuterated compound, or pharmaceutically acceptable salt thereof according to claim 1, **characterized in that** $R^2$ and $R^3$ are each independently selected from H, D, halogen, cyano, amino, hydroxyl, $C_{1-6}$ alkyl-O-$C_{1-6}$ alkyl, hydroxy $C_{1-6}$ alkyl, $C_{1-6}$ alkoxy, halo $C_{1-6}$ alkyl, halo $C_{1-6}$ alkoxy, deuterated $C_{1-6}$ alkyl, deuterated $C_{1-6}$ alkoxy or $C_{1-6}$ alkyl; or $R^2$ and $R^3$ together with the carbon atom to which they are attached form $C_{3-5}$ cycloalkyl or 4- to 5-membered heterocycloalkyl; provided that when $R^2$ is selected from H or D, $R^3$ is not selected from H or D.

3. The compound, or the stereoisomer, solvate, deuterated compound, or pharmaceutically acceptable salt thereof according to claim 1, having a structure of formula (II) or (II-b):

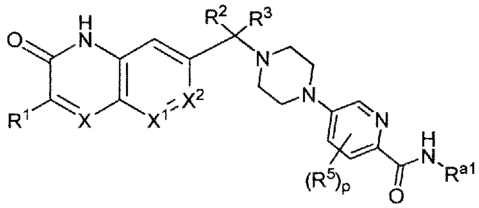

(II)

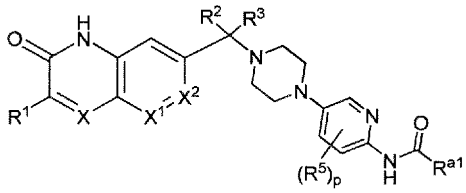

(II-b)

**characterized in that** X is selected from $CR^x$ or N;
$X^1$ and $X^2$ are each independently selected from N or $CR^x$;
p is selected from 0 or 1;
$R^x$ is independently selected from H, D, halogen, cyano, amino, hydroxyl, $C_{1-2}$ alkyl, $C_{1-2}$ alkoxy, $C_{2-4}$ alkenyl, $C_{2-4}$ alkynyl, $C_{1-2}$ alkyl-O-$C_{1-2}$ alkyl, -$(CH_2)_r$-$C_{3-5}$ cycloalkyl, -$(CH_2)_r$-$C_{5-9}$ bicyclic spiro cycloalkyl, -$(CH_2)_r$-(4- to 5-membered heterocycloalkyl), or -$(CH_2)_r$-(5- to 9-membered bicyclic spiro heterocycloalkyl);
$R^1$ is selected from $C_{1-6}$ alkyl or halo $C_{1-6}$ alkyl;
$R^5$ is selected from D, halogen, cyano, amino, hydroxyl, $C_{1-4}$ alkyl, $C_{1-4}$ alkoxy, halo $C_{1-4}$ alkyl, halo $C_{1-4}$ alkoxy, deuterated $C_{1-4}$ alkyl, or deuterated $C_{1-4}$ alkoxy;
each $R^{a1}$ is independently selected from $C_{1-4}$ alkyl, $C_{3-5}$ cycloalkyl, $C_{5-9}$ bicyclic spiro cycloalkyl, 4- to 6-membered heterocycloalkyl, 5- to 9-membered bicyclic spiro heterocycloalkyl, 5- to 10-membered heteroaryl, $C_{1-4}$ alkoxy, $C_{1-2}$ alkyl-O-$C_{1-2}$ alkyl, $C_{1-4}$ alkyl-O-$C_{3-5}$ cycloalkyl, halo $C_{1-4}$ alkyl, halo $C_{1-4}$ alkoxy, deuterated $C_{1-4}$ alkyl, or deuterated $C_{1-4}$ alkoxy, wherein the alkyl, cycloalkyl, heterocycloalkyl, or heteroaryl is optionally further substituted with 1-3 substituents selected from D, halogen, cyano, hydroxyl, amino, $C_{1-2}$ alkyl, halo $C_{1-2}$ alkyl, deuterated $C_{1-2}$ alkyl, $C_{3-4}$ cycloalkyl or phenyl;
$R^2$ and $R^3$ are each independently selected from H, D, halogen, cyano, amino, hydroxyl, $C_{1-2}$ alkyl-O-$C_{1-2}$ alkyl, hydroxy $C_{1-3}$ alkyl, $C_{1-3}$ alkoxy, halo $C_{1-4}$ alkyl, halo $C_{1-4}$ alkoxy, deuterated $C_{1-4}$ alkyl, deuterated $C_{1-4}$ alkoxy or $C_{1-4}$ alkyl; or $R^2$ and $R^3$ together with the carbon atom to which they are attached form 3-membered cycloalkyl, 4-membered cycloalkyl, 5-membered cycloalkyl, 4-membered heterocycloalkyl, or 5-membered heterocycloalkyl, provided that when $R^2$ is selected from H or D, $R^3$ is not selected from II or D.

4. The compound, or the stereoisomer, solvate, deuterated compound, or pharmaceutically acceptable salt thereof according to claim 1, having a structure of formula (III), (IV), (III-a), (III-b), (III-c), (IV-a), (IV-b) or (IV-c):

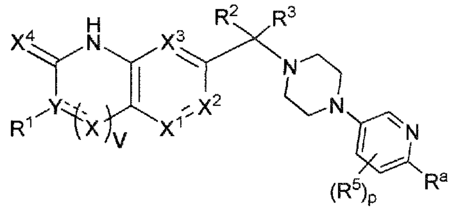 (III)

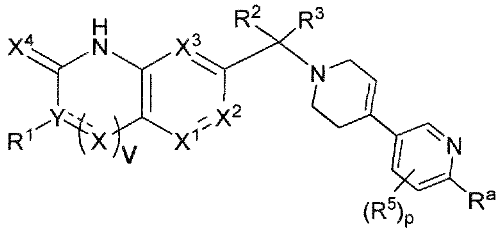 (IV)

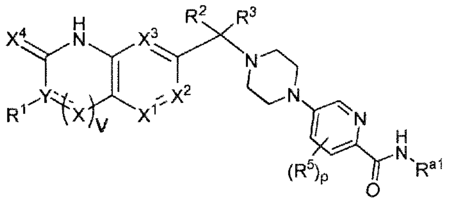 (III-a)

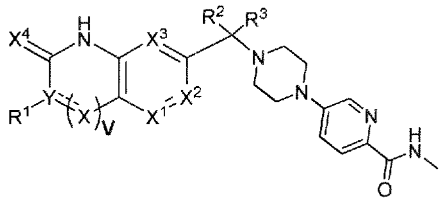 (III-b)

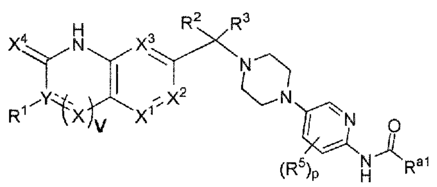 (III-c)

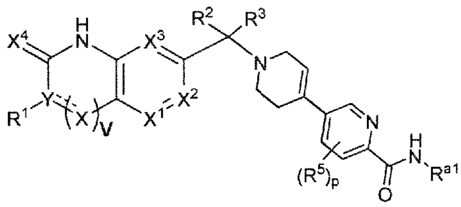 (IV-a)

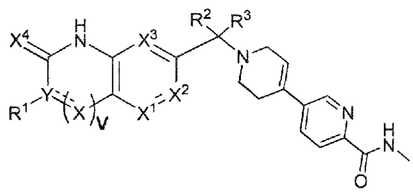 (IV-b)

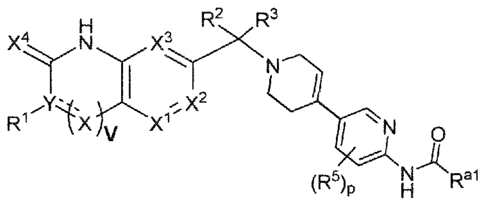 (IV-c)

**characterized in that** X is selected from $CR^x$, $C(R^x)_2$ or O;

$R_a$ is selected from $-C(O)N(R^{a1})_2$, $-NR^{a1}C(O)R^{a1}$, $-C(O)R^{a1}$, $-NR^{a1}$, $-NR^{a1}C(O)OR^{a1}$, $-NR^{a1}C(O)N(R^{a1})_2$, $-C(=S)N(R^{a1})_2$, $-S(O)_2N(R^{a1})_2$, 5- to 6-membered monocyclic heteroaryl containing 1-4 nitrogen, oxygen or sulphur atoms, 4- to 7-membered monocyclic heterocycloalkyl containing 1-4 nitrogen, oxygen or sulphur atoms, or 3- to 7-membered monocyclic cycloalkyl, wherein the heteroaryl, heterocycloalkyl, or cycloalkyl is optionally further substituted with 1-3 substituents selected from D, halogen, cyano, hydroxyl, amino, $-NHC_{1-2}$ alkyl, $-N(C_{1-2}$ alkyl$)_2$, $C_{1-4}$ alkyl, halo $C_{1-4}$ alkyl, $C_{1-4}$ alkoxy, halo $C_{1-4}$ alkoxy, deuterated $C_{1-4}$ alkyl, or deuterated $C_{1-4}$ alkoxy;

each $R^{a1}$ is independently selected from $C_{1-4}$ alkyl, $C_{3-5}$ cycloalkyl, $C_{5-9}$ bicyclic spiro cycloalkyl, 4- to 6-membered heterocycloalkyl, 5- to 9-membered bicyclic spiro heterocycloalkyl, 5- to 10-membered heteroaryl, $C_{1-4}$ alkoxy, $C_{1-2}$ alkyl-O-$C_{1-2}$ alkyl, $C_{1-4}$ alkyl-O-$C_{3-6}$ cycloalkyl, halo $C_{1-4}$ alkyl, halo $C_{1-4}$ alkoxy, deuterated $C_{1-4}$ alkyl, or deuterated $C_{1-4}$ alkoxy, wherein the alkyl, cycloalkyl, heterocycloalkyl, or heteroaryl is optionally further substituted with 1-3 substituents selected from D, F, Cl, cyano, hydroxyl, amino, $C_{1-2}$ alkyl, halo $C_{1-2}$ alkyl, deuterated $C_{1-2}$ alkyl, $C_{3-4}$ cycloalkyl or phenyl;

$R^1$ is selected from halogen, nitro, cyano, amino, hydroxyl, $-SF_5$, $C_{1-6}$ alkyl, $C_{1-6}$ alkoxy, $C_{2-6}$ alkenyl, $C_{2-6}$ alkynyl, $C_{1-6}$ alkyl-O-$C_{1-6}$ alkyl, $-(CH_2)_r-C_{3-12}$ cycloalkyl or $-(CH_2)_r-$(3- to 12-membered heterocycloalkyl), wherein the alkyl, alkoxy, alkenyl, alkynyl, cycloalkyl or heterocycloalkyl is optionally further substituted with 1-3 groups selected from D, halogen, cyano, amino, hydroxyl, $C_{1-6}$ alkyl or $C_{1-6}$ alkoxy;

each r is independently selected from 0, 1 or 2;

provided that when $R^2$ is selected from H or D and $R^3$ is selected from H or D, $X^4$ is selected from O, $X^3$ is selected from CH, ------- represents a double bond, v is selected from 1, and Y is selected from C, $R^1$ is not $C_{1-6}$ alkyl and halo $C_{1-6}$ alkyl.

**5.** The compound, or the stereoisomer, solvate, deuterated compound, or pharmaceutically acceptable salt thereof according to claim 4, **characterized in that**

X is selected from $CR^x$, $C(R^x)_2$ or O;
v is selected from 1 or 2;
p is selected from 0 or 1;
each $R^x$ is independently selected from H, D, halogen, cyano, amino, hydroxyl, $C_{1-4}$ alkyl, $C_{1-4}$ alkoxy, $C_{2-4}$ alkenyl, $C_{2-4}$ alkynyl, $C_{1-4}$ alkyl-O-$C_{1-4}$ alkyl, $-(CH_2)_r-C_{3-6}$ monocyclic cycloalkyl or $-(CH_2)_r$-(4- to 6-membered monocyclic heterocycloalkyl); or two $R^x$ on the same carbon atom together form =O;
each $R^5$ is independently selected from D, halogen, cyano, amino, hydroxyl, $C_{1-4}$ alkyl, $C_{1-4}$ alkoxy, halo $C_{1-4}$ alkyl, halo $C_{1-4}$ alkoxy, deuterated $C_{1-4}$ alkyl, or deuterated $C_{1-4}$ alkoxy;
$R^{a1}$ is selected from $C_{1-4}$ alkyl, $C_{3-5}$ cycloalkyl, $C_{5-9}$ bicyclic spiro cycloalkyl, 4- to 6-membered heterocycloalkyl, 5- to 9-membered bicyclic spiro heterocycloalkyl, 5- to 6-membered heteroaryl, $C_{1-4}$ alkoxy, $C_{1-2}$ alkyl-O-$C_{1-2}$ alkyl, $C_{1-2}$ alkyl-O-$C_{3-5}$ cycloalkyl, halo $C_{1-4}$ alkyl, halo $C_{1-4}$ alkoxy, deuterated $C_{1-4}$ alkyl, or deuterated $C_{1-4}$ alkoxy, wherein the alkyl, cycloalkyl, heterocycloalkyl, or heteroaryl is optionally further substituted with 1-3 substituents selected from D, halogen, cyano, hydroxyl, amino, $C_{1-2}$ alkyl, halo $C_{1-2}$ alkyl, deuterated $C_{1-2}$ alkyl, $C_{3-5}$ cycloalkyl or phenyl;
$R^2$ and $R^3$ are each independently selected from H, D, halogen, cyano, amino, hydroxyl, $C_{1-2}$ alkyl-O-$C_{1-2}$ alkyl, hydroxy $C_{1-3}$ alkyl, $C_{1-3}$ alkoxy, halo $C_{1-4}$ alkyl, halo $C_{1-4}$ alkoxy, deuterated $C_{1-4}$ alkyl, deuterated $C_{1-4}$ alkoxy or $C_{1-4}$ alkyl; or $R^2$ and $R^3$ together with the carbon atom to which they are attached form 3-membered cycloalkyl, 4-membered cycloalkyl, 5-membered cycloalkyl, 4-membered heterocycloalkyl, or 5-membered heterocycloalkyl.

6. The compound, or the stereoisomer, solvate, deuterated compound, or pharmaceutically acceptable salt thereof according to claim 4, **characterized in that**
$R^1$ is selected from halogen, nitro, cyano, amino, hydroxyl, $-SF_5$, $C_{1-4}$ alkyl, $C_{1-4}$ alkoxy, $C_{2-4}$ alkenyl, $C_{2-4}$ alkynyl, $C_{1-4}$ alkyl-O-$C_{1-4}$ alkyl, $-(CH_2)_r-C_{3-6}$ monocyclic cycloalkyl, $-(CH_2)_r-C_{5-9}$ bicyclic spiro cycloalkyl, $-(CH_2)_r$-(4- to 6-membered monocyclic heterocycloalkyl) or $-(CH_2)_r$-(5- to 9-membered bicyclic spiro heterocycloalkyl), wherein the alkyl, alkoxy, alkenyl, alkynyl, cycloalkyl, or heterocycloalkyl is optionally further substituted with 1-3 groups selected from D, F, Cl, cyano, amino, hydroxyl, $C_{1-3}$ alkyl or $C_{1-3}$ alkoxy.

7. The compound, or the stereoisomer, solvate, deuterated compound, or pharmaceutically acceptable salt thereof according to claim 1, **characterized in that**

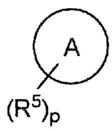

is selected from

| | | | | | | |
|---|---|---|---|---|---|---|
| 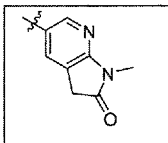 | 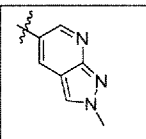 | 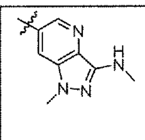 | 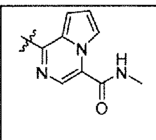 | 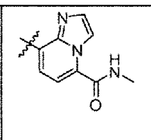 | 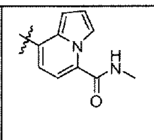 | 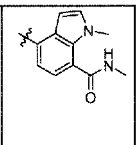 |
| 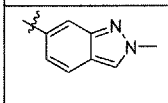 | 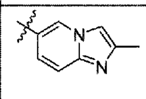 | 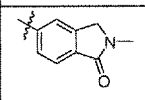 | 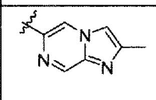 | 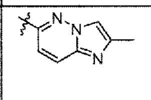 | 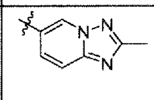 | 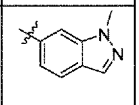 |
| 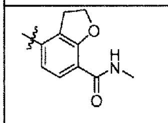 | 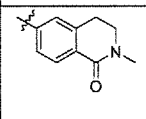 | 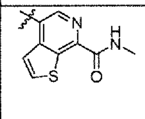 | 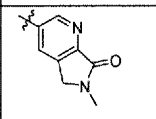 | 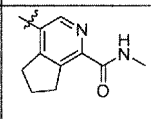 | 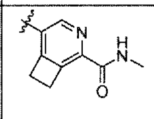 | 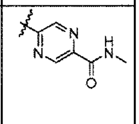 |
| 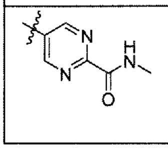 | 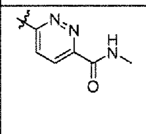 | 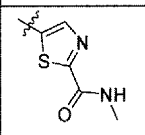 | 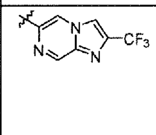 | 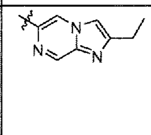 | 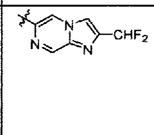 | ; |

or

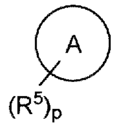

is selected from

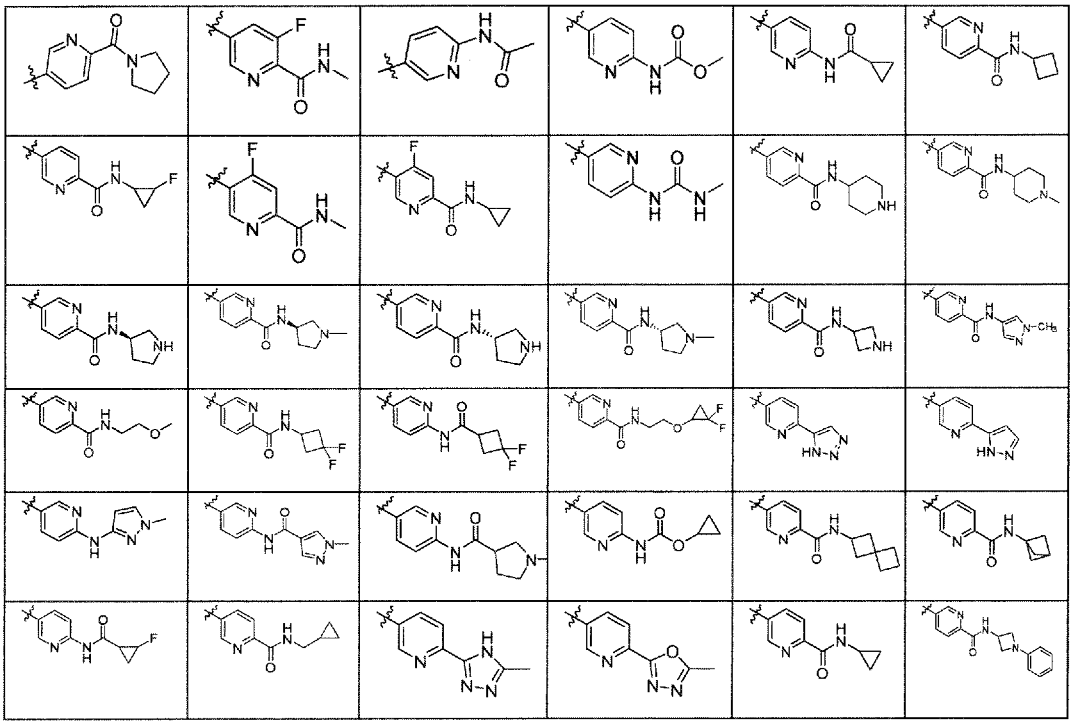

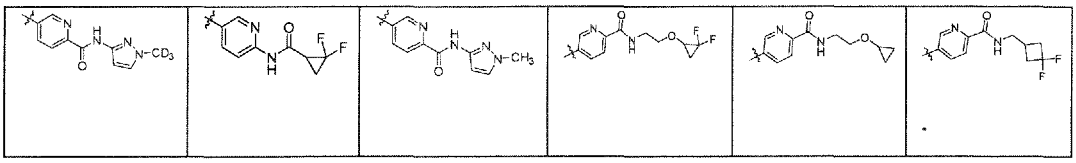

8. The compound, or the stereoisomer, solvate, deuterated compound, or pharmaceutically acceptable salt thereof according to claim 1, **characterized in that**
ring B is selected from piperazinyl, piperidyl or

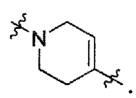

9. The compound, or the stereoisomer, solvate, deuterated compound, or pharmaceutically acceptable salt thereof according to claim 1, **characterized in that**

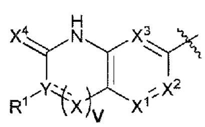

is selected from

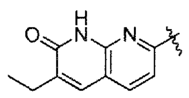, 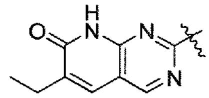,

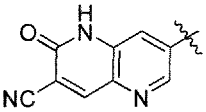, 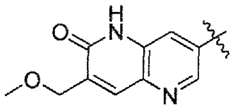, 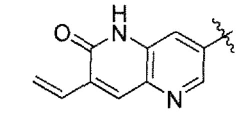, 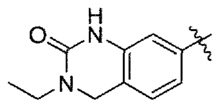,

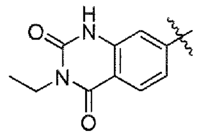, 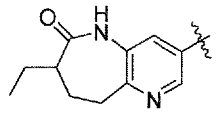, 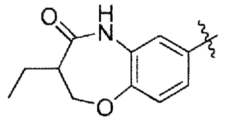, 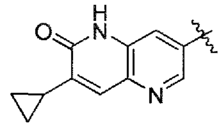

or

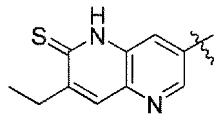.

**10.** The compound, or the stereoisomer, solvate, deuterated compound, or pharmaceutically acceptable salt thereof according to claim 1, having a structure of formula (V):

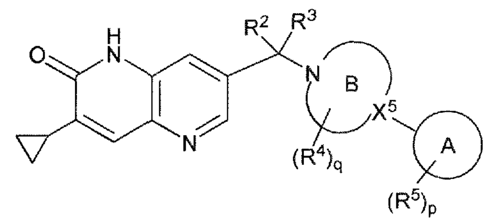 (V)

**characterized in that** $R^2$ and $R^3$ are each independently selected from H, D, halogen, cyano, amino, hydroxyl, $C_{1-2}$ alkyl-O-$C_{1-2}$ alkyl, hydroxy $C_{1-2}$ alkyl, $C_{1-2}$ alkoxy, halo $C_{1-2}$ alkyl, halo $C_{1-2}$ alkoxy, deuterated $C_{1-2}$ alkyl, deuterated $C_{1-2}$ alkoxy or $C_{1-2}$ alkyl; or $R^2$ and $R^3$ together with the carbon atom to which they are attached form $C_{3-5}$ cycloalkyl or 4- to 5-membered heterocycloalkyl;
each $R^4$ is independently selected from D, halogen, cyano, amino, hydroxyl, - $SF_5$, $C_{1-2}$ alkyl, $C_{1-2}$ alkoxy, halo $C_{1-2}$ alkyl, halo $C_{1-2}$ alkoxy, deuterated $C_{1-2}$ alkyl or deuterated $C_{1-2}$ alkoxy; or two $R^4$ on the same carbon atom together with the carbon atom to which they are attached form =O;
each $R^5$ is independently selected from D, halogen, cyano, amino, hydroxyl, - $SF_5$, $C_{1-2}$ alkyl, $C_{1-2}$ alkoxy, halo $C_{1-2}$ alkyl, halo $C_{1-2}$ alkoxy, deuterated $C_{1-2}$ alkyl or deuterated $C_{1-2}$ alkoxy;
q is selected from 0 or 1; p is selected from 0 or 1;
ring B is selected from

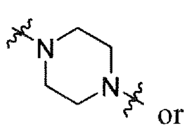 or 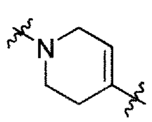;

ring A is selected from

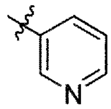,

which is further substituted with 1-3 substituents selected from $R_a$; or ring A is selected from 7- to 10-membered bicyclic heteroaromatic ring containing 1-5 nitrogen, oxygen or sulphur atoms, or 7- to 10-membered bicyclic aromatic ring, wherein the heteroaromatic ring or aromatic ring is optionally further substituted with 1-3 substituents selected from $R_b$;

$R_a$ is selected from -C(O)N($R^{a1}$)$_2$, -N$R^{a1}$C(O)$R^{a1}$ or -C(O)$R^{a1}$;

$R_b$ is selected from D, halogen, cyano, hydroxyl, amino, $C_{1-2}$ alkyl, $C_{3-5}$ cycloalkyl, 3- to 5-membered heterocycloalkyl, $C_{1-2}$ alkoxy, $C_{1-2}$ alkyl-O-$C_{1-2}$ alkyl, halo $C_{1-2}$ alkyl, halo $C_{1-2}$ alkoxy, deuterated $C_{1-2}$ alkyl, or deuterated $C_{1-2}$ alkoxy;

each $R^{a1}$ is independently selected from H, D, $C_{1-2}$ alkyl, $C_{3-5}$ cycloalkyl, 3- to 5-membered heterocycloalkyl, 5- to 6-membered heteroaryl, halo $C_{1-2}$ alkyl, halo $C_{1-2}$ alkoxy, deuterated $C_{1-2}$ alkyl, or deuterated $C_{1-2}$ alkoxy, wherein the cycloalkyl, heterocycloalkyl, or heteroaryl is optionally further substituted with 1-3 substituents selected from D, halogen, cyano, hydroxyl, amino, $C_{1-2}$ alkyl, halo $C_{1-2}$ alkyl, deuterated $C_{1-2}$ alkyl, or $C_{3-4}$ cycloalkyl.

**11.** The compound, or the stereoisomer, solvate, deuterated compound, or pharmaceutically acceptable salt thereof according to claim 1, having a structure of formula (VI), (VII), (VIII) or (IX):

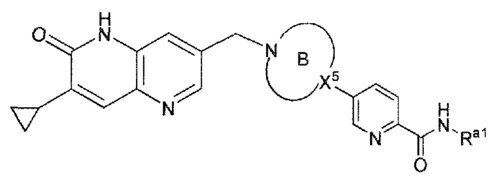 (VI) 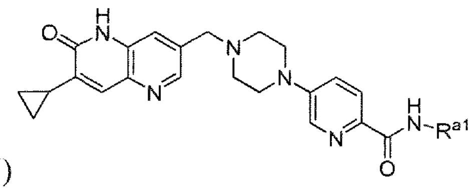 (VII)

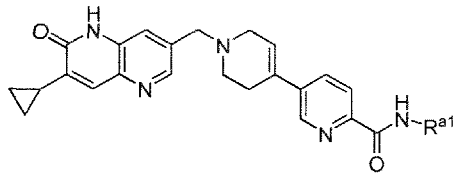 (VIII) 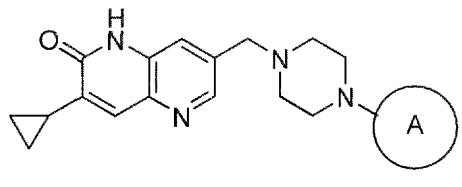 (IX)

**characterized in that** ring B is selected from

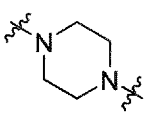 or 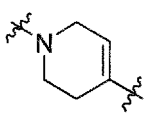;

$X^5$ is selected from N or CH;

each $R^{a1}$ is independently selected from $C_{1-4}$ alkyl, $C_{3-5}$ cycloalkyl, or 5- to 6-membered heteroaryl, wherein the alkyl, cycloalkyl, or heteroaryl is optionally further substituted with 1, 2 or 3 substituents selected from D, F, Cl, cyano, hydroxyl, amino, $C_{1-2}$ alkyl, halo $C_{1-2}$ alkyl, or deuterated $C_{1-2}$ alkyl;

ring A is selected from 8- to 10-membered bicyclic heteroaromatic ring containing 1-5 nitrogen, oxygen or sulphur atoms, wherein the heteroaromatic ring is optionally further substituted with 1, 2 or 3 substituents selected from - C(O)N($R^{a1}$)$_2$, -NH$C_{1-2}$ alkyl, -N($C_{1-2}$ alkyl)$_2$, $C_{1-2}$ alkyl, =O, $C_{1-2}$ alkyl, $C_{3-5}$ cycloalkyl, halo $C_{1-2}$ alkyl, halo $C_{1-2}$ alkoxy, deuterated $C_{1-2}$ alkyl, or deuterated $C_{1-2}$ alkoxy.

**12.** The compound, or the stereoisomer, solvate, deuterated compound, or pharmaceutically acceptable salt thereof according to claim 1, **characterized in that** the compound is selected from one of the following structures:

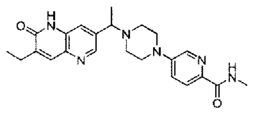 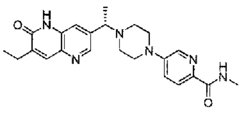 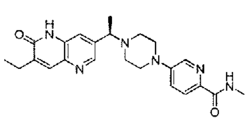 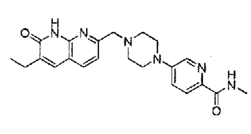

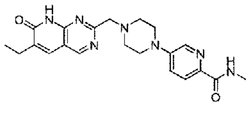 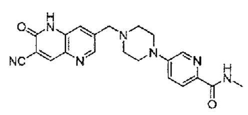 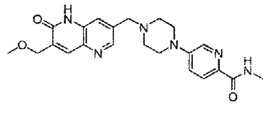 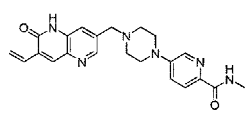

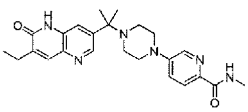 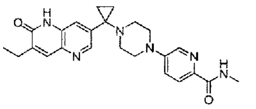 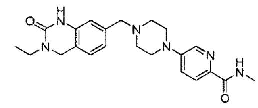 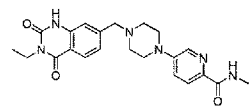

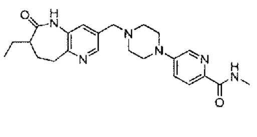 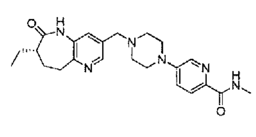 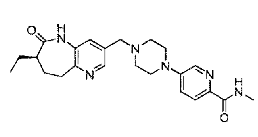 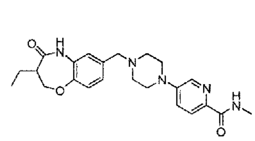

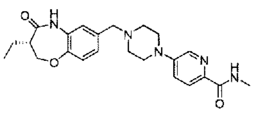 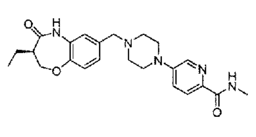 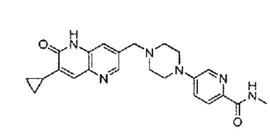 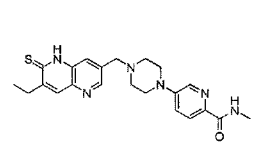

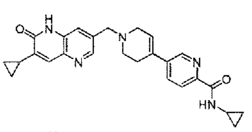 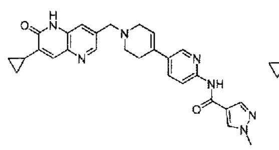 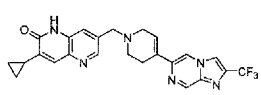 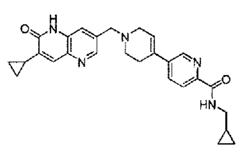

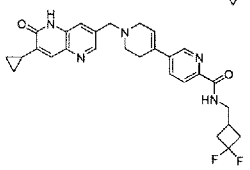 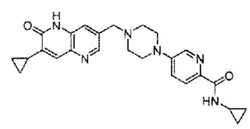 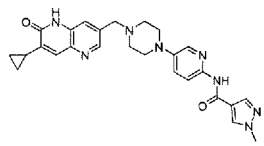 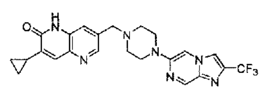

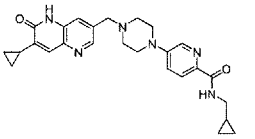 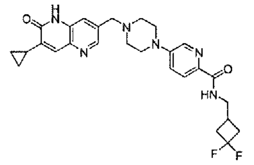

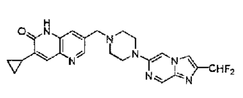 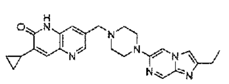 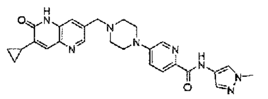 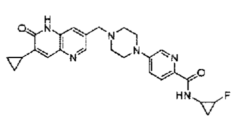

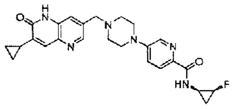 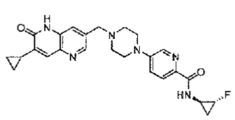 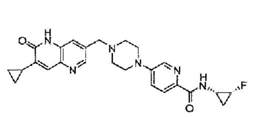 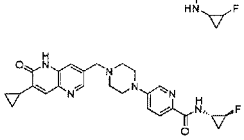

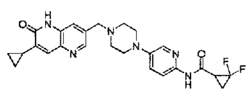 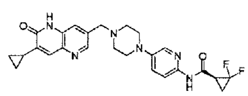 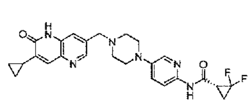 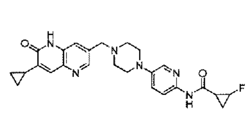

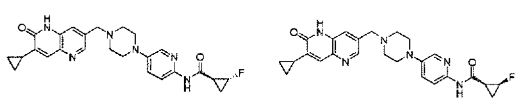 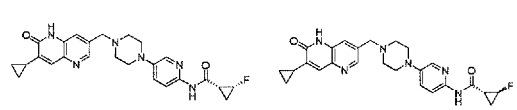

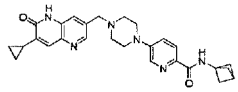 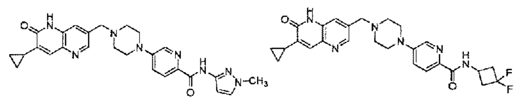 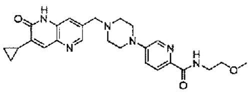

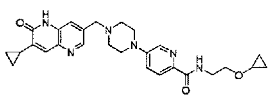 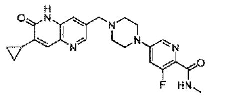 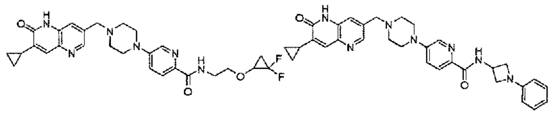

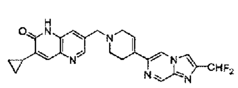 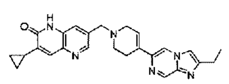 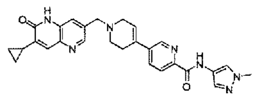 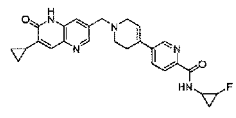

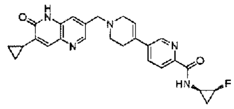 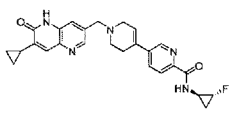 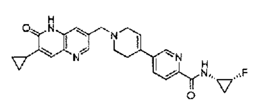 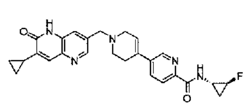

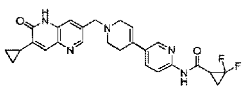 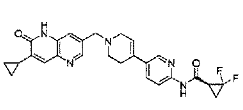 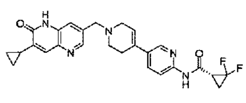 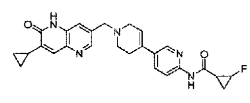

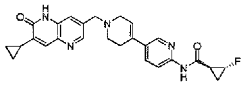 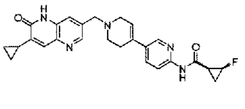 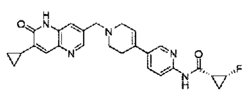 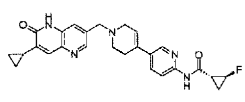

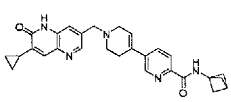 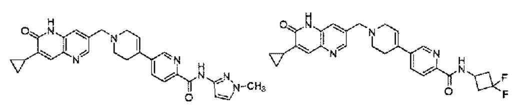 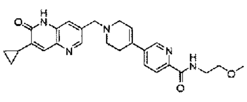

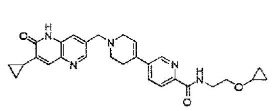 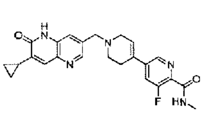 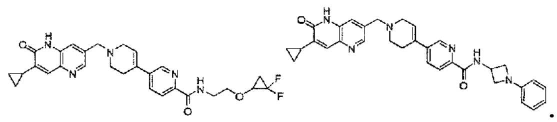.

**13.** A pharmaceutical composition, **characterized in that** the pharmaceutical composition comprises the compound, or the stereoisomer, solvate, deuterated compound, or pharmaceutically acceptable salt thereof according to any one of claims 1-12, and a pharmaceutically acceptable excipient and/or carrier.

**14.** Use of the compound, or the stereoisomer, solvate, deuterated compound, or pharmaceutically acceptable salt thereof according to any one of claims 1-12, or the composition according to claim 13 in the preparation of a drug for treating/preventing a PARP-1-mediated disease.

**15.** The use according to claim 14, **characterized in that** the PARP-1-mediated disease is cancer.

**16.** A pharmaceutical composition or pharmaceutical preparation, **characterized in that** the pharmaceutical composition or pharmaceutical preparation comprises 1-1440 mg of the compound, or the stereoisomer, deuterated compound, solvate, or pharmaceutically acceptable salt thereof according to any one of claims 1-12, and a carrier and/or excipient.

**17.** A method for treating a disease in a mammal, **characterized in that** the method comprises administering to a subject a therapeutically effective amount of the compound, or the stereoisomer, deuterated compound, solvate, or pharmaceutically acceptable salt thereof according to any one of claims 1-12, wherein the therapeutically effective

amount is preferably 1-1440 mg, and the disease is preferably cancer.

**INTERNATIONAL SEARCH REPORT**

International application No.
**PCT/CN2022/123423**

**A. CLASSIFICATION OF SUBJECT MATTER**

C07D 471/04(2006.01)i； A61K 31/4353(2006.01)i； A61P 35/00(2006.01)i

According to International Patent Classification (IPC) or to both national classification and IPC

**B. FIELDS SEARCHED**

Minimum documentation searched (classification system followed by classification symbols)

C07D471/-;A61K31/-;A61P35/-

Documentation searched other than minimum documentation to the extent that such documents are included in the fields searched

Electronic data base consulted during the international search (name of data base and, where practicable, search terms used)

CNPAT, WPI, EPODOC, STN: 结构式, PARP, 癌症, cancer

**C. DOCUMENTS CONSIDERED TO BE RELEVANT**

| Category* | Citation of document, with indication, where appropriate, of the relevant passages | Relevant to claim No. |
|---|---|---|
| X | WO 2009053373 A1 (JANSSEN PHARMACEUTICA NV et al.) 30 April 2009 (2009-04-30)<br>description, pp. 9-15, 40-43, 51, 75, 77, and 80 | 1-17 |
| X | WO 2010111626 A2 (TAKEDA PHARMACEUTICAL COMPANY LIMITED et al.) 30 September 2010 (2010-09-30)<br>description, paragraphs [0002], [0008]-[0029], [0101], [0111], [0181], [0188], [0253], [0274], [0374], [0417]-[0418], [0553]-[0557], and [0579]-[0580], and claims 1-95 | 1-17 |
| X | WO 2010085570 A1 (TAKEDA PHARMACEUTICAL COMPANY LIMITED et al.) 29 July 2010 (2010-07-29)<br>description, paragraphs [0002]-[0004], [0008]-[0009], [0100]-[0101], [0223]-[0227], [0238], [0313], [0446], and [0454]-[0460], and claims 1-40 | 1-17 |
| X | WO 2021013735 A1 (ASTRAZENECA AB) 28 January 2021 (2021-01-28)<br>description, pp. 1-2 and 8, embodiments 1-32, and claims 1-37 | 1-17 |
| PX | WO 2021260092 A1 (ASTRAZENECA AB) 30 December 2021 (2021-12-30)<br>description, pp. 1-3 and 10, embodiments 1-61, and claims 1-35 | 1-17 |

☐ Further documents are listed in the continuation of Box C.　☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **11 November 2022** | **25 November 2022** |
| Name and mailing address of the ISA/CN<br>**China National Intellectual Property Administration (ISA/CN)**<br>**No. 6, Xitucheng Road, Jimenqiao, Haidian District, Beijing 100088, China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/123423**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **17**
because they relate to subject matter not required to be searched by this Authority, namely:

[1] Claim 17 relates to a method for treating diseases. The present opinion was formed on the basis of a use of a compound or composition in the preparation of a drug.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2022/123423**

| Patent document cited in search report | Publication date (day/month/year) | Patent family member(s) | Publication date (day/month/year) |
|---|---|---|---|
| WO 2009053373 A1 | 30 April 2009 | PL 2215075 T3 | 30 April 2014 |
| | | US 2010222348 A1 | 02 September 2010 |
| | | ES 2448870 T3 | 17 March 2014 |
| | | EP 2215075 A1 | 11 August 2010 |
| | | JP 2011500758 A | 06 January 2011 |
| WO 2010111626 A2 | 30 September 2010 | JP 2012522007 A | 20 September 2012 |
| | | US 2012094992 A1 | 19 April 2012 |
| | | EP 2415767 A1 | 08 February 2012 |
| WO 2010085570 A1 | 29 July 2010 | EC SP11011284 A | 31 October 2011 |
| | | CR 20110452 A | 28 February 2012 |
| | | JP 2012515786 A | 12 July 2012 |
| | | US 2013274239 A1 | 17 October 2013 |
| | | CL 2011001754 A1 | 20 January 2012 |
| | | AU 2010206744 A1 | 04 August 2011 |
| | | SG 172958 A1 | 29 August 2011 |
| | | CA 2750106 A1 | 29 July 2010 |
| | | DO P2011000237 A | 15 September 2011 |
| | | EP 2389379 A1 | 30 November 2011 |
| | | CN 102341394 A | 01 February 2012 |
| | | US 2015031652 A1 | 29 January 2015 |
| | | CO 6410305 A2 | 30 March 2012 |
| | | US 2011158989 A1 | 30 June 2011 |
| | | EA 201170963 A1 | 30 March 2012 |
| | | KR 20110107396 A | 30 September 2011 |
| | | IL 213993 D0 | 31 August 2011 |
| | | BR PI1007358 A2 | 06 March 2018 |
| | | MY 152386 A | 15 September 2014 |
| | | MX 2011007741 A | 06 September 2011 |
| | | PE 20120418 A1 | 04 May 2012 |
| | | TN 2011000339 A1 | 27 March 2013 |
| | | NZ 594322 A | 25 January 2013 |
| | | US 2010190763 A1 | 29 July 2010 |
| | | US 2012122835 A1 | 17 May 2012 |
| | | MA 33053 B1 | 01 February 2012 |
| WO 2021013735 A1 | 28 January 2021 | AU 2020318599 A1 | 10 March 2022 |
| | | US 2021040084 A1 | 11 February 2021 |
| | | UY 38793 A | 26 February 2021 |
| | | CR 20220070 A | 21 March 2022 |
| | | EC SP22012826 A | 31 March 2022 |
| | | BR 112022000534 A2 | 10 May 2022 |
| | | AR 119424 A1 | 15 December 2021 |
| | | IL 289534 A | 01 March 2022 |
| | | KR 20220035941 A | 22 March 2022 |
| | | JP 2022541483 A | 26 September 2022 |
| | | CL 2022000110 A1 | 20 September 2022 |
| | | CN 114144413 A | 04 March 2022 |
| | | DO P2022000006 A | 15 March 2022 |
| | | CO 2022001590 A2 | 18 March 2022 |
| | | US 2022227768 A1 | 21 July 2022 |
| | | TW 202116750 A | 01 May 2021 |

Form PCT/ISA/210 (patent family annex) (January 2015)

**INTERNATIONAL SEARCH REPORT**
**Information on patent family members**

International application No.
**PCT/CN2022/123423**

| Patent document cited in search report | Publication date (day/month/year) | Patent family member(s) | Publication date (day/month/year) |
|---|---|---|---|
| | | EP 3999506 A1 | 25 May 2022 |
| | | CA 3145644 A1 | 28 January 2021 |
| WO 2021260092 A1 | 30 December 2021 | US 2022009901 A1 | 13 January 2022 |
| | | TW 202218664 A | 16 May 2022 |
| | | UY 39296 A | 31 December 2021 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021013735 A1
- US 2018141923 A1


### Non-patent literature cited in the description


- Synthetic Organic Chemistry. John Wiley & Sons, Inc,
- **S. R. SANDLER et al.** Organic Functional Group Preparations. Academic Press, 1983
- **H. O. HOUSE.** Modern Synthetic Reactions. W. A. Benjamin, Inc, 1972
- **T. L. GILCHRIST.** Heterocyclic Chemistry. John Wiley & Sons, 1992
- **J. MARCH.** Advanced Organic Chemistry: Reactions, Mechanisms and Structure. Wiley-Interscience, 1992
- **FUHRHOP, J. ; PENZLIN G.** Organic Synthesis: Concepts, Methods, Starting Materials. John Wiley & Sons, 1994
- **HOFFMAN, R.V.** Organic Chemistry, An Intermediate Text. Oxford University Press, 1996
- **LAROCK, R. C.** Comprehensive Organic Transformations: A Guide to Functional Group Preparations. Wiley-VCH, 1999
- **MARCH, J.** Advanced Organic Chemistry: Reactions, Mechanisms, and Structure. John Wiley & Sons, 1992
- Modern Carbonyl Chemistry. Wiley-VCH, 2000
- **PATAI, S.** Patai's 1992 Guide to the Chemistry of Functional Groups. Interscience, 1992
- **SOLOMONS, T. W. G.** Organic Chemistry. John Wiley & Sons, 2000
- **STOWELL, J.C.** Intermediate Organic Chemistry. Wiley-Interscience, 1993
- Industrial Organic Chemicals: Starting Materials and Intermediates: An Ullmann's Encyclopaedia. John Wiley & Sons, 1999, vol. 8
- Organic Reactions. John Wiley & Sons, 1942, vol. 55
- Chemistry of Functional Groups. John Wiley & Sons, vol. 73
- **P. H. STAHL ; C. G. WERMUTH.** Handbook of Pharmaceutical Salts. Verlag Helvetica Chimica Acta, 2002